(19) Europäisches Patentamt
European Patent Office
Office européen des brevets

(11) EP 1 217 079 A1

(12) **EUROPEAN PATENT APPLICATION**

(43) Date of publication:
**26.06.2002 Bulletin 2002/26**

(51) Int Cl.[7]: **C12Q 1/68**

(21) Application number: **00403659.6**

(22) Date of filing: **22.12.2000**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **INSTITUT NATIONAL DE LA RECHERCHE AGRONOMIQUE (INRA)**
**75007 Paris Cédex 07 (FR)**

(72) Inventors:
- **Bernard, Michel**
  **63360 Saint Beauzire (FR)**
- **Sourdille, Pierre**
  **63340 Le Breuil/Couze (FR)**
- **Guyomarch, Hélène**
  **91400 Orsay (FR)**

(74) Representative: **Texier, Christian et al**
**Cabinet Régimbeau**
**20, rue de Chazelles**
**75847 Paris cedex 17 (FR)**

(54) **Microsatellite markers from triticum tauschii**

(57) The present invention relates to a map of the D genome of wheat bread, microsatellite markers and primers for detecting said markers and uses thereof for selection, detection of mutations, study of diversity, phylogeny, and evolution and for the identification of cultivars and hybrids of wheat bread species, Triticum species and related species of commercial interest.

EP 1 217 079 A1

SEQUENCE LISTING

<110> INSTITUT NATIONAL DE LA RECHERCHE AGRONOMIQUE INRA

<120> MICROSATELLITE MARKERS FROM TRITICUM TAUSCHII

<130> D19004

<160> 555

<170> PatentIn Ver. 2.1

<210> 1
<211> 891
<212> DNA
<213> TRITICUM TAUSCHII

<220>
<223> n means a, g, c or t

<400> 1
ngctatttat caaagtgaca atcaatcggg tgctaggagt taagatggat aacttgcaac 60
acagtcggaa actaacatct agcaaacatc attacccaac acatacatca cgaatctgcg 120
agaccaaaga acttgcctgg tgcctcgctt gtcggtgcca caacgggttg cgacatcatg 180
ccgccgtgaa cgggggaaga ccggcggaga acaggggaag accaatgagg aatagaagaa 240
gaggggctta gcacgaggnn gaagccgccg ccgccgccgc cgtgcaggag cgcttggtcg 300
ggtggaggat tgggtattat acctatttgg gctaggtcag gcttgtgcct tgggctaatg 360
ggctgcccaa aaaaacaang cgctacaagc tatgccgtgc gaaactttag tgtgggctct 420
ccgtgtaagc tatagcgccc tattagcgaa gaaccgacgc gctatttttt cgttgaaaaa 480
gccatgatca tcaagtctca aagatggaaa cagtgtgtgg tatttagtgc tccaaaagca 540
caaaaaatgc attaactatt tgtgttggat atgaaaatag tcgagaaaat attgtaatct 600
ataattgata tttatgggat tataagttgt ggttgacatt agcattgtca aaaaaanncg 660
gatagaggga tcggtgccat ncttctatna aacaaggaag ccaaggattg aaggtaatta 720
atgttgtcgc tttccagtca cttatgtatg tntgtgcttt tcattgtatt atgatatgtt 780
tgagcctatc aatagctcta cttggctatt tagttagtcc annnnnnnnn nnnnnnnnnn 840
nnnnnnnnnn nnnnnnnnnn nnnnnnnnnn nnnnnnnnnn nnnnnnnnng t 891

<210> 2
<211> 512
<212> DNA
<213> TRITICUM TAUSCHII

<400> 2
cttagatagc acatccaagg aaggaaggag attgaggttg gcacctcctg aaggcatgca 60
tgctcttcac caagagaggg aagggcatct ttttcttggg ttgcagtttc caccttgttg 120
ttgggtgagg tgagtcatga tttctctcct cccgcttcat tttttagaga taatgaacta 180
taatttccgt tttgtgctca tgaattgcat acttggaggt ttattcgatt tagagatatc 240
acaaacacac acacacacac acacacacac acacacacac acgcacgcac gcgcacacac 300
gcacccacac gcgcatgcgc acgcacaaac gcacacacac acacacttat gatctataag 360
agaagctgcg attttggcaa tagatgcgcg acaaagtagt ggttggtgtt tggtttaatc 420
aattatcaaa tgttttgcca ataacagaaa attaccttcc tcgtaaattt attgtccttc 480
tgtttcatgg tccagctgat ttgttgtttg tc 512

<210> 3
<211> 517
<212> DNA
<213> TRITICUM TAUSCHII

<220>
<223> n means a, g, c or t

<400> 3
aacagaacgg ctgcaggccc acctacggca ccaacacacg gagaagccgc cggcatacca 60
caacaacgtc gagagtgcac gaccacggcc aacacacaca cacacacaca cacacacaca 120
cacaccgcaa gacgtgctga cgctcgccga agccctcccc acagccccca taaccaagcc 180
ctcctctcaa cctcgcannn nnnnnnnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnntnnn 480
nnnnnnnnnn nnnnnnnnnn nnnnnnnnnn nnnnnnn 517

<210> 4
<211> 539
<212> DNA
<213> TRITICUM TAUSCHII

<400> 4
ctactagcta gaagcaactc tacgagtagt catcacagat gtcgacgacc tccgtgccgg 60
cagtaccgga caggctggcc ctgctccgtc tccgagtaga tggagtcggg gatggcttgg 120
tgctccgcga catcctccgc ctgcgtgact tcaaactcgg ccagagaatc tctctctctc 180
tctctctctc tctctctttc tctctctctc tctctctctc tctctctctc tctctctctc 240
tcgtttttcc cacctcacgt gggagagttc ctccactatc gcgattcgca cccaacctcc 300
ttcctgtcct ggccagcgca gacgtttccc atctctcctt cccctccttg tcgcgtcgag 360
ttgctacgag gtcatgctag taggggctgg agatggcagt caaatgtgtt gcgacgagtt 420
cacggggcaa cccactagga ccatttgaaa ccttccggaa ggttttgaaa ttttctgaaa 480
ccttctggaa ggtttcacca gaacatttac gtgacctctc tggaacactt ctggtataa 539

<210> 5
<211> 394
<212> DNA
<213> TRITICUM TAUSCHII

<220>
<223> n means a, g, c or t

<400> 5
ctccatccat gaaatggcgc aatgccctgt ccacagtgaa gagtgtgtgt gtgtgtgtgt 60
gtgtgtgtgt gtgtgtgcga gttccagagt tctaattatt cacttcacac ttggtgcaga 120
cacatggagg ctagtgaagg gctaagaact cagattggct tgcagaagca acttcatgca 180
cagcttgagg tatgcatatt ctccttggaa ctggcaagag aaggcgtgca gactttcatc 240
tattttagta acttgggttc tcttctattt tttccctctc cacagctcca aagaaagatg 300
cagctgcaag tagaagaaca cagcaagtta gtccannnnn nnnnnnnnnn nnnnnnnnnn 360
nnnnnnnnnn nnnnnnnnnn nnnnnnnnnn nnnn 394

<210> 6
<211> 542
<212> DNA
<213> TRITICUM TAUSCHII

<220>
<223> n means a, g, c or t

<400> 6
cgtagacaca ctctccccct cgttgctata catctccatg gattttttta ggatacgcac 60
gagcgtgcgt atcatatatt agagatagag agggggtaaa gatccccaac caccaatgtc 120
ttacaggcga ttacaagccg tgatgcaacg gcccaccgtc cactacgtgc actcctacgg 180
ctatctaccc tacggctagc cacctagcta gagaaccgag gaaaggcccg atgtctccct 240
taaatagccc tgctgccgtc cacgatcgag cctcactttc tactctcgca agttagtcca 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nn 542

<210> 7
<211> 552
<212> DNA
<213> TRITICUM TAUSCHII

<220>
<223> n means a, g, c or t

<400> 7
caagtagcat ataccagaag ctaccagcct agcagcagca acaaagattt tgtaaataca 60
gcagataaaa gcttagctcc atggagcaat gccatccaga catattgcgg ccaccagcgg 120
ttccctggat cattcagcca ttttgatctc tctctctctc tctctctctc tctctctctc 180
tctctctctc tctctctctc ttttgaatta tttttttgtg gttgttgcga aaactctctt 240
atttgaattt gtcaggagac gtgtctgaaa accatacttg acatcatagt tagtccannn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn nn 552

<210> 8
<211> 534
<212> DNA
<213> TRITICUM TAUSCHII

<220>

<400> 8
ccaccaccgt catgtcactg aggtaaccag aaaccgacaa cctctctctc tctctctctc 60
tctctctctc tctctctctg cgttggtcct tccatctttc ctcgcaacag cggcaacgcg 120
gcgacacctc gtcactccga gagtttgcgt cttgtcgtct tcactttcca tggctacgtc 180
ctcccatccc atggatccac ccacccgccg tccgcttcct cgccacatat ccctttccct 240
ttcctttccc actcccatgc agggattact cctacaatga tgattaccca tgtgtgctcg 300
tcatacacaa ggattactcc tgcaatgacg aatcgcgtga tcatctaaac gacggaattt 360
gagatgaagg atttgggtat aacctcgagc acctccactc atacattatg gttagtccan 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnn 534

<210> 9
<211> 385
<212> DNA
<213> TRITICUM TAUSCHII

<220>
<223> n means a, g, c or t

<400> 9

```
cagttcacat gtagttgctg tcagtgatga tgattcatgc gggctacgat tctcttcttt 60
ctccgctgga gatggacgtt gcaccagcag cgagcgagtc tagcacacac gcatggcctg 120
gcaccttgta aagtaacgtt gcacgcacct aaactctggc ctggctactg accgatcgac 180
ctcgccacga cgactgcacg gagagatcga tcgagcacgc acggctttct ctctcgcttg 240
ctcaaaatag cacaccgagt cttctctctc tctctctctc tctctctctc tctctctctc 300
tctctctctc tctctctctc taaattgccg acgctcacac ttgtagtcca nnnnnnnnnn 360
nnnnnnnnnn nnnnnnnnnn nnnnn                                       385
```

<210> 10
<211> 505
<212> DNA
<213> TRITICUM TAUSCHII

<220>
<223> n means a, g, c or t

<400> 10

```
attcggaaca gtttacaagg tatgactaac accattttttg cctttagtct acaattcatg 60
caagctttgt aattaatggt ggagagtcat tgacgaattt ggctctgcat acattcagat 120
ttcagagtca ccgttctatg acgtgtcatg ctaactttat aatagttcgt ttttttaat 180
ggaaatcttt cttttctcga gaagatgcaa aagttcacat ctcgatgcat tgatagaaag 240
agagaataaa tcttattaac agatatacaa tcatatatgc accatcctca cacacacaca 300
cacacacaca cacacacaca ccatattatt gcactttcaa tatattttct ctactagtat 360
aacaaacaat tttgagtata ttaacaagag gtgtcaaaca gggtgttttt gaaaatggag 420
aagatattgc tgtcaaggtt agtccannnn nnnnnnnnnn nnnnnnnnnn nnnnnnnnnn 480
nnnnnnnnnn nnnnnnnnnn nnnnn                                       505
```

<210> 11
<211> 503
<212> DNA
<213> TRITICUM TAUSCHII

<220>
<223> n means a, g, c or t

<400> 11

```
ctgtagttac tggatttgcc ttggatttga agaagcaagg cagatggttc cttccaacac 60
acaaacacac acacacacac acacacacac acacacacac acacacacac acacacacat 120
ttggtggttc atttttgtgg ttgcgtgtca gtaaaaacct taggtgcgtg agatgcaacc 180
tttttccgcg gcgatggtta gtccannnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnntcttcc gcttcctcgc tca                                         503
```

<210> 12
<211> 533
<212> DNA
<213> TRITICUM TAUSCHII

<400> 12

```
ctccgtccgt gctccctcaa tgcacacatt gttacccaaa cctgcccttt gttttagtgt 60
tactttttac acacaaaatc aagtttgtgt gtgtgtgtgt gtgtgtgtgt gtgtgtgtgt 120
gtgtgtgaga gagagagaga gagagagctc accgtatgca tgtgtgagcg agccatgccg 180
ggtttagcgc gtcagcattg ccatgctgat cccgactcgt aggcgtagtg gacaccggat 240
gacggtgcat tcccatggta gggatggctg gagtagattg ggttgtgata cttttccttg 300
gcattttctg ttgaattaaa gctgaccaag ttgtgttttg gggtatggat tgtgatccaa 360
gaagaagaag agaagaggta gggtggtaac tagtaagagc tgcatccacc cttgcttacc 420
ttcaacatac tatgggaatg ggattcacat aagagtttaa gcaacagtcc tgctgtgttt 480
ttgtccaagc catgtaaaat ctgaccaagt cctgccgagc gcgcctactt tcc        533
```

<210> 13
<211> 551
<212> DNA
<213> TRITICUM TAUSCHII

<220>
<223> n means a, g, c or t

<400> 13

```
ccacaccact aaccaagctg ccatttaaag catatctctc tctctctctc tctctctctc 60
tctctctctc tctctccagt gcaacttgta tgtctgtatt acgtggatat tttttgagg 120
ggaaaataac tgtattacgt ggatatagta tgcatgtatg gtgcacattt gacagatcgt 180
ccatcgtgca cgtgcgtaac catccaatca gcgggaacac aaaaaaatta tcatatattc 240
agcagatcaa tgccaaaaac ataacgtgtc gtgcaccatc atccatgcat atgttgatga 300
gaaaataatt aactactact agtaatgtta gtccannnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn n                                                      551
```

<210> 14
<211> 297
<212> DNA

<213> TRITICUM TAUSCHII

<220>
<223> n means a, g, c or t

<400> 14
caagggccac cggccagagt agtattctga acttatctgt aaggaggtta gactagttag 60
atctctctct ctctctctct ctctctctct ctctctctct ctctctctct ctctctctta 120
tctccacgcc ttggaatctt ctcgttgtta gaccaggact ctaccatgac ttgtagtcca 180
nnnnnnnnnn nnnnnnnnnn nnnnnnnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnn 297

<210> 15
<211> 430
<212> DNA
<213> TRITICUM TAUSCHII

<220>
<223> n means a, g, c or t

<400> 15
aaaaacatgc catcgggatg gtgaagcttg ttgcaaaaaa taaaagatgg tgattggtga 60
agcgagtggt gagcaaaaaa ataggaggag gtatgtgcag actgcagacc ttgacggcgg 120
cgtagacggc ggcgccgacg acgccgggtt cagcctgcca ggtgccgagc cccaccgagg 180
gcatcttcgc tcccgtattg agcaggaagt gtctcgccat agccgcctct ctctctctct 240
ctctctctct ctctctctct ctctctcaac aggggactgg ggaagcagga gagcgggtgg 300
ttgtgatatg catccggttt tggcgcgctc aacgcattga caagaaggag atcatcacca 360
cacctgcccc attctctgtt tatttgcttg tctttctttt tctctaatta ggtggaactt 420
cgacatctag 430

<210> 16
<211> 532
<212> DNA
<213> TRITICUM TAUSCHII

<400> 16
cgagagattg aaacacctaa ataaccgaag aaaatggcgg ctgctctgga gatgtagaag 60
aagcatggtg tccatccgtg gtcggcgatg caggggcgtt catccctggt cagtgcggag 120
tgacggcaaa acccagcgcg tcggcatcgc ggggcttgag gcggcccaag tgtggaggat 180
ggaggggatc caagggaatc caaattgggt tttcccaaac tcatgaatgg ctgcacgaca 240
gcggcggcat ggtgtgatat cgtggcggcg acggcgtcgt cgtgtgatat gggaaccgag 300
ggaatccgga tcggggcttg ccggaagtca tgaacggctg cgtagtgtga tatggcgacg 360
gcagcggcgg cgtggtgtga tatggtggcg gcggcggcgg cgtcgtgtga tatgggaacc 420
gaaggaatcc ggatcggggc ttgccggaag tcatgaacgg ctgcgtggtg tgatatggcg 480
acggcagcgg tagcgtggtg tgatctgacg gcggcatcgg cggcggcggc gg 532

<210> 17
<211> 424
<212> DNA
<213> TRITICUM TAUSCHII

<400> 17
cccacgttct cttggtttgt tgggtggtcg ttcaggcttc ttcggtatcc aggcaaaaga 60
gatatgtcat taaaaacaga tttaaatgtg tgtctctata tcttctactg acaaggacgc 120
agtgaataca ttcgacacgt gtcaacagaa tgcatacgac tggatagata gaatttgatg 180
aagaagcaca gaaggggtta gggtccgatc acattcactt tgtttagaaa atggcaagtt 240
tgaagatata gctataagtg aatgatgtag aggacagaac acacacacac acacacacac 300
acacacacac acacatagat aagcgttagg atgtgtgtcc aatcacagga cattcaagga 360
ttctaagata tttagctcac accgcagctt gaaaaatctt ttctcatcca agggcttagt 420
gaag 424

<210> 18
<211> 495
<212> DNA
<213> TRITICUM TAUSCHII

<220>
<223> n means a, g, c or t

<400> 18
gtgtcgccac agagggctca ccggactgcc ccatccaaca gcaccaagag aggacgccat 60
gcgcacacct tgtctttcgc agttccctag agtttcatag ttggtcccat tgagattata 120
tccagagaga gagagagaga gagagagaga gagagagaga gagagagagc gagagctggt 180
cgcaatgaaa tagtagtagc atatgttagt ccannnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nntct 495

<210> 19
<211> 361
<212> DNA
<213> TRITICUM TAUSCHII

<400> 19
tacgcaggtt tgctgcttct cagcctggtt ggttggttga tatcctcaaa cagaacagaa 60
gctctcatgc aggccagccc gagattagga attttgctac aaaaacacga ccccatggac 120
gcgtccgttt tggagatgct agagaagcca atgaactgct gagagagaga gagagagaga 180
gagagagaga gagagactgt gatgtatgtc cgttttggtg gtatggagtt gtgccgggac 240
atggatgaac ccatgcttgt gaactccggc gagactgtga tgtatggagt gatcacggag 300
aaaaatagtt gagcagtaga aacaagcaag ccaggcattt aaaaagaccg aggggagcgt 360
g 361

<210> 20
<211> 455
<212> DNA
<213> TRITICUM TAUSCHII

<400> 20
ttttcgcctt gagtgcagcc gacgttacat tcaactgaag ataaggtatt acaggctttt 60
gccttctaac cgtggaatta acaacagttt tctcaatcta gtgtgatggg aaggtaatgg 120
gaggggggtca gggaagtggg tggaggaagg caggaatggt ggctacctgc ctacggactg 180
gtccgcggcg ttgctcggca tgaccggatg ctcgacggag ggatgcgcta caggggggcgg 240
tggggcagag gaggcttcgc cgggctcggc acgccgcgcc tgatggcatg cagttactgc 300
ggctcctggc ggcaatccgg cgggatccct tgattgcggc cgcggcgagg tccggtggga 360
tcccttgttt gcggcggcgg ctttggacga gaactggatt aggggagggg cgagaaaaag 420
tataggcggc tgcggccgtg tgacggtctg aggca 455

<210> 21
<211> 552
<212> DNA
<213> TRITICUM TAUSCHII

<400> 21
actgtcaaag ttgttggtgg catgtataag ctcagcttct gaaaatatag tgaaagcaag 60
acctttctca gatttcatcc tatcaaacaa aagcaaccct ccatgtaggc ggaaatactc 120
cctcttaact ttttccaatt ttctcttctg gattatcatt tgtccccaaa atacagtgat 180
catggcgata atgattgccc caaataatcc tagtgtgact cctgcatgca tgacgatatg 240
ggatgagaca actatttcag tgagagagag agagagagag agagagagag agagagagag 300
agatagttag ttggtgttct ttcgctaaca aaagatgtat tattagttta aaaatttacg 360
gttagtgaat gggacacaaa taataaaggt tttagtctat ttctcatgtc caagtccaca 420
cactgatcct catctgatgt cttaattaag aatatcaaat aactagcacc ataaattaga 480
caaaaggaaa actcaatgga cttaaaggtc aagggatagg acaaaaataa tatttttagc 540
tagatgatct ca 552

<210> 22
<211> 542
<212> DNA
<213> TRITICUM TAUSCHII

<400> 22
cagttgcatg tccgacacta ggctttgcag ctataagtgt cgccccatct acaattgcat 60
gtctccctcc tggctgcaat tggtcttgtg tttttgtcgg aggacggcgg gagagggatg 120
gcagttgcat gtccgatact aggcgtcagg agatattggg cccaattgca tgtcccatgc 180
taggcatgca actgcatgtc ttctaccttg gtcgcaactg catgtcttca aacttggttg 240
caaaccgtct tgtttttgtt aaacgacgag agggaccagt ttgcatgtcc catgctaggc 300
acgcaatttc atatcttcta acttggttgc aattgaggcc tttgtttgtt agaggggcgg 360
catcaagaga gagagagaga gagagagaga gagagagaga gagagagaga tagagagaga 420
gagaggcact tgcatgctca aatgtagtca tgtagttgca tgtcgtctaa ctttggtcgc 480
aactcgactt tttcttctgt cgggcaagga gaggggccag ttgcatgtct tccaacatgg 540
tc 542

<210> 23
<211> 505
<212> DNA
<213> TRITICUM TAUSCHII

<400> 23
ccagtagcag tagcagtagc agtagcagga gcaggagcag cagcaggagc agcagccgga 60
gcagcaacaa caacagcacc agcagtagca ggagcagtag cagcagcagc agcagcagta 120
gtagtagtag tagtggagca ggagcagcag cagtagcagc agtatcagta gcagtagcag 180
cagcaggagc agtagcagta gcagtagtag tagtagtagt agtagtagta gtagcagtag 240
gagtagtagt agtagtagta gtagtagtca gtagcagcag cagtagtagt agtagtagta 300
gtagtaggga gtggtgcgcc gcccgctgca tgcccggctg aacactcttc cttgccgcca 360
tcaaacgcct tcattaaacc cgcatggaat tcgagaaacc tactccggcc atacgtccgt 420
tcatgagagg gccgggatta aacacaacgc tgattgagct cctcacgtcc gccctctatt 480
taaacgaggc tagacatcgg ccaag 505

<210> 24
<211> 463
<212> DNA
<213> TRITICUM TAUSCHII

<400> 24
ttttgagcag ctcaaggaga gagagagaga gagagagaga gagagagaga gagagagaga 60
gagagagaga gagagagaga gagcacaaac aaagatagca aagaaaaggc aacactggcg 120
caatctgaaa gaaaagcaag agaggaccat atttcatgag aaaatcctgc acgccatgcg 180
ccacgattta cttctttgtg tgttttactt tttttttcg tcagtgcagc tacctatcct 240
gcaccgtccg tcctcattac tttctctctc ctcataacaa gcataccata tctctttaga 300

```
aagagaaata gcaagaagat aaaccccaaa ttaaagttga tgcaaatata taaatggata 360
gggggcagcg agacgaaagt gggacctgga acggccccat ggggtggtgg tggtcatttg 420
gggagagggg ctgcaccatg ggcggcggcc gtgggctgag aaa                  463


<210> 25
<211> 432
<212> DNA
<213> TRITICUM TAUSCHII

<400> 25
cttaaagagc agtaagacag tttaatttcc gttgggcggc taaccatgca agtcagcaag 60
gagtgaccct tttaagccac catatataaa atattgacgc gctaccgtca gtcgtttaat 120
ggcagccaat gaagcataaa tgcctcctga cagccatgca gccacccaac agggattaat 180
ggacatggag atgatgtcaa agagccatgc ccaggacttt ccccaaacca tcgctcatgc 240
taaggtcaaa cagacgaaac agatgtgacc gcctagtgcc atacctaaga atcaaccaga 300
cgggatcgat ttgagattaa aatgctaacc tgagagagag agagagagag agagagagag 360
agagagagag agagagagag agagagagag agagagagag atgagaaaat accacccagc 420
taacagacac gc                                                    432


<210> 26
<211> 488
<212> DNA
<213> TRITICUM TAUSCHII

<220>

<400> 26
catcaagatc gtgccaaatc aagaacacga gaaagagaaa gagagagaga gagagagaga 60
gagagagaga gagagagaga gagagagaga gagagagaga gagagagaga gagatcaaac 120
acatagtgtg atagacagac ttattaggga tgacagtgtg atagcctgac ttattattag 180
ggatgataga ctactcatat caataaggaa ttccttcttt tccgggagcc cattcggaaa 240
gaactccata gttaaacgtg ctcagcttgg agtagtttca ggatgggtga tcgaccggga 300
agttgctccc gggtgcgcat gagtgagtta gtccannnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnn                                                         488


<210> 27
<211> 542
<212> DNA
<213> TRITICUM TAUSCHII

<220>
<223> n means a, g, c or t

<400> 27
caagcgtggt gacagagtat acatcctttg tagtgtagga acccagattg tcccaaatat 60
gcacataaac aagttaagca agtcatctag caactattga agggtaggca tataggaacc 120
attcctaaaa taagtttgca gcaagatcaa atcgacatgg acagtcacac acacacacac 180
acacacacac acacacacac acacaaaagg caagcctttt gttggttcca ttccatccat 240
ggaacagagg agatttatac ttcctacaag gaagcaacga tggcaccaag tcctcagtag 300
tccannnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nn                                                               542


<210> 28
<211> 511
<212> DNA
<213> TRITICUM TAUSCHII

<220>
<223> n means a, g, c or t

<400> 28
ccatttgtag aacagtttaa ttgcatctta ttactggagg cattaaatta acaataacat 60
tggttggaca tgtccttact gtaacagtgg gattgcttag ttttacaacc gcccttacat 120
taacaacaac aacaacaaca acaacaacaa caacaacaac aacaacaaca acaacaacaa 180
caacaacaaa aagttggtat aagggcatgc gacagtggac gcattagcac catgttagtc 240
cannnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn n                               511


<210> 29
<211> 503
<212> DNA
<213> TRITICUM TAUSCHII

<220>
<223> n means a, g, c or t

<400> 29
```

```
attatattgt gtatagaatt aggagttgtt agataggttg tcaggcagga tatttgtttt 60
aactcgtcct tatctcctgt aatctctctc tctctctctc tctctctctc tctctctcgc 120
gcgcgtcctt atgtgaggtt cacgatcgat gtatgctccc acgggatgtg cgccctgatc 180
tatcaatata tatcacannn nnnnnnnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnntnnn 480
nnnnnnnnnn nnnnnnnnnn nnn                                          503


<210> 30
<211> 534
<212> DNA
<213> TRITICUM TAUSCHII

<400> 30
caaaccttat agggtcactc aagcaaatac cacaaatcat ataatcgcac aacaatggtt 60
cacagaatta cagaaaaaat gagagagaga gagagagaga gagagagaga gagagagaga 120
gagagagaga gagagagaga gagagaagca gcaactggtt ggagttctga gaagaactgc 180
agcaaacggg tttgagaaga gcttcagaag aagtgcagta gcagcaacgc taggagagat 240
ggggcagcca cagctagcaa ggagcagaga ggagaggcag cagagccaag gcaagactac 300
tacagaacct acgactaagt cgaactatat acttttacga ttgatgggca tactcgcgac 360
ttcacccata ataattcaaa aaaaggtgaa tccttcattt ttcaaatttg agcattggca 420
catatccact gtatggatta gtcatattat caggttgggt cagtatatta gtaaatttgt 480
cacatataca cagtttgtcg aaccaattgc aataataatc aatagacatg cata       534


<210> 31
<211> 512
<212> DNA
<213> TRITICUM TAUSCHII

<220>
<223> n means a, g, c or t

<400> 31
caaggaaaca actgcaacgg acgtgggtgg ttatatatag ctactgatgg gtcgagcgag 60
cacactggcc cggctacagc tctagcacca accttgatag ggaaacgcgg agagagagag 120
agagagagag agagagagag agagagagag agagagagag agagagagag agagagagag 180
agagagagag agagagagag agagagagag aggagcagga agatggccag gaaaggagag 240
gggccaacat cacgtccgga gccatgacac gccaccttaa aaaatctcaa gaaaaccaca 300
ccccaaaccg aactgagccc ctctgcgtca cctccaccgc cgggtaaaat catcaggcac 360
cacagcatgc catggccacc gtaaccacct ctgcaacagc actaacagcg tcatcgatcg 420
gcgacaagaa cggtgtcacg atctctcttc ttttgcttca tgttcatacg tgcgtgtgca 480
gccgnttctc tcttttccta catgttcatc cg                               512


<210> 32
<211> 529
<212> DNA
<213> TRITICUM TAUSCHII

<400> 32
ccctttgacg ccgcgatgtg ctgtgagtgg catcgttgcc aagaacagaa cagggcatgc 60
atacagatca gcacacgcac tgtaaaaacg agggaaaatg tgagaaatgt gttgttcgtc 120
tacttaccag agcggttctt cccttggagt caccgacgtc agggtccatc ccgaccttga 180
ggaggtcctc aaggaagcca gcgttccccg tggcggcgac cgtcagcaga ttgcacggga 240
cgatgttgcc gcagccgccg gctccggtgc tctcgccgag caggtccttc atgtcatgca 300
cctcaatctg atgctgcaac acaaccacaa tttccgactc aacaatcttg acacatcaca 360
cacacacaca cacacacaca cacacacaca caaaccctag tagcaacata gtaagatgca 420
tcgaccaacc ttgaggaagt tcctgacgat gagggcgctg tcgtcgggct tgctctgcat 480
gacctccctg agcgtggcct gctttagccg cagcagctgg ctcagcgtc          529


<210> 33
<211> 527
<212> DNA
<213> TRITICUM TAUSCHII

<220>
<223> n means a, g, c or t

<400> 33
tatctattgt tggatgagtt ttccttgatt aactcttgga aaccaagatt gcattttact 60
atcagtagta tcacattgca atcgcagggc atactaagcc ctttctaccg tgtttcttta 120
ttattctgac gcctacattt ctatctaccg caataatcac acccaacaca cacacacaca 180
cacacacaca cacgcacacg cacacacaca cacacgcaca cacacacaca caaatggtgt 240
gattgttctc taactgtatt tcccatatat agaagcttca cagtggttgg aaattttagg 300
gacagtccat cgaccataat ctgacggttg taaaatagat accagactga aaataccagt 360
tagtccannn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnn               527


<210> 34
<211> 495
<212> DNA
<213> TRITICUM TAUSCHII
```

<220>
<223> n means a, g, c or t

<400> 34
ccttggcacc gtagagttgt cgtcgtcgcc gtccgagggc tcctcgagtt ccagttttta 60
gaaagggagc tcgctcacga ccggatcacc atctcgaagg cctccatggg aagaaccgca 120
acagacatgc tcctcgatag ccgaagcact gatcgcagca gcggtgcaga agggggcatc 180
agggcgccac tgccacgacc tcggtgggca gcgttggaag gggaacaaga gcttgacgcc 240
aagactggga ggacgagaag gaggtgaagc atggagcatg gggggggggg ggaggaggga 300
ggagaagatg ctctttatag ccatgtacgg cggtgctgga ctcgacatgc ccctttgcgc 360
gcgcgagaga gagagagaga gagagagaga gagagagaga gagagagaga gagagagaga 420
gttagtccan nnnnnnnnnn nnnnnnnnnn nnnnnnnnnn nnnnnnnnnn nnnnnnnnnn 480
nnnnnnnnnn nnnnn 495

<210> 35
<211> 551
<212> DNA
<213> TRITICUM TAUSCHII

<220>
<223> n means a, g, c or t

<400> 35
catgtaaggg atgacacata acggacaaga gattgacacc ttcgtataca agcacatctc 60
tctctctctc tctctctctc tctctctctc tctctcgtaa tgatcaagga gtccaacttt 120
tgagattcaa ttcaaaaagc atccattcca tcttctggat agatcactca aaaaatagcg 180
tactatagcg ccgctgatag cacactatta cgtgtagaga attgtctagc taaattgatg 240
agtgtataat gtctcgctaa tagcatattt tcaggggcgg cgctattttt tgtagcacgc 300
tatttttttt cttggtatag atacctaagt ttacatagga tccatcaaag gatatgacac 360
atcaaggtgt ggtgtccgtt gacaacatct gtggagtttt gatatttaga tattaaacat 420
gtttgttcgc cctaataagg agcaaaatta aacttaaatt gcaagttcct agctaaccac 480
agtagttatg agagaagccc tcttccagct atatatgtag tccannnnnn nnnnnnnnnn 540
nnnnnnnnnn n 551

<210> 36
<211> 549
<212> DNA
<213> TRITICUM TAUSCHII

<220>
<223> n means a, g, c or t

<400> 36
caatgtgcct gaggggactg agtaatgcaa gcgaacctta tgtcttcaaa aatgggaggg 60
tgcaaagtgt agccgaggaa ggcggcacac acacacacac acacacacac acacacacac 120
acacacacac acacacccgc ccgcccgcac gcacgcacgc acatcatgac gcaaaattgc 180
ggagttcaag gacatatgat gttgcttcat catgacattg acatgttaaa ctgaaggcgc 240
tgcaaaactc taaatatatg ggagatatga gaaatgggag aaaaatattg gcacttgtac 300
gacacagtta gaaaccgtag aaaaggtgac agtgttatgt agtccannnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnn 549

<210> 37
<211> 497
<212> DNA
<213> TRITICUM TAUSCHII

<400> 37
cactagcttc ttttgctgct tttgcagcct tagctctctc tctctctctc tctctctctc 60
tctctctctc tctctcttct cctgggcttc agcacatgtt ggatgtgatg catccaggac 120
aatctcattg tcctcaaatt ctggcctcga gggaagatgc tctttatcaa gaagatatgt 180
ccctgttccc atcttggagt tgattagcct ctgtatgtgg ggggcacatc cacatgatct 240
cttctgatca gctgcagttc tcttcactgt ctcaacaatt agagtaatca cttttgaact 300
tctgtggcac atcaaacata tggagcaagt tgatggagtg accacggatc atcctatgat 360
ctcctgactt gggcaacaaa gtgtgcctca agatggtgtt gatggtggtc aatcctgaca 420
gcagataata tatggaccca agcttgtgag tctccaaggc atcatctgga atggtcttgt 480
agtccacgcg cctcgag 497

<210> 38
<211> 514
<212> DNA
<213> TRITICUM TAUSCHII

<220>
<223> n means a, g, c or t

<400> 38
ccacttttgg catgttaaca aggcggcaag gtgaataggg ttggttcttt aactggtttg 60
tcatctttag ttgacctttg atatacaaca tagtggccat tcgatattca aaacgatcac 120
ataaaattac aaatgtgata aatacaatag ctattgttgg gtattttcac ataattggta 180
tttatattta ttttaccata acaacgcttc agggagagag agagagagag agagagagag 240
agagagagag agagagagag agagagagag agagagatgt atttatttta tcatgcgcct 300
caactcacca acgacatgcc ggcagactct gaacaaaagg atcgatggag ttagtccann 360
nnnnnnnnnn nnnnnnnnnn nnnnnnnnnn nnnnnnnnnn nnnnnnnnnn nnnnnnnnnn 420

nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnn 514

<210> 39
<211> 541
<212> DNA
<213> TRITICUM TAUSCHII

<220>
<223> n means a, g, c or t

<400> 39
ataccacagc tacatcatct ttcctttcac aacagagaac aaaagtagag agaggaaaga 60
gtgggtttag agagagagag agagagagag agagagagag agagagagag agagagagag 120
agcagtgtgc ggtgcatgat gggtggtgat gcagcatgtg gctgctgttc aaactttgct 180
ttctagaaaa agggaaattg aggcgcaata atacggctat ttttggcatt ttctcttgca 240
attgtagcca ggcgcatctt gcatgtgcaa cgccgttgtt agtccannnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
n 541

<210> 40
<211> 552
<212> DNA
<213> TRITICUM TAUSCHII

<400> 40
attgatttct atccatttct gcgacaagta attcagaacg gagggagtat catgtaaaaa 60
taataactga caaaaaggga cttcgtatcg atcataattg aactcaatat cacccccccc 120
cctccacaca cacacacaca cacacacaca cacacacaca cacacacaca cgagaatgtt 180
ttttgttaaa taaggatagc aaaaacttta ccgaagcggt gttattctta ttatttcgag 240
taatattcct tgtgtatatt tgttgagtta gttccttaat gtcaactata ggatagtgct 300
gctcacttct ccatattgga attgttccca agggacacga aaccaaatgg atactagact 360
ctaatgttgt atcaggtatc gagttgccat cgagtccaat aggatgtgag cttggttcca 420
tctgcatgca agtcaaagtc gtttgttagt tcccctaaaa aaatcgttgt tagtttgata 480
catacaaacc aacatcctta catgaatgta gaataattca ctaccataaa tcaatgttga 540
ttacattatt tc 552

<210> 41
<211> 442
<212> DNA
<213> TRITICUM TAUSCHII

<400> 41
cgtgcttata atttgtgtag ctctgtcaat agattaatca aacaaacaat cgatgggaaa 60
aaagttagca aactttgcta gctcatggtg atttctattg atgtcaattt ctctttcgcg 120
agtgaaacat aaagtctcag gcgacccacc agataaaagg tgctgagaag aacatcttac 180
ttagaccaca attagttaca aacaaacatc aagtaaagaa acacacacac acacacacac 240
acacacacac acacacccct tgagaatatc aaaatcttca aacacaagag cgcgccttga 300
ccaaggttga agcgaatgca atacctagat ttgggaatgg caaacgactt gaaaaatatg 360
ccttcttctc ttcaaagcaa gatggacctc gtcatggtgc catccgtcta tcactgtaga 420
gaccccccttg ccctggctcg aa 442

<210> 42
<211> 368
<212> DNA
<213> TRITICUM TAUSCHII

<220>
<223> n means a, g, c or t

<400> 42
catggcctca ggccaacgac ggcggcaaca tcatcgactg gttcgcccga ggggttgggg 60
catgaggagg ttctaggggg catgtctagg gaggaaaggg acaagggcta agtggccgac 120
acagaagaat agatgtggag aacaatgaga gagagagaga gagagagaga gagagagaga 180
gagagagaga gagattgggt tagaagattg atatgacatg tcggccagtg cagtcattga 240
gagcgacgac tgatcctagt tgggatcgtc cttctacaag tattccaaaa aaagtgtcat 300
tagtagtcca nnnnnnnnnn nnnnnnnnnn nnnnnnnnnn nnnnnnnnnn nnnnnnnnnn 360
nnnnnnnn 368

<210> 43
<211> 494
<212> DNA
<213> TRITICUM TAUSCHII

<220>
<223> n means a, g, c or t

<400> 43
caaacaaaag tcggtgcagt cccattttct aaagaaatta ctctataata tgcacacaca 60
cacacacaca cacacacaca cacacacaca catttcaacc taaataacac actattaaag 120
gaggggacat cccccttttaa ccatgttttt ggatagtttc tttgttttag ttggattttt 180
catgttgctt ggttgtagtc cannnnnnnn nnnnnnnnnn nnnnnnnnnn nnnnnnnnnn 240

```
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
ntcttccgct tcct                                                    494
```

<210> 44
<211> 537
<212> DNA
<213> TRITICUM TAUSCHII

<400> 44

```
ctccctgatc cgctgaaaat acaaatgttg ttgacactct atcaaaaacc caatggctct 60
cacactcaag ccctcgtggc tgtcacagat actctctctc tctctctctc tctctctctc 120
tctctctctc tctctctctg tgtgtgtgtg tgtgtgtgtg tgtgtggggg ggcgcgcctg 180
tgtgttgtcg agcgcccccg agagacccca tgatgatgcc cctgtgccac taccaactac 240
gtcctgaact cctaatgggc tttgagttgc ataattgggc catgggatga tatatatggg 300
acacatagtg ggggtagtgt gagaagtaga cgtgatgtgc gcatgagttg tatatttgct 360
gaatacataa tttcatacaa ataatgtaat cacgacattt tctgccaaaa agaagaggta 420
ttcatctaaa tacccatgaa ttatgttggg accgccctcc ttcccaaaat aattggacac 480
ccatgacggg tctctatgtc cagagtgata gttagtccac gcgcctcgag gaattca    537
```

<210> 45
<211> 535
<212> DNA
<213> TRITICUM TAUSCHII

<400> 45

```
cattgttgag ggagacaaca gagcgaacga caccacgcgg tgcggggtta ttcagccccc 60
acacgttcct gatcagaagg ttgcaatcta tagggaaact ataaaattga caagctggaa 120
gctagacctt ggctggcccc cgcgtcacgg gccttggcaa gcgcggtcaa agcctcgatc 180
tgttccggtg agaggggttg accgaagaca tgcttgtatc tctccagttg ctcctcgtca 240
gactcgtccc ctctctcagc gatggctaga cctctcttgg acatgaccaa ctttccggcc 300
atcttcatgg tgcggttgcc gccgccgccg ccgccgccgg aacccttcct cccctggaca 360
cgagcactgt gcgcacgggt aagcacgagt agaccggttc cacatcatgg gccagcccat 420
ccaccagctg cgagtcgagc accgctgaag tagcaacagc gtcttctcaa aaaaaaaaaa 480
aaaaagtagc aacagcgatt aataacacag tttttccctc aaaaaaaaaa gatta      535
```

<210> 46
<211> 224
<212> DNA
<213> TRITICUM TAUSCHII

<400> 46

```
ccattggaaa gacacaagat ggtaataatc cagcaaacat atgtggtggt atagtcgttg 60
gagctaggta ttggatatta gttttttcgt gtgtgtgtgt gtgtgtgtgt gtgtgtgtgt 120
gtgtgtgtgt gtgtgtgtgt gtgtgtttga gttctatgat atttgtgag gcgaccgcat 180
ccccctccgc ccgctgcttt gatggtgtgt gtgtgtgggg gggg                  224
```

<210> 47
<211> 487
<212> DNA
<213> TRITICUM TAUSCHII

<220>
<223> n means a, g, c or t

<400> 47

```
ctacaaaatg accatgtcat gttttatacc acttgatgtg tgtgtgtgtg tgtgtgtgtg 60
tgtgtgtgtg tgtgtgtgtg tgtgtgtgtc ttgtctaccc gaattaagat acaagacatg 120
cactcaaata tttctgccca ggataatcca cttagacatg tattcagaaa tttgtgcttg 180
acatgtagtc cannnnnnnn nnnnnnnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn ntcttccgct 480
tcctcgc                                                           487
```

<210> 48
<211> 448
<212> DNA
<213> TRITICUM TAUSCHII

<400> 48

```
ttctttcagt gatgatagcg aagggttgat atatgttgat ggttgatggt gggtgtttgc 60
gagcgtggta tcatcaagat tcacatccat catgcatctc cctctctctc tctctctctc 120
tctctctctc tctctctctc taggtctata tggggctata tggagctgtt tgtattcttt 180
ttccaaatga gacatataga tgcatagata gataacatac tttggatgag aatcttgatg 240
atgctgcatc cgcacacaag cgcctccctt ggcccttcat cgatacataa tgatgcgggc 300
gggccggggc atggcatcac tatattccca tatggcatac atacatgatc aggtgagaac 360
ttaggttcat tatgtttgtt tttattccgc tgctaccact ttctaaatat gcatggttct 420
acacttcctg cagcaattgc tgattgat                                    448
```

<210> 49

<211> 508
<212> DNA
<213> TRITICUM TAUSCHII

<400> 49
cctgtattga acaactacaa atgattagag ataattgtcc gcgtatacaa tattattaat 60
tgagttcttc tggtgaggca tgagagagag aaagagagag agagagagag agagagagag 120
agagagagag agagagagag agagagagca tgttttatgt aagaactgta acatcctaat 180
tttcaatttg gatgttatca ctaagtatca ttcatatgca tccatattct tcatgcatct 240
tgagctttat ttgatttccc ttgtgaaccg attcaatact ttgtgcaact caatgaccac 300
ttgagagaag ataccatgac ttcttcaaaa gtttataaga aaagagttga aagtctcttg 360
aatcaatttt gaaagttagc caaagtgagg ttccaaattt ccttttccat ttattaaaat 420
aaagataaaa gagaagaaaa tatgatttct tcaaagtggc attaaaagta tggataaagg 480
gttttggaca aactttgatg catttgaa 508

<210> 50
<211> 503
<212> DNA
<213> TRITICUM TAUSCHII

<400> 50
ctcgttagag atgaaactat ctaatcgggt atgattattg acccaagtgt gcacaggtga 60
tctacatgcc agtaatacgc ctggcgaaat gatgttctaa aattctacta tgtgcatttt 120
gtatcataat tttagaagat agaatgccat gagattcacc ttgcactcag actctctgag 180
tgactcgtgc tttttgtgca acagattttt tttgacatgt ttttctgcaa cattttgtcc 240
cacatataat tgcggtaata gtaaacaatg agaagaaggt agcagtccac attcccatct 300
cttgttacca tcaatagaag tagtcagact atcacctgcc taacacacac acacacacac 360
acacacacac acacaaaatg taacacaact tttctggttt tatttcactt tttattcttt 420
gcaaaaaagg agaaacattt cctggttaat gtattttggg cttgccctcg ttaggatcat 480
acgatatgac tgcatacaca gcc 503

<210> 51
<211> 486
<212> DNA
<213> TRITICUM TAUSCHII

<220>
<223> n means a, g, c or t

<400> 51
ctagaacagg aggcttctct atgggaggat ttcacctcag ctcagagaga gagagagaga 60
gagagagaga gagagagaga gagagagaga gagagagaga gagagagaga gagagagaga 120
gagagagaga gagagttcca ccgccgagaa tggttggctg gctgcacagg ataagatgca 180
agccatgtta gagatgcgat aggcaagaaa acaaggaaga tcaaatcaat atgctgggca 240
ctagattgta acgaggaaaa cccctccaat gggcaccagc ccatgaaact tcactatgtc 300
ttgagaggct acaaactctg gggatttgtg taacgcccaa gatgcggtta gtccannnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnn 486

<210> 52
<211> 854
<212> DNA
<213> TRITICUM TAUSCHII

<220>
<223> n means a, g, c or t

<400> 52
ttccctaaga gcnccgattg gagcagncgg cccgccgttc ggcgaggtcg accgggcaca 60
gtctatcacc atttgcaact tcttatcggg acggctggga tgnaccacgc acgcgatcga 120
ctttacaaga cgcggagcga ttgtgcgact tcgttccact gcttggaggg cgtgtgaggc 180
aggagacggt gttggttcct gaggcttcct gccgatcagc ttgccgtgag ctagcggcac 240
cttgctcttc ctggggtcct ggatctttcg cttgtagctg gcgtatgggg gtgagtgtgc 300
gcgtgtgagt gtgtgagcgt gcggcgcgga atgaactact ctagctacca gccctgctaa 360
aaagagtaaa cgtcagttgg gcgctgtgcc ccaacttagc tacttctact aatcttgatg 420
ctgctcgcca cgtttgggtt taattccacc ggccactatt tgttaatact ctactatgag 480
agtcatctac aataaaggaa actatgtagg agtcgaacan nnnnnnnnnn nnnnnnnnnn 540
nnnnnnnnnn nnnnnnnnnn nnnnnnnnnn nnnnnnnnnn nnnnnnnnnn nnnnnnnnnn 600
nnnnnnnnnn nnnnnnnnnn nnnnnnnnnn nnnnnnnnnn nnnnnnnnnn nnnnnnnnnn 660
nnnnnnnnnn nnnnnnnnnn nnnnnnnnnn nnnnnnnnnn nnnnnnnnnn nnnnnnnnnn 720
nnnnnnnnnn nnnnnnnnnn nnnnnnnnnn nnnnnnnnnn nnnnnnnnnn nnnnnnnnnn 780
nnnnnnnnnn nnntttcccc ttgcttgtta ctgaatccct gggttaggcg accggtgcgg 840
caagcggaaa gctc 854

<210> 53
<211> 372
<212> DNA
<213> TRITICUM TAUSCHII

<400> 53
caaaattaca aagaacgaag aattgggttc tctcaattcc ctaatatcat ctttcctagc 60
catcatcaca aatccctatt tcccccatgt cttgcttaat ttgagttgtg gggtcttgtt 120
tggctttggg ctctctctct ctctctctct ctctctctct ctctctctct ctctcacaca 180
cacacacaca cacactttgt tggtgtggat ctcgctcaag tgtttctcgc tcttgctatt 240
tctttttcaa attgtccatt aatatctctt cgggtatcga agggagcaac gatatgtgcc 300

ctccttcata gtagaatgtg agaagattag cacaccggtg atttgaacat catgatcaaa 360
caaccaacgt tt 372

<210> 54
<211> 503
<212> DNA
<213> TRITICUM TAUSCHII

<400> 54
cacaaacctg aaatcgagtc cctcgttcca aaatgcatga aagcaattac aggatctgtg 60
agtggtgctg tgattcctgt cactcgaggg agggagagag agagagagag agagagagag 120
agagagagag agagagagag agagagagag agagagagag agagagagag agagttgact 180
gtggatgtct aggtgtgcac ggcacacaga tttctggccc ttgaccaaga gaggcccagt 240
ggccagtagg agccatcggc agtggccatc aaaaaagact gcccgtggaa cgcaatggcg 300
tctgcttttt ttccgaggaa atcaaaggct atctgatgac agccactcaa acggacggat 360
cagtcacgaa tgtggcggtt ccaggcccag cctcatcgtt gacctgacca gcccacttca 420
tcctggatga aaaatcgtgc acattgcaca ggggcagaga cgagtggttt attaccacga 480
gactttgaaa tgcatgcatg gca 503

<210> 55
<211> 527
<212> DNA
<213> TRITICUM TAUSCHII

<400> 55
cggagctagc agaccagtgg ggatgctcca atgttatctt tgaatttgat tgtcttgaga 60
tttttctggc atgcaagggg gaagttgata tctcgagtta tgctcttatt atcttgagat 120
tgtagagcca gtagccggcc ctactattag ctatgctctt attaagtcac acccatgtgc 180
aacccatccc tcctctaatg tgtgtgtgtg tgtgtgtgtg tgtgtgtgtg tgtgtgtgtg 240
tgtgtatata tatgttagag ttcaagtgcc aatcttctta agtataaagg gtttaaaagg 300
tcaaaaaatg cataacggga caaaatagga atatataccc acacatagct tactattgtg 360
tcatccaaac tgattgtccg tatctcgtgc taccatcttt cgctagttgc acccgaatga 420
cataagttga tagttcacta aactgtttgc aacagtgatt ctgaactgtg tgcgaggact 480
cagtttgcac ttgtgtatga atcaaacagc ccaaatgaaa aaaagaa 527

<210> 56
<211> 378
<212> DNA
<213> TRITICUM TAUSCHII

<220>
<223> n means a, g, c or t

<400> 56
acaagttgca taattacttg ccctcctaat taaattatct agcaatacag atgaagttaa 60
cttatttaat acccaatgga atgtatgtaa cgtgtgcact cttaccataa aaattcaaaa 120
attgatttca cgagagagag agagagagag agagagagag agagagagag agagagagag 180
agagagagag agatgtagag aattccatgg atggaagttg gaccagatga gtggatcgag 240
ttcttcatat gcatcggtta gcacatcacc agttagtcca nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnn 378

<210> 57
<211> 535
<212> DNA
<213> TRITICUM TAUSCHII

<220>
<223> n means a, g, c or t

<400> 57
tcacatggtt aaggtatgct gaatcagtta ttcccgcccc ctgaaaagag gctagaggct 60
aaaaccaacc tgaagagggc tgtgttccgg ccggctgtgt tcacattcat cattaacaaa 120
acaaaacgaa atcgccgtta acataggcag ctctcctcgc gcaaagagag agagagagag 180
agagagagag agagagagag agagagagag agagagagag agaccgtatc aaattgtcga 240
tcgagtggca ttattaattc ctcgattaag tctcggcatg ccgcatgcat gcgctattat 300
taccgtgtag tagctgggcc gatcgaaggt gtgtgaatta tcacgggctg aattccacgg 360
gctcctcttc cggaaacgct acatgcatca acagcgaatg gcggagcaaa tacagcagtg 420
agacacacga tgcagaatag agttagtcca nnnnnnnnnn nnnnnnnnnn nnnnnnnnnn 480
nnnnnnnnnn nnnnnnnnnn nnnnnnnnnn nnnnnnnnnn nnnnnnnnnn nnnnn 535

<210> 58
<211> 526
<212> DNA
<213> TRITICUM TAUSCHII

<220>
<223> n means a, g, c or t

<400> 58
ctaatgggcc tttaagagca aaataaatga aaatgactca ccctaaaatc aggccaacct 60
ctctctctct ctctctctct ctctctctca cacacacaca cacacacatt cactgcatgg 120
ttcttagtag agcgatccga tggcatgtgc tcccattcat ggtctccaac ctttcacccc 180
tctaattagt ttctactatg ataatatttg ttctttctga tacttcatta atattttctt 240
gttgggtaga tttacatttt aagataaaat gtgaattcac tgaaaaaatc tgtagcatca 300

ggataattta tacaaatatt ttaatttata ttttttaaat aaagggcgta attttcattt 360
tgcacggggc cccaaatttt aaaggttagt ccannnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnn 526

<210> 59
<211> 519
<212> DNA
<213> TRITICUM TAUSCHII

<400> 59
cacatttaga tctagttctc cactactgtt gttacgcaag aatcgtcaaa taattaatca 60
cgtcccggtt atcaccttgc caaatgggga aactatttgg tgaagtgcag ttattctgtc 120
ccctagcctc tccttcctca cctggaaaat ggtcacaatg aataaacaaa tgcttgtaaa 180
ctggatggta gtggaaataa atgtcaattg tgagagtcat tttcgtgtgg tcttgccgca 240
cgaacttcta tatagtcaag tgccctttat gcgcgcgcgc gcgtgtgcga gagagagaga 300
gagagagaga gagagagaga gagagagaga gagagtaatg ggctagagtt atgcatggaa 360
agcagggatt tgacgaggaa gaaaccttta gagtagattg tgctgaggac gaggcctgaa 420
ccctagcctt cttgagagct gaacctgtcg cggtgctgtt gcactagaga aagacaaaca 480
aaccaaagcg tgaataatca tgaccccatt tttatctta 519

<210> 60
<211> 543
<212> DNA
<213> TRITICUM TAUSCHII

<400> 60
ctgttaatgt tgcagaagag gataagtaaa tagaagtgta acataaatgg aacatacttt 60
gaacattatg aaaataactt ctgcaccatg acaggacggg tcatattcct tgggctgaat 120
cattgcattg atctgttcaa ggtgctttat aactctgtct tcatcaagat gccataatac 180
cttcggaagg gggtcaatat tcaatactac ctgaccggca ttcagtatca aaagattcct 240
gtgcctgatg gtgttcacac acacacgcac acacacacac acacacacac tgtgcgaatg 300
cccagttctt gagaagtccg tgaccatatt gttctaacga gtgagttttc ctgctcatca 360
aagtgaccat ggaatatttc ttaaaaaaag gtcccataat atcagcatat gctcttcttt 420
tggcaaactt cccagagaat caaaacaatg tgaaaacctg caggtgattc gacaaatgaa 480
gttagatttt gttaggataa tcagaagaat tatcactttt ataaacagtg ttattaaact 540
agc 543

<210> 61
<211> 543
<212> DNA
<213> TRITICUM TAUSCHII

<400> 61
ctgtggcaga gtttaaaatt caaatgcaac gcaaacacaa cacaaacagg atacaaaccc 60
atctagggtc caaggctcaa gagccgacgc gcgcgcgcac acaaacacaa acacgataca 120
aacccattag gttccaggcg ggctcaacgc tcaagagctg acacacacac acacacacac 180
acacacacac acacacacac acacgataaa aacccgaaag gggtccttgg ctaacaagcc 240
gaaacacaca cacaaacact aagctgagga agaccacgat agtgaggaac atcagccacc 300
atcagcctcc ccgaagttgt cgaatgagag catcaaggat ccgacgatga agatccctgg 360
gtcggaggcc tcgtgttcga cgaagatccc tacaataaga gctttgttaa ttacaaattt 420
ccatcggccc acgcaaatgc agaaatccga cccaagccag ccaaaagttt ggcaagttcg 480
ttcctcaaaa cgaaggtcgc ggagtgcatc tattagagct acctatcagt agtccacgcg 540
cct 543

<210> 62
<211> 528
<212> DNA
<213> TRITICUM TAUSCHII

<220>
<223> n means a, g, c or t

<400> 62
caagagctga ccaatgtgga ggcgtggagc agactgacaa cggaaataca caggctcatg 60
cctttattct ccatgtttgc taaaagagag ctgcccaggt ctcctgtgtc tctccccttg 120
cccccgtgct ccttttttcct ctctctctct ctctctctct ctctctctct ctctctctct 180
ctctctctct ctgagctctt ctcatctcac cgccgttagt ccannnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nntnnnnnnn nnnnnnnnnn nnnnnnnn 528

<210> 63
<211> 534
<212> DNA
<213> TRITICUM TAUSCHII

<400> 63
ccgtagttcg gttatgctcc aatcctgagg atgttgagga ccaatgtctc gttgattgta 60
attttgatga ttgagtaata taagttctat tcacatacac acacacacac acacacacac 120
acacacacac acacacacac acaaagatag gtctaccgta gttcggttat gctccgatcc 180
cgaggatgtt gaggaccaat gtctcgttga ttgaaatttt gatgattgag taatataagt 240
tctattcaca tacacacaca cacacacaca aagataggtc catgtttcgc ctctctcaac 300

```
ttcaaaatca gaagtcgcac cctcaactca gcgtgaccct caacttcgca taccagacaa 360
gttttatccc ctcccctaag caaagtgatg tctgcaatga gccaaagtta cggaaggcct 420
caccctccta tccgaccgtc gtcatcattg ctttgtctcc tccctaatgc cccaccttgc 480
caagttttaa attttgaaat gtcatctcgc actttaaatg tcacaaacga ccct       534


<210> 64
<211> 543
<212> DNA
<213> TRITICUM TAUSCHII

<220>
<223> n means a, g, c or t

<400> 64
ctcataaaat atagcgtcgt gaaccgaata atttgccaca gtgttgttgc ccctttcgcg 60
cagctctctc tctctctctc tcacacacac acacacacac acacacacac acacacacac 120
acacaaagag tttgatgact attgtgttcc atggcagcct ggtggcagtt ctccacctcc 180
caccactcct cctccacctc cactcttccc tccacgtagc taattacact ttgtgttaaa 240
acggaggtca cagttgcctt ggacgacgtc gaggagctca acccaaagct gtaacatggg 300
gtatatccaa cgcaacccga tccggctccg atggcaagcc agattattag tcagcaagcc 360
acagtgcaag actggtgaaa tcaagaattc tgacgttgcg tagggcgcgt tgacgtctcg 420
cgagcgtata tgttttcgtg tttcgtagtc cannnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnn                                                                543


<210> 65
<211> 376
<212> DNA
<213> TRITICUM TAUSCHII

<400> 65
cccaacagtt cttcattcac cgctgaatct actaccagaa catgcattac ttgcctgtcg 60
gctagcacag ccgccgtcac caattcagct gacacgtgaa cagtcactaa gctccgaaaa 120
tagtttcaaa gcgaggaaat atcttgcaga aaacctcccg tcagagtcag acgatgagaa 180
ggaagccaac tgtggcaagt ctgccgctgg cctgttaggt gaactggtga ggcgaatggg 240
cctggcacac gcagttgctc tctcgctctc gctctctctc tctctctctc tctctctctc 300
tctctctctc tctctctctc tgccgacgat gcggacgccg cggacaaaat cccaaaaaca 360
agggaggaaa actgta                                                  376


<210> 66
<211> 473
<212> DNA
<213> TRITICUM TAUSCHII

<220>
<223> n means a, g, c or t

<400> 66
cctggtgccc cattactgga tcagtgcaaa tattactgtt ggcaagcaca ggccatgtga 60
gtgaagggag agctagcaat gcatcttatg catgcatgac atggcacccc ggccggccag 120
aacttgaaag ataaaagatg acaaggtctt ggtggttttg gtgagaaaaa gacaaggcgc 180
gcgcgcgcgc gcgcgcacac acacacacac acagagagag agagagagag agagagagag 240
agagagagag agagagagag agagagagag agagagagag agagagagag agagagagag 300
agagagagag agatactatc tgagccaact gtgggcatgt gaaaaaggaa actcctaccc 360
ttatcatttg ggagtaatga attgttactc caagagacgt agtctttttt acaaactcac 420
aacaactggt tagtccannn nnnnnnnnnn nnnnnnnnnn nnnnnnnnnn nnn        473


<210> 67
<211> 489
<212> DNA
<213> TRITICUM TAUSCHII

<220>
<223> n means a, g, c or t

<400> 67
aggccccgcg gacaaattga gccttagacc aatgcaatga agtcatattt ccatgagcaa 60
aacaacacac tgcaactaaa acacacacac acaaaacaac acactgcaac taaaacacac 120
acacacgcac acacacacac acacacacac acacacaaaa caacacactg caactaaaac 180
acacacacac gcacacacac aaagtactga agtagaattc gaatggagat atgaaatgag 240
gtcctaaaaa ttaatgtagt gagctacaag tatataatgg gtacataatt ctcaccaaga 300
atcacgttac aatagataca ttagttgggg ttcggatcac ttggatagtc atatttcgct 360
tcgatgatca atgtttggac tgtgtccttg tgtaaaagga agacataatg atgtaggcca 420
nnnnnnnnnn nnnnnnnnnn nnnnnnnnnn nnnnnnnnnn nnnnnnnnnn nnnnnnnnnn 480
nnnnnnnnn                                                          489


<210> 68
<211> 456
<212> DNA
<213> TRITICUM TAUSCHII

<400> 68
tattttgcag catcacacgt tttatttatt tgtgtgtgtg tgtgtgtgtg tgtgtgtgtg 60
agtccttcgc aaaaagaaag taacagagta gttttcatat tgttagtcat taagaacaaa 120
ccaaaaaact taacttgatc tggtttgatt gggtggtttt ccacatggga gagatcctat 180
```

tgtaggacca cgggggatac aattttctct atactttttt ttgcactttt agttttttag 240
tagtttttgc gtttcattct agattacttg aaacatgttt ttattttatt ttatctggcc 300
tagtgtgctt gtttcataaa tgcaattaag agtatgtata atcaccacaa aaagaaaaat 360
ctgtataaaa tcgagaatgg cattttggag ctccgcctcc atggggccgc tttttttgaa 420
aaattcaaaa ttcatacttt tttgtttcaa aaaaat 456

<210> 69
<211> 527
<212> DNA
<213> TRITICUM TAUSCHII

<220>
<223> n means a, g, c or t

<400> 69
ctttattttc atgatgaaat accttgaatt gtgagctgca caaaaataag attatttacc 60
ttattttaca cacacacgca cacacacaca cacacacaca cacacacaca cacacacaca 120
cacacacaca cacacacaca cacacacaca cacacacaca ctccttttgg ggtatgtgcg 180
taaaatttca tgatgaaata gcttgaattg tgagctgcac aaaaaataaa aatcatggac 240
tttggggatg aacagaacat gtgctgaaaa gcacaaattt gttcttttat ttagccgtca 300
ttttattgta tttcatcatg aaaatttgga cacatgttag tccannnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnn 527

<210> 70
<211> 504
<212> DNA
<213> TRITICUM TAUSCHII

<220>
<223> n means a, g, c or t

<400> 70
actctcgtcg gcatagtcgc gcacgcacaa cctcggattt ctacacactt gtcggcatag 60
tcgcacatac gcggccgagg aggtgaacat tctcgccggc atagtagctt acgcacgatt 120
gcaagagttg cacatgtgcg gcggcatagt cacacacaca cacacacaca cacacacaca 180
cacacacacg actacggagc tgcacactcc ccttggcata gttagtccan nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnn 504

<210> 71
<211> 518
<212> DNA
<213> TRITICUM TAUSCHII

<400> 71
ccttcagctg ctgcatggac gcaccgcgct agcgctacgc tacgtgccag acctccatga 60
ttgatcagag ctctcccgcc tgacaagtta gccatgcatc gacgacccct gaccaatgca 120
aacaaagata tacgcatcca tctcggcgcc cgcatgcaga tacagtgaga tggaccgatg 180
caacaaagat atacacatcg atctcggtat tacggcccca tgcgtgctgc ggggctcacc 240
aatcaggatt cctgccctcg tgtctcgtcg tccaggtcac actgaccaat aagtaggccg 300
ggacaaggcc aaacagagca atctccatgg cgttggtgtc cgtgtgtata catgcactgt 360
gcacgactgt tggacttgtg tatatatact tacaaagata acatcacaca cacacacaca 420
cagagagaga gagagagaga gagagagaga gagagagagg gagagagaga gagagagaga 480
gagagcaaac tcaactggca cagccatgcc gggcccta 518

<210> 72
<211> 496
<212> DNA
<213> TRITICUM TAUSCHII

<400> 72
cgtgctcccc tcatgtggtg ttgctctgct ggtggccgag ggtgacagga aggagcacca 60
ccatggccga agacggtgtg gaggagtgct gatgtggcca aagatgacgg ggagggtgag 120
aaattaaaaa aactccttgg aatctcaccg aagtcggtgg gggaagggag cagggctgca 180
ttggggaagg ccccccgtgc aacgggctgg aacaggggtg ggagatggtg atgcaggcaa 240
catcgggtgt gcccggagag agagagagag agagagagag agagagagag agagagagag 300
agagaggggg agggagagtt gagacttttt tctagaggag gcatattccc aaggagtatg 360
ctttgagggg tagattctca aattttggag gcactacagg tataaaggaa ggactatgaa 420
gggcttaatt ccctgaaggg cttaattccc tgaagggctt agttttaggg gactaggaag 480
ggctagacat gctctt 496

<210> 73
<211> 528
<212> DNA
<213> TRITICUM TAUSCHII

<220>
<223> n means a, g, c or t

<400> 73

```
ctatttaacg atgttgaaag tgtttggaca agaggataga tcaatgtggg ccgtgatgag 60
tgtgatagat ggatgaccag gagacccacg tcctcggagg ggatgtgagc atgacagaaa 120
gaaatctgtg gcatgcgcta gcgttgcaac ggaagcgcta tgcggctatg ccgtttgggt 180
gtgcaccacc atgaccacag gtaaaaagca actctctctc tctctctctc tctctctctc 240
tctctctctc acatcagctc agatggcaga acagttagtc cannnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnn             528


<210> 74
<211> 504
<212> DNA
<213> TRITICUM TAUSCHII

<400> 74
cattggtcta ggtagcgaga tgttcggaat aatctaaacc ctgttttcat atgcataatg 60
tgtttaacac acagaacaat gcatccccac tgcccacaca cacaacattt tgatgttaga 120
tttgaaagcc acacattatc tcaaattcga atagttatgt ccatgcatgg atgcaatgtg 180
gtcagacgat gcgtgccatc tgtgtcgcaa gttcaaatca atttgccctt gttcgatgtg 240
aatacagtat ataacatgtt tgattcaaaa ccacaccagg catatctgca ttatattcat 300
gcatgcatga gttacaaaca tcattggctc ttattcaaat atttgaatcc acttttatac 360
tgattatcat ctatgtaagt cttttacacg cacacatgca cacacgcaca cacacacaca 420
cacacacact cctcctccct tcgtggatac caataactct cctactgtgc atgcatatat 480
gcacacacac tccccaccac tttg                                        504


<210> 75
<211> 542
<212> DNA
<213> TRITICUM TAUSCHII

<400> 75
catgacttac aatatccatc gatgcaaaat ggttgctaga actgaagcct aaaatacaaa 60
atgcagataa gttacatgat agaaatgcat aaacttggac cctggacaac taactgtcta 120
ccaagctaat ttgccatata tcatccatgg gtggatggat ctgaaacaga acatggcaca 180
gctcaaagca gaggagaggg gggcagaggt agggggtggg gcctgagaag aagtcaccgg 240
agaaggaggg aggctccatg ggcgcagcag agagagagag agagagagag agagagagag 300
agagagagag agaagggggg attttctccg agcggtgcga gatacacgga tgggtgtgcc 360
cgtgagtggt agcgtggctt agcaaacacc ccctactact aaatgggcct actagtttac 420
acgcagaaga aatagcagta gcgcgttttg cacaccaggc gctacagcta tgtctttagc 480
agtagcgctg ggttgatcac tcacgctgcc actatgtatt aacctggggg tatggtgtgg 540
ca                                                                542


<210> 76
<211> 548
<212> DNA
<213> TRITICUM TAUSCHII

<220>
<223> n means a, g, c or t

<400> 76
catgaaaata gtataccttc ttgtttttct ctttgataat taggggtcaa catccatctt 60
tgcttgcaag ctttggacta acacactttg catatacttg acaaagaagg ttagtcatca 120
atacatattg ttatttacac caaaaccaac tcgaaagaca attgcaattt cacacgcgac 180
ttagatcgat tgagagaaga gagagagaga gagagagaga gagagagaga gagagagaga 240
gagagaggga gatcctcttc ctcaacgctt ctctccacga gacaactgaa tctactccag 300
aaaatgtcga agtgtcatgt tgtcgagcgt tagtccannn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnn                                                          548


<210> 77
<211> 464
<212> DNA
<213> TRITICUM TAUSCHII

<220>
<223> n means a, g, c or t

<400> 77
caatctaccg gcattcaaag agcagcagct agcactagca agcagccaag gacagcatcc 60
aggagcttaa tggaagaaat caaatctgaa taaaacagat caagaagttg aggagaggct 120
gctgcttcag ggattggaga gggttacctg ccgtcaggag aagagggtat tgcctcctgt 180
tgtggggtag tggtcgccgg tcgatttcga gcgtcgccgg cggcggcggc ggcggcgttc 240
caggaggtgg cagctcgatc tgggaaccgg atcctctacc agggtgccgt gccttcttac 300
gacccccaac tgtggtttag cccaggaaac tactaaaggc ccatatcgac agcccagcgg 360
cttgcctgat gcctgatgcc ctttaaaaaa aagtagtcca nnnnnnnnnn nnnnnnnnnn 420
nnnnnnnnnn nnnnnnnnnn nnnnnnnnnn nnnnnnnnnn nnnn                  464


<210> 78
<211> 543
<212> DNA
<213> TRITICUM TAUSCHII
```

<400> 78
catcaggcca ctcgtttgtt ctcagacggt tttgccgact gcacgctgtg catccttgtg 60
ggcaaaaaag taagtttttt aaaatagact atgataaagc gtatgaaatc cttgccctca 120
gaggctttgg ctataaatgt atactatgga tttaaaaact acctggaggt cggtggcgcc 180
gccgcgctcc tcccgcaacc ctagctggac gccgctacca ccctcctccc gcaacgctag 240
acactgttgt tactctctct ctctctctct ctctctctct ctctctctct ccgccgttcc 300
ggaggccact gtcggacaag gccatgcggt gtctgtgatg agggcggcaa ggatctgatt 360
ggcgatgatc tcatgggtca tcgacaggtg cagtgcggcg agtcaatgga tggcacccat 420
cataaaggcg gaacgcccacc tcccacttaa agaatattcc gcatttaggg tttaaggcaa 480
tttactctcc tgacatgatt gtgcctgatt tcttggcgcc cacggtaaca caggccgcca 540
cat 543

<210> 79
<211> 551
<212> DNA
<213> TRITICUM TAUSCHII

<220>
<223> n means a, g, c or t

<400> 79
ctgtgtgtgt agggggttag gatttcggag caaggagaag tgggagagat gagatgagat 60
gagaagaggg cctagctgca gagacaagtt attggtggct agctaccctt ttataaggac 120
ttgtggcaat catatggagc gtgatctttt ttttagggta actgcatcca cgctcctcca 180
aaggctcaag gaccttggcc tgcagtctgg ttcttgggag gaagatagaa gggcaagtgt 240
gttgttggaa atcaaaagct ggtgagatgt ttcaaagctt ctttcgtttt taatggggat 300
gagagagaga gagagagaga gagagagaga gagagagaga gagagagaga gaggctttgt 360
gggaagaaat agtggcctac tgatgtttag attctttcct acatgaagcg tgtttgactg 420
aacaatttga cacccttgca ctattaggat tagacgagag agggaggga tgggcaaatt 480
gttggatgta gtccannnnn nnnnnnnnnn nnnnnnnnnn nnnnnnnnnn nnnnnnnnnn 540
nnnnnnnnnn n 551

<210> 80
<211> 480
<212> DNA
<213> TRITICUM TAUSCHII

<400> 80
ccctggttgt atgatcatta agatgtatac gtataggggt tttgaatcac tcctggagag 60
agagagagag agagagagag agagagagag agagagagag agagagagag cacgagtaat 120
tcactcactg tcacaagatg taaggtgacc agctcaatga tagaactgtg cccaacaaat 180
atgtagcttt cagaaggctc tgcaaatcca atgggatcat ggcattgtgc aaaaagacac 240
tgcgctccat gcctacgtgg accctcatgt gtgttggatc atgtgcgcac acaggcggga 300
aggtatatat gtgtaattag tataaccata ggcttcataa ctaaagaata ttaaagtaga 360
acagtaaata acataagtag gcagaagaat atcgacagca gcgggataag catgggctgt 420
tggtccaacg ctgaagtgct tagctattaa aacttcagga ataaaacgag aatatacact 480

<210> 81
<211> 496
<212> DNA
<213> TRITICUM TAUSCHII

<400> 81
taataatttg agagggccat ctaaaaacat tttagaggct ttagtgtgga ctgttatgtc 60
agctacaaaa tcgcaatctt tatttttcct accatttgtc tatgagacac ctaattattt 120
tcctttttcg tattattata tccccaatcc cctctttcta ctcggcacac ccactcggtt 180
gtgtgtgtgt gtgtgtgtgt gtgtgtgtga gagagagaga gagagagaga gagagagaga 240
gagagattat ctcagtgaca tgtaggccta tgccacctgg ctaaagcggt gccatgtcat 300
caagtcaaaa aaaaatgggt ttgaccgagg tataccacca ttggggacta aatctacctg 360
atattaatag ctgccatacg gagttgcaca aaataatgtt tagggacaag ccacaattga 420
cgataagttg aggcacgaaa atgacacttt gctctattca ttttgttatg cggcggaaat 480
gagaaaaaat aaataa 496

<210> 82
<211> 502
<212> DNA
<213> TRITICUM TAUSCHII

<400> 82
caagtgctga tgctgctgta agtgctgctg tgcaagtgct gctgctgctg ctgtagaagt 60
gctgatgctg ctgtgcaagt gctgctgctg ctgtataagt gttgctgctg ctgctgtgca 120
agtgctgctg ctgctgtata agtgctgctg ctatagaata agtgctgttg tataagttat 180
gttgttgtat aattgttgtt tttggataag tgcatctact ttataattgc tgccattgta 240
ttcttcatat attatcattc aaaaaaatac attttgcata taatttagtt tcaaacatta 300
catcaaagtt cattttcatc atttgtcaaa atggacaatt ccaatgtaga gaatgacctt 360
ccaaacaagc tcagaatcga gcctctcagt cgaattccaa gtggcaattc tgtgcacatc 420
caaacaacat aattcatgtt caatgtgaat atcaaagtca aggagatttg atattggggc 480
caattcaatg ccatggccct ga 502

<210> 83
<211> 541
<212> DNA
<213> TRITICUM TAUSCHII

<400> 83
ccaaaaggcg caagggaagg aaagggtgga tccttcggat cccttccata ctttgtgggc 60
acaagggaag gatggagagg acccctagag atcctcaaac cttggtgcga cgagactagg 120
ggttgcccct ctcccaagtc gcccccttg tcctattata aatagagggg acgggcagcc 180
ccccaaggac acacaagttc ctgtaaggtt gcaccctccc cttcctctct gtgtgtgtgt 240
gtgtgtgtgt gtgtgtgtct catctttcaa tgataggttg ctccacctcc accctagttc 300
tttggcactt cttcgtcctg gacggtgaag ccctactaga ttgatgatct cgtatttgcg 360
gatacaggcg aaggggtcat ttcttttaac ctagttcgag ggagaaaaaa atctatggtt 420
gggaagtttg atcctagcta cgggaatatg caccaatgat cttcatcaac tcttctctct 480
gctgcgtagt tgataaagat aagatctaaa catgccttgc atcttcatag cgatccttgg 540
c 541

<210> 84
<211> 384
<212> DNA
<213> TRITICUM TAUSCHII

<400> 84
ccccgccccc cacggccgcc atggatgttg cctcggtgtc gtttatgggg ctctcgcgca 60
tggcaacgag ctgcaagccg tagaacattg actggatgga gcggcgaagg aagcaacaag 120
agagagagag agagagagag agagagagag agagagagag agagagagag agggggcagc 180
tcagagagtt ccaaccaagg atgttttgga cctcgaggac gatgtgatcc ctctgctgcg 240
cccggttacc ccgcatctct ccatcctacc atcgacaatg aattccatac agatttacgg 300
aaacgcgggt gccagacgac gacgtgtgca gctagccgca actccaacgg gcatggacaa 360
gcacggccag cgtcggagcg cctg 384

<210> 85
<211> 907
<212> DNA
<213> TRITICUM TAUSCHII

<220>
<223> n means a, g, c or t

<400> 85
ctatgatgtt atgagtccaa gactccaagt tcttaattag agacagtagg cttaatgagc 60
gtcagtnctc ccgtgtgtgt gtgtgtgtgt gtgtgtgtgt gtgtgttttt atatnaatcc 120
tggacaatta atctagaggg ctatgtgagg gaatanggga ggctgatgca tgcttagtgt 180
agcgcgtgtg gggcttcatt tgcggnggaa gctactntat gtaatcatgc atcttatgtt 240
atgaaatnaa ggtgttanaa aattcctgtg cgtgtgcttg aaagcgtgaa cttgtncgta 300
tcaagatcgg cttaaacatg gttgcacagg aacttgtaaa atttatgcag actctgcagg 360
aaatgcttgg cagattttgc cattggcggt gtttattctc ttatcgtcgc tactntcttt 420
tgcgggaccg ccattcatac ttttatcatg tagtccacnn nnnnnnttna ataantttnn 480
tnnnnnnnnn nnnnnncnnn nncnnnnnnn nnnnnnnnnn nnnnnnnnnn nnnnnnnnnn 540
nnnnnnnnnn nnnnnnnnnn nnnnnnnnnn nnnnnnnnnn nnnnnnnnnn nnnnnnnnnn 600
nnnnnnnnnn nnnnnnnnnn nnnnnnnnnn nnnnnnnnnn nnnnnnnnnn nnnnnnnnnn 660
nnnnnnnnnn nnnnnnnnnn nnnnnnnnnn nnnnnnnnnn nnnnnnnnnn nnnnnnnnnn 720
nnnnnnnnnn nnnnnnnnnn nnnnnnnnnn nnnnnnnnnn nnnnnnnnnn nnnnnnnnnn 780
nnnnnnnnnn nnnnnnnnnn nnnnnnnnnn nnnnnnnnnn nnnnnnnnnn nnnnnnnnnn 840
nnnnnnnnnn nnnnnnnnnn nnnnnnnnnn nnnnnnnnnn nnnnnnnatn tgcntatttt 900
nanaggt 907

<210> 86
<211> 1115
<212> DNA
<213> TRITICUM TAUSCHII

<220>
<223> n means a, g, c or t

<400> 86
nacattattt gtccgtaaca atgccgacac actgatgatc gaggttgctc tggtctttcg 60
atgatctgtg tgtcggcaat gcatgaccgg tttgcggaca tacgatgtgt cggtataaga 120
tttttgctgt tacattatct gtcgctcttg ctttgcgata gaaagagtgt aggcatagtt 180
ttgggcctgg cccacctatt tcgggctgcg cggganaaan agngcgcgag ggactgtccc 240
ggtccgctca atcccccgcc ccatcttatc tcttactccc acgccacaat cccacctaaa 300
cctaggtcac ccaccgccgc cgccgccgtt ccctgacctg cctgccgatt tgctcgcgcc 360
gcctcctctc ccatcaacgg tggcgcttcc cgaagcttcc ttctaccatg tggtgcttcc 420
ccacggtcca agcccaaggt gccgcctcat cgtccgcagc agaggagccc ctccaccgcc 480
cgcagccgag gagtcgagtc gaccgccgtc gaggatccgc gccgccaacc gccagcgtgt 540
agtcgaccgc cgccgagcag cctcgccgtt agttcannnn nnnnnnnnnn nnnnnnnnnn 600
nnnnnnnnnn nnnnnnnnnn nnnnnnnnnn nnnnnnnnat ancngtntnn nnnnnnnnnn 660
cttttntnnn ctcnntnnnn nnnnnnnnnn nnnnnnnnnn nnnnnnnnnn nnnnnnnnnn 720
nnnnnnnnnn nnnnnnnnnn nnnnnnnnnn nnnnnnnnnn nnnnnnnnnn nnnnnnnnnn 780
nnnnnnnnnn nnnnnnnnnn nnnnnnnnnn nnnnnnnnnn nnnnnnnnnn nnnnnnnnnn 840
nnnnnnnnnn nnnnnntnnn nnnnnnnnnn nnnnnnnnnn nnnnnnnnnn nnnnnnnnnn 900
nnnnnnnnnn nnnnnnnnnn nnnnnnnnnn nnnnnnnnnn nnnnnnnnnn nnnnnnnnnn 960
nnnnnnnnnn nnnnnnnnnn nnnnnnnnnn nnnnnnnnnn nnnnnnnnnn nnnnnnnnnn 1020
nnnnnnnnnn nnnnnnnnnn nnnnnnnnnn nnnnnnnnnn nnnnnnnnnn nnnnnnnnnn 1080
nnnnnnnnnn nnnnnnnnnn nnnnnnnnnn nnnnn 1115

<210> 87
<211> 549
<212> DNA
<213> TRITICUM TAUSCHII

```
<220>
<223> n means a, g, c or t

<400> 87
gngagtaatt agcagataag agctaagcta tggtagaaag aaagaaaata gatgagattg 60
atccttacag gtcaagaacc gaactaacct agtgccctag agattaagtt gccggaacac 120
aagcaatctc aaaccaaatt aaggaacgca ggaaaaagac tgccgcatca tcagatgcat 180
ctgaaaatat tttttatcaa ctaaaaccta tcttttacat ggccgtatga tctttattta 240
taggtgcacg caggaaactt gggctaagcc cgatttaaat nacgacangg antggactta 300
acggaaatac aagaaaacac ttaacaggat aaaaaaaata caccgtaata aaaccaggag 360
accttcacat aagcttgctt ttcaggctaa acttgtagta gttgcttggg gttctaaagc 420
aagggtccac ttcatccgaa ggattaaatt agtgggcatc tcttcaatta caaccctggt 480
gatacttcga tccctgaata ctggcttttg catgccttgg atagttagtc cacgcgcctc 540
gagggatcc                                                         549


<210> 88
<211> 512
<212> DNA
<213> TRITICUM TAUSCHII

<400> 88
cagaaattat agcatgaagt tgaaacaaaa gcacctgaaa gatcaagggt tgtttgtctc 60
ctgtcgcgat gttgtcgtga gaatataacc atgacaaaat aacacaaatc caaacccttt 120
gccattttac ccttgttgat ctccttcccc agcacacctt ctctctctct ctctctctct 180
ctctctctct ctctctctct ctggctactg gagggctagg gagcagaact agatggaatc 240
agaatatcag atcgtatggg gtgttgggag aaaaaccaac aaacctataa atttcttagg 300
aaccaaaaga ttgtgatctt ctcggcacgc catcgtctgg tggtggggga agaggaactt 360
cctggtgttg ccgtcatgag agaatgatcg atcggcatga ggatgaagaa acgaaacata 420
cagacgccga gccgcatcga agcttggacc ctggaggcgc acgttacctc ggacgatcga 480
gggtcctgtt gcttgtatgg ctcaaaggcc ca                               512


<210> 89
<211> 159
<212> DNA
<213> TRITICUM TAUSCHII

<400> 89
aaaactttca atcctgactt taaagccttt ccaagtgaat ctttggtgtg tgtgtgtgtg 60
tgtgtgtgtg tgtgtgtgtg tgtcctgctc aacaaaactt tcgctgtcat ttcattggta 120
aatgatatgg aggctgacct ccaaaatatc atacagatg                        159


<210> 90
<211> 540
<212> DNA
<213> TRITICUM TAUSCHII

<400> 90
cagagtaaac tgaatcgatc gaccgaagag agcagaatag agagagagag agaggagggg 60
ttgttaccag attatctttg tcgtgggagg cgaagaggag gaacttgttg gtgccgccgt 120
cgccgtggtc gtggttccag tagaagtagc gcctccactc ctcgcagtcc ccgcagtcgc 180
agccggcccc gtgcgccccg ccgcagtctg cacattgacg cacgcacgga gaccgccgca 240
ggccgtgtca agaaaaggcg gccggcgtct cccaaagcaa ggagaacgaa atttcttgca 300
agaaaaggac gcattcttct ccggtcgggg gcatcaagaa ggatgggggt cacctgggtt 360
gaagagctcg cccgtgtcca tgaatcgccg ccgccgccgc cgtgcagaat ggagagacgg 420
acggacccca agctgcttct tttctttggt tgatcgtatt gggtgtgggt ccgcggcagt 480
gcacagtgag ctgagcttcc aagccaaatg ggaaactgcg ggcggtggca ctgagcgccc 540


<210> 91
<211> 409
<212> DNA
<213> TRITICUM TAUSCHII

<400> 91
ctatggtaat gtgatttcaa tttctcaatt tatggttttc tagattcgaa ggcataaaag 60
aagagaaaga atcttgtgga agggtttgat gaagaagaac aagttgaatc atctctctct 120
ctctctctct ctctctcaca cacacacaca cacacacaca cacacacatc catccacatt 180
tgttcctgca ttttagtttt gatgttatgg tttctcaatt tatgattttc cagatatgag 240
acagaaaaga aagaaagaat ctcatggaag agtttgatgg agaagaacat ggcaaaccct 300
ctctctctct ctctctctct ctctctctct ctctctctct ctctctctct ctcagctatg 360
tttggtcctg cattttgct ttctcagttt atcgttttct agatctgag              409


<210> 92
<211> 448
<212> DNA
<213> TRITICUM TAUSCHII

<220>
<223> n means a, g, c or t

<400> 92
cttgacctca cgggtggttt tgctcagtcg ccgccgccca gatccaaatc gccgccgcca 60
ccgccgttgt tgttgtgttt gtgggagacg tgggattagg aattatttgc ccgttcagtt 120
ttttcgttag cgaccagaat tgcgtgattg cggggacgtg ggactggtag ttagaatatt 180
tctccgctgg tggagttagt ccannnnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
```

nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnn 448

<210> 93
<211> 518
<212> DNA
<213> TRITICUM TAUSCHII

<400> 93
cctcaaacct caaggcattc atcaagatgc agttaagacg tacgccattt taagattaca 60
ggtcagaact cagaagtgtt ccgagtaaaa agaagcaata ctttcctaac tataattatc 120
agttccacat ccaatgaaat ttaacaagtc aacatgtttc agtcctaaag gaagcacttt 180
gcccattaca acaagcacca ttcaagataa catgaatgta aaaaacaatg gccactctca 240
catctgactt accacaacaa caacggaaaa cagagcacat aacctagaat agctactgaa 300
aatacataac tcccccgcag agagagagag agagagagag ggagagagaa accttcagct 360
gagaaatgtg aatcaataac cactgtcatg aggtgctgct tgagatagac ctgttaccaa 420
ccaatgaaca tgtgttagat catgaagagt ttaaaatgaa gaaccatggt gtataaccag 480
tagtaagatc actaaaggcc aattaactag agttagtc 518

<210> 94
<211> 534
<212> DNA
<213> TRITICUM TAUSCHII

<400> 94
ataaacacca gggaggtcca cgtctatggg gcgccgcaga acgacgacga ctacaacggc 60
gaccagaaga ggcgtggtgg cggcggcggc gggggcagcg ggttcttcgg tccggccttc 120
catgccgtcg gccatttcgt cgatcgtagg ttcggcctcg acgacaggaa ctgatcgatc 180
acgcccatat atatggctga ttgcgctggc catgcagata ggcatggatg ctgacgacga 240
tcgagttagt ccacgcgtgg actacattta ccatcacatc atctttgaca agaccaaagc 300
aagcataacc tgagagcgag agctgtggag gcaaaccaaa cagcccctgt tttggcacgg 360
atcacgtgca gttcagtgca gagtatcgtg tattttgtgt gtgtgtgtgt gtgtgtgtgt 420
gtgtgttttt gataaaacga acagctgagc ttggtggaac ggaacaaagg caaacattgg 480
tttggtatat aaatgcgatg gatgcccgct tgtgagagca atggagtcgg acga 534

<210> 95
<211> 534
<212> DNA
<213> TRITICUM TAUSCHII

<220>
<223> n means a, g, c or t

<400> 95
ccaaatgcta atccccgccc ctctctctct ctcacacaca cacacacaca cacacacaca 60
cacacacaca tcaatcacct tgtttgtttt cttgacatgg cctttactct tctcttggat 120
tcataatatg ccacctgcga ccttgtttac agcttcttga tacatccact aaataacgca 180
gataagctac aatctgttag gggtagtcca nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnt nnnnnnnnnn nnnnnnnnnn nnnnnnnnnn nnnnnnnnnn nnnn 534

<210> 96
<211> 519
<212> DNA
<213> TRITICUM TAUSCHII

<400> 96
cctggatctc ggggatgtcc tcggagagag agagagagag agagagagag agagagagag 60
agggagagag agagagaggg gggagagaga gagattgtca gtgcgttcca gctgctctgg 120
aaaaaaaatc atgtcacttc tggtttttca tggtctatcc taccaagagg ctgcagcatt 180
ccaccatgcc ggtcaacccc atgaagaagg tgcttaacga tgaaaacctc ctcatcgaga 240
tcgtccttgg ccatggattc cccaccaccg ttgtctgcaa gttctctgcc aggcctttaa 300
ccccaccttc ttctgcatga tccacccacc acgcctcctc agcttttaca tctacaacct 360
ccaccaccca ttcttcgacc cgatgctgcc ttagcccctg gattttgccg ccatctccca 420
tcatgtggca agctgcaact acgatgcctc tcaccatgta tggatctagg actgccagaa 480
ctgcaagatt cacattggcg gtagcggtga tgacatatc 519

<210> 97
<211> 527
<212> DNA
<213> TRITICUM TAUSCHII

<220>
<223> n means a, g, c or t

<400> 97
actatgaccg actgaatttc ttcttcttct ccgtcttgtg aaagctttct ctttcagaga 60
gagagagaga gagagagaga gagagagaga gagagagaga gagagagaga gagagagaga 120
gagagatgga tgtgtattga agttgcaaat ccgcaagtcg ttcagataaa acgacgttct 180
cctacctttt gaaatcaacg taaagatccg agagggtgag gacatgtagc tgtgtatacg 240
tgcattggat cgataaccta gccatggtaa gccacaaaga aaacggaacc gcattcggca 300

ttcaatccat caatcaatgt atcacgggtc gggctggtag gtagcacctc ggagtcgatg 360
gagaggtgtc gtggacgtgc atccacgcag cggccgttcg cgccaannnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnn 527

<210> 98
<211> 503
<212> DNA
<213> TRITICUM TAUSCHII

<220>
<223> n means a, g, c or t

<400> 98
ggcgacacct tccgcgaaaa attatgcaac aaatgcaagc gtcgcgtgag catacaagnc 60
tttaagcgtc tcctgttcgc tgcaaatcgc ggccatggac gacggggacc tggacttctc 120
catgggcagc tacttggacg acgtcctcgg gggcacagcg gagcacctcg cgtgctgcac 180
ccacacccac acctgcaacc cgccggcata cgacctcccc cacacccaca cctgcctcca 240
cgtccactcc aagttggccg cctccgcctc cttcgacgcc gccgccgact cccacgccga 300
gcctgaggac gcccacgcca cctcccggag caagaaacgc cgcccgtcgg gcaaccgggc 360
agccgtccgc aagtagtcca nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnn 503

<210> 99
<211> 510
<212> DNA
<213> TRITICUM TAUSCHII

<400> 99
gtgctatgcc taatcagggt cgttagctcc acctccactc gggaggaagg tggtatgcta 60
tgctgaagac ctgtcagtga tgcatctgca ttaggtgcta caggcccaaa gttcttcaac 120
attcagaaaa aaatgcagtt ctatcggaat acacgatatt atagtgcatc ctactggaag 180
tctggaacca ctcccgttcg cctgatgagc agcaaatttg gttgagtttc ttacacccaa 240
attcagtgtg attttcctac gtcagttttc agcgtgacct gtgcatgcac tgagatctcc 300
taatcctggc gcatcgacaa atttacatgt cgctataact ctgacgtatg tgctctgagt 360
ccggtccaga tcaccaggat tcgacgggat ctctctctct ctctctctct ctctctctct 420
ctctctctct ctctctcttc attgagagtg tggtctggtt gctaacctgg taactaaggc 480
cctgttcgga agtagtccac gcgcctcgag 510

<210> 100
<211> 534
<212> DNA
<213> TRITICUM TAUSCHII

<220>
<223> n means a, g, c or t

<400> 100
ctagcagtgt aataaaaggg cgcgcttaca agaaggaagg aagaaagaaa gaaagagatt 60
tacctctgcc aggttggttt tcaccattgc tcttgttgaa catcgggaat tgattctgct 120
tactaagcct tttaccaaaa gagttgtgcc acatggttaa gtccgtgcac acggattctg 180
gtgcgaatac acctgtatga actatatgtg ggtaaggttc attagctcga tgacaagagc 240
atatagatag gaaccagttt agtttacagt ataagaccag tgctatgttg ctagcagaga 300
atataaatag ctcgatgaca caacatctaa gccatctcaa ccaatgtgtg tgtgtgcgtg 360
tgcgtgtgcg tgtgcgtgcg tgtgtgtgta tgtgaacaag ttagtccann nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnn 534

<210> 101
<211> 542
<212> DNA
<213> TRITICUM TAUSCHII

<220>
<223> n means a, g, c or t

<400> 101
ccatcttcta ctggctgcga ttgcatccac tactgcggct aggtctctgt agtggtagct 60
ttgaatccgt ttaaataaag aaattactac agtttgcttt ctgcacccgg gtcgtattct 120
tcggcgccga gcgtgataac acacgtcgtg aaaaggaaaa gtaaaaaaga aaagcccctc 180
ccatcccctc ccctcccctc cccctggcta gggagacacg gaaaagggcg aggagannnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nn 542

<210> 102
<211> 432
<212> DNA
<213> TRITICUM TAUSCHII

<220>

<223> n means a, g, c or t

<400> 102
tccctattaa attgtagttt gtttagcttt tgtagcagga aaggtaaagg gcaaagaaag 60
cgaggaatcc aatccaccta gccaaagggc aaagggcctg catgcatagg agggagaggg 120
aaaggtaaag gcgtgcacgg tagcaggaaa cttcactagt tgccccggca caaaggtaaa 180
gaaagcccat gaaacacaaa tcagcactga tgagtgcgtg cattttgctt cagaggtctc 240
cactcaaaag taaggcatct tcgcgtctcg tccctgcaga ttaagaacgc agaagccata 300
tgccgtggct ggagattcag gttccccctc tctctctctc tctctctctc tctctctctc 360
tctcctcgnt gngctgttca cgtcgggtcg caacgaccac gaggccgacc cgtccaaatc 420
tatcatggac cg 432

<210> 103
<211> 480
<212> DNA
<213> TRITICUM TAUSCHII

<400> 103
ctctgattct atcggttggg aggttgccgg aaattttctg catctgctga gcagatcgtg 60
gttagaagac cattctactc gccctgctta ccattttcct tggatccact caccgtgccc 120
gggtgttcta ttctcctgct tccagtgaat attggacctt ttttgggttc aacgagcatc 180
cgcaggcttg gcccttcttt tgtgtgtgtg tgtgtgtgtg tgtgtgtgtg tgtgtgtgtg 240
tgtgtgtgtt ggggggatcc gcaggctcag ttcatccgac gaagattctt gacgtttaat 300
tcaggacgag aaatgaataa ctcttcttgt ccctgggttg tttccttcca cctttttatc 360
agagaaaagg ccatgaaggc gtttccatcc acttccctgt atccattact ttgaactact 420
ttagaggggc aagtgctttt taggttgatt ttgtggggtt taccacaaga ggaaaacgag 480

<210> 104
<211> 488
<212> DNA
<213> TRITICUM TAUSCHII

<400> 104
cctctgggtc agccttaaac tcttgcactg cttcttctct atcgtcaagg gacattgacc 60
catcaagcct tctgaagttt atgaaattgc tattcagtga aagctccagg acgttcagca 120
taccagtcca ctgggagaag actattgcct taggagtatc actatcagta ggaccatgtg 180
tgttgaagat tgatttaagc gtgtcaacgg cagccttgat cttcgatgag atgtaactgg 240
cttcgcaaat cgaggatgat tcatctgctg ctgctgctgc tgctgcagaa ctatctgctc 300
ctgatgctgc tgcagaacta tctgctcctg ctgctgctgc tgcagaacta tctcctactg 360
ctactgctgc tgcagaacta gttattgcat atgactccaa ctcatcagag atacagagcc 420
ttaatactgg gcgtgaaaaa agcaattcag aacttaattt ctgtttgcaa acagggcaga 480
cttcctct 488

<210> 105
<211> 447
<212> DNA
<213> TRITICUM TAUSCHII

<220>
<223> n means a, g, c or t

<400> 105
cctgtcggac gacgacgagc cggcagcagc gcctggcggc ggtggcggtc tcgaggcgcc 60
cctgctgtcg aggcagagca cggaggtgga aggtaagacg aacgccaacg atccacggag 120
ccagtctatg cagaggttca gcagtatggg cggcggcgtg gacgcggcaa gcaccatggg 180
catcggcggc ggctggcagc tggcgtggaa gtggaccgag aaggtgggcc ccgatggcgt 240
gaagcgcggc ggcgtcaaga ggatgtagtc cannnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnn 447

<210> 106
<211> 496
<212> DNA
<213> TRITICUM TAUSCHII

<400> 106
ctagttcata atatggagaa aaataattac ttgtaagttg ctgctagcaa ccagcagtag 60
aagaattaat acgtactcgt gagggatgtc gcttgccttc ctgagagttt gttgtgttga 120
ggacgagata tggcgcatta tatgtcgggg acactctctc tttttctctc cctctctctc 180
cctctctctc tctctctctc tctctctctc tctctctctc tctctctctc tacattgggc 240
taccaaagga tctgattccg atgaatgacc tgatatatat ggagatggaa tttccacttt 300
ttatgtgtca agtcgatgtc cccaagcaag gaaattgctg ccagccaggg aaagtatcca 360
ttgttgagct tcctcttatt ccaacggctg ctggacaaga gcacaaagaa gcatcccatt 420
ggtctgctgg taagtaaaga aaagcatccc atcccatgca tttgaggagg ctgctggtga 480
agacaacgta gtccac 496

<210> 107
<211> 537
<212> DNA
<213> TRITICUM TAUSCHII

<220>
<223> n means a, g, c or t

<400> 107
acttgcactt gctatactta cgaatatgtt accaaaatta cgaagtaaca ctaggttcac 60
acacacacac acacacacac acacgcacac acacacacac acacacggac acacctatag 120
tcctatacac cttagtttag tgattagaag tagcagttgc tttccacaca ccgacacaca 180
tacatcttaa tgttttaaaa tgcagtgttg atgtttactt ttatgaatta ttaatcgatc 240
tgcttgagtg tctcttgggc gcaatgaatg gttagtccan nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnn 537

<210> 108
<211> 551
<212> DNA
<213> TRITICUM TAUSCHII

<220>
<223> n means a, g, c or t

<400> 108
cacattcaga actaatttaa agtgatccat aggaattttt gacaggtcaa aggaatagat 60
agttagtgca agaatataag cctcttcaga gcaaacaaaa attgcaataa gtttaattac 120
ctttgggact aatcttagtg aaaaaagagc tatatataaa cctgcaataa cattcagaat 180
ataatggctt cactgtttgc cttattaact aatcagactt tggccaaata cctaccactg 240
tcacacacac acacacacac acacacacac acacacacac acacacacac acacacaaca 300
gttggtaggt ttgaggttat acaggaaaaa aatcgaagtg gggaaatatt aatgctttac 360
taaagaagaa gaataggacg aagaaacaat agtgcaatct cactattaca cttgaatgct 420
gggaccattt tccatcagtt agtccannnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn n 551

<210> 109
<211> 526
<212> DNA
<213> TRITICUM TAUSCHII

<400> 109
ctttttggag atcattttta tatttgattt tttttgaaa cacctcaata tatgaattaa 60
ttaaaatatt cttataatca ttcattacag ctaaagccac ataggacgga aaatataaaa 120
aatcatatat gtctattacg gaaaaaatag attgatagat tgtggttaca aaaaatatga 180
ccattcatgt ccttaaaaag gaaaatatgt tttgctcata cacatgtttg aacttttaac 240
caaaagtttc atatttttaa ccttttaaag tatacatcat ccatagtata tgtgtgtgtg 300
tgtgtgtgtg tgtgtgtgtg tgtgtgtgtg tgtgtgtgtg tgtgtgtata gccttgcaaa 360
atcttgtgca agcgacataa gcactgtgca aaccaacata aacattattc aagagatcaa 420
aagtcattac aatgagccga aagttaggct cgtctatggt cacattgtca ctagtgacaa 480
atttatacta cttacgcaac aggatacact tcagtgatac tttttg 526

<210> 110
<211> 545
<212> DNA
<213> TRITICUM TAUSCHII

<220>
<223> n means a, g, c or t

<400> 110
tgctctgcca atcagaagcg ccattgttac ataaacatat gaatgcaaaa caacagtgtg 60
tgtgtgtgtg tgtgtgtgtg tgtgtgtgtg tgtatgagag agagaggggc gaacctttttg 120
tagaacattt aagaaaagta taattacatc ttcattctgt ggagaaaaag aaaacatcag 180
ggtaaagaat caagttacaa tgcaggttag tccannnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnntnnnnn nnnnnnnnnn nnnnnnnnnn nnnnnnnnnn nnnnnnnnnn 540
nnnnn 545

<210> 111
<211> 503
<212> DNA
<213> TRITICUM TAUSCHII

<220>
<223> n means a, g, c or t

<400> 111
ctgctgctgg ctgccccttg tgccgtcgct gctgtttgcc gctgcagccg ccgcctgctg 60
tgtcgctcat gttgctgccg ctactgtctg tcgatgccgc tgcttgctgc cgccgctgct 120
acggtcggcc atggccgagc tgtgagtgta gtgtgtgtgt gttctgcata gtgtgtgtcc 180
gtgcagctgg acatggccgt gtgcgcccgt gtgtggtgtg agcagcgtgg tgtgtgtgtg 240
tgtgtgtgtg cagtgtggtg tgtgagtgtg ccgtgcgtgt ggctggctgg gctacgccca 300
gtagtggcca ggcccttgct tagattagtt agtccannnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnn 503

<210> 112
<211> 551
<212> DNA
<213> TRITICUM TAUSCHII

<400> 112
tccttgagct acaagaccac acctccaagg agaaacaacg gcatgacgcc gccgccgccg 60
ccgcctggtc catagccagg accaagagtt ttccttaagc acgaggaagg aagtcggatc 120
ggggccaaag caacgcattc aggaagggga cggcgtccac gaaccgttgc tagcctcagc 180
atgtgaagct gggatttcac ccggcccaag atcacaaaac ccaaaccgga gttgttcaag 240
caggccgacc actgcaacgg atagcgtcac atcgccacac agcagccatc accacgccac 300
cacctgacgt gtcatcggtg aattcactct taaacacatg gtaaactgcc aattagtgct 360
acatgtcatc acctgccctg aaagtgacgg ttaagttgaa cgaacaatgg tggttttcta 420
ttaccctagg tcactgtttt catgcaattt actctatcca ccgttctacg gagcctaatc 480
ggtgtctcgt tccagcattt aatcgcaaat tgcattgtgt ggcgctccct gacaaaccgt 540
ggcaaccgtg t 551

<210> 113
<211> 547
<212> DNA
<213> TRITICUM TAUSCHII

<220>
<223> n means a, g, c or t

<400> 113
cacaagaaga ctgcacgcaa ggataggagg ctgccatgca tggcctgggc tttataggtg 60
tggcgatacc cataagttgg tgcaattgcc aattatccgt ggccattgcc ttgtataaaa 120
atcattacat tgttgctgtg taagagaaaa tcatcgtgca accatgtgtt cgctggtgta 180
atgagaggga atggagagat gcaaagagat gtgcgtgtct actgcaagat gagagagaga 240
gagagagaga gagagagaga gagataacac gaagatgctt tagtgcaagt gtttaattta 300
caagttgctt gtgtcctact cagtagtcca nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnn 547

<210> 114
<211> 489
<212> DNA
<213> TRITICUM TAUSCHII

<400> 114
ctggaggtgg ttgctgccta gatccctggt cgccgccttg cctcagtgcc tccggcttgc 60
ggccatgcag ccgccggccc gccgagcacg gctatgatga accgccgagc cgccgcaacc 120
tgggaggaag agaagggagc tgggacagag cgctaggttt tctggttgag gtgctctatc 180
ggggtaaaaa tttgggccga gatcttttg ggcctcttat tcggtccttg cagcgatacc 240
agcgaccgct aaggctttta gcgctgtttc gcgtcgcgat cgcgggcctc gcggcagatc 300
gcgcaatatt ggccatagcg tcgcgggaag catccgaggg agcgattgcg ccgcggtagc 360
tgcactttcg cgggcgatct taatctttga aaaaaaggca gaacatcctt ctctgaaaga 420
tagtttaata tttatatagt agcctgtttt gccacgagat aaatgatcgt tctcccaaag 480
aaacaggag 489

<210> 115
<211> 519
<212> DNA
<213> TRITICUM TAUSCHII

<220>
<223> n means a, g, c or t

<400> 115
ctactctagc cagagtgtat tacaggtgag gccagtgggt ttagtcaaca gttgaccaat 60
caagattgac tggtcaacaa ggtcaaagca gggcccatct gtcattgact ttagtttaac 120
aaaatggtaa ttaacaggga tgatcccaca tgtcatttac ttaggctaaa ttaacactaa 180
ataaatacta attaatccta gacctaaatc taaacaaggg ccagcctcaa cacagccagc 240
catcaggctg gctcacacac acacacacac acacacacac ctagtataca cacagagcac 300
acacggccat ggccgttgcc gtggccagca acagcagcag ccggcggtag tggcgcagcg 360
catccgctaa gcgagcaaca caagctgcgg cagccagcat gcagtagcag aagcagcaat 420
aaggggcaac agttagtcca nnnnnnnnnn nnnnnnnnnn nnnnnnnnnn nnnnnnnnnn 480
nnnnnnnnnn nnnnnnnnnn nnnnnnnnnn nnnnnnnnn 519

<210> 116
<211> 527
<212> DNA
<213> TRITICUM TAUSCHII

<220>
<223> n means a, g, c or t

<400> 116
cagtttgaaa ttctgaaaac atgacttgaa cacacatgca tgtttcagat tttaggtctg 60
aaaaaatgaa aaagagctac aatgaccagc agataaaatg tcttgttgta atgtaatgtg 120
tgtgtgtgtg tgtgtgtgtg tgtgtgtgtg tgttcatttt gaagctatgt tcgagcttga 180
aagtcgtttt ttgacagata catgcttctc ctgacataca ctgcttgtta gtccannnnn 240

nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnntcttcc gcttcct 527

<210> 117
<211> 862
<212> DNA
<213> TRITICUM TAUSCHII

<220>
<223> n means a, g, c or t

<400> 117
attgaaggac tacttctaat actaccacaa actttgatga atgattactt tatatgtgtg 60
tgtgtgtgtg tgtgtgtgtg tgtgtgtgtg tgtgtttgtg tgtgtgtgtg tgtgtgcgtg 120
tgtgtgtgtg tgcgcttata aagcagtatt gcaagaaaca caaaattgat gccctcactc 180
attagattat atcatggatg ctatagtcta ctggtgtagt cgttgatcaa aggtttaccc 240
taacaaaacc taatatgcaa catattttca aatgtcatga agcatagcgc acactagact 300
atattttagg aagtaacata tttgaattac tgaagttatt ctttttctg ttaaacgatg 360
cccgtgctac tcccttcgta cggaaagaaa ggagcaaaaa ctaccctaaa agaaggttta 420
aaatcttggg cgtgggaaat aattatttc catgtttacc cccgagcccc aagctaattg 480
tttaatgcaa gacttttttg agatggagaa tgggatacct ggagttatat ttgcccattg 540
tagcacannn nnnnnnnnnn nnnnnnnnnn nnnnnnnnnn nnnnnnnnnn nnnnnnnnnn 600
nnnnnnnnnn nnnnnnnnnn nnnnnnnnnn nnnnnnnnnn nnnnnnnnnn nnnnnnnnnn 660
nnnnnnnnnn nnnnnnnnnn nnnnnnnnnn nnnnnnnnnn nnnnnnnnnn nnnnnnnnnn 720
nnnnnnnnnn nnnnnnnnnn nnnnnnnnnn nnnnnnnnnn nnnnnnnnnn nnnnnnnnnn 780
nnnnnnnnnn nnnnnnnnnn nnnnnnnnnn nnnnnnnnnn nnnnnntttt ccctttctgg 840
ctaattgatc cgtgtccttc gc 862

<210> 118
<211> 543
<212> DNA
<213> TRITICUM TAUSCHII

<400> 118
caatatgtca ttcccaaaat tgatgccatt taccgtgact tccaatagaa ttttatagtg 60
cacaaatttt gtaaggggtg actttagaca atgcacatta aattattaca tgaatttttg 120
gtggcctgtg tggagagaga gagagagaga gagagagaga gagagagaga gagagagaga 180
gagagagaga gagagagaga gagagagagc aatgcacatc taaaagggaa atatatatga 240
cattttgatt catgatagcg aaggaataga ttccatacga attcggtaaa cctaatccta 300
tgaatccaat ttcttccgta cacaaaaatc cttagcattc aagtcctcta gaattcccat 360
ttgaaggaac ttattttttt ccaaaagtcg gtgcagtgat ttatctttgg aaaaaacgtt 420
tgcgcgtaaa aataaattga tactaattaa gatcttacaa gaatttaaat aagatgtaaa 480
tacgacacag cataaacaac gctcataata ggtttggctt ggagaatact cccttcgtaa 540
act 543

<210> 119
<211> 519
<212> DNA
<213> TRITICUM TAUSCHII

<220>
<223> n means a, g, c or t

<400> 119
cctgaggctg aggaggagcg gcgcgcggcg gcggaggttg aggaggacaa agaggcggcg 60
ggcgggggcg agaccgggaa gggacgacgg ctgcggtggt aaaagaagca gcgggtccga 120
ctgcgggagg gagggaagag tatgcgtccc gtgtctgccc tgctgtattt atgggctcgc 180
tcgtatcgta tgcgcttggt agaggacgac gcggcgacag atgggaattg gatcttgtgc 240
tccatctgtc cgggctggct tgccgttgcc ggttccaact tgcccttctc cctagactag 300
actagactag actagcccga ccttgatcgc tttcttcctt tctgccgacc tccttttact 360
gttagtccan nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnn 519

<210> 120
<211> 550
<212> DNA
<213> TRITICUM TAUSCHII

<400> 120
ctctcgttct ctccaggctc ctcgccgccg ccgccgccgc tcgtctcagc agccaccagc 60
cgccgccatc gaccacttcc tccactcctc ctcggtgtcg actaccgagt tgaagttgga 120
tgatgcgggg gacgaagctt ccgtgctcgc cggaggcagg gccgaggtgt ggagggcagg 180
aggagcacgg ccccgacatc cgcaatggca agctctcctc tctccttcct cccttccctc 240
tcatgcacca gcgcacctcc acagcacgca gcgcaccact cagacatggc cgccgccggc 300
cgtgtccgcc ctagcttaga acccggctta ttacaacggt caggagctca gcgagatgct 360
ctgttgctct tcatacgatt agattacatg tttctgcgtt caagtctact ctgtgtccat 420
attagccata attaactaac taaaatcacg cttgcagatg gattggttgc tgcttcttca 480
agggttaaga gaatgaaggt ctcgcactgc cggcaaaatt gttcgtcaac tgacgatgca 540
aatggccaga 550

<210> 121

<211> 489
<212> DNA
<213> TRITICUM TAUSCHII

<400> 121
ccaatccctc ctcccctacc cttgtcttct tcttcatctc caacatctaa tgtctgcttg 60
tattgtgtca aggaattcaa gggcatgtgg gagagagagg aggtccatgg agaagatgct 120
ctgctccaac cctccctgcc tcctactaga agatgtcttt tttgtgtgtg cttgtattgc 180
agcagccatg tgcgtatcaa aaaaagggtc cagtttgcag cagtcacatg atgtggacat 240
gtttttttcat cacaattgga tttttggatt tactaggcta attggttgtt gttgttgttg 300
ttgttgttgt tgttgttgtt gttgttgttt tagcagcagc agcagcaggc cgagtgacgg 360
tggtggctgt gtgcaggcca tggaggcgat gatgagcatg aggggaaggc tgctgcttca 420
accccgttaa ggtctgctgc tttgaattgt cccagatcgg ttgatttctt cttgctttcc 480
cgttaagga 489

<210> 122
<211> 432
<212> DNA
<213> TRITICUM TAUSCHII

<400> 122
gactattatt cagactacgt atgggaatcg agatgaagaa catgaagacg aggaggccgt 60
cctggaggcg gcttgcgtcg gcggcggagc ggcggaacgt gaggcagctc atcaccctgg 120
ctccaaaccg gcaacaccca atgggatccc ctgcctcctt ccttcctccc tccctcaccg 180
gcggcggcga gaagcaaaga tgctttcttt cttccggcgg gcggtggctg gagaaggcag 240
aattgatctg tgttgcgtgg cttgggagga aggtggggcg tggcgttata tagagcggag 300
gagggaaggg gaggggaggt cggcctgggc acggacggac ggacggacga gggcggctcg 360
gagaaggaaa ccggctgggt gcggggtcaa gatattttcg ttcgcagtgg gccactggat 420
tttggctagc cc 432

<210> 123
<211> 509
<212> DNA
<213> TRITICUM TAUSCHII

<400> 123
ttggtgaggt gattcttggc ggtggcgccg agccactcca gttggcggca gcggtggtgg 60
aatcaattac ctcactcgag gcggcagcga ggcaagtcga caggtcggtt gaggcgactc 120
caggcggcgg caaggcgagt cgataggttg gtcaggtgac tccaggcggc ggtgggtcga 180
tccaaagctt cctcgtgcga tcgaacagag agcgagagag aaaatgttag ggttattcgt 240
ggctcctggg cttctattgg gctcggggct gcgcttaacg tcctcgccgt atcaagctgt 300
gtccatacag agccgtatcc tgcgtaaagg ccgcgtgcag gacgctgaca cggcaaatct 360
gggtgggttg gtgcttcaga gtggatcagc tgccaactaa tactccaaat atgccaaagt 420
tatcaactaa aacttgcagt ttttagtctg ttttactcaa tacttgttaa ttctgttctg 480
ttacttggaa ccggagcatt ccggtgttc 509

<210> 124
<211> 838
<212> DNA
<213> TRITICUM TAUSCHII

<220>
<223> n means a, g, c or t

<400> 124
ccaagcaccc aagcagtaat agagcttctc aacttgcaac tgccacgtat caccgtggag 60
caactcaaat ggatgcacca aatgcaatgg caagggcaca cagagagccc aagtccttct 120
ctctcaaatc ccactgtagc aactaatgct agggaggaaa acgagaggaa gaacaagaag 180
gagaacacca cgaattccaa gatctagagc caaggggatc ccctcacata gaggagaaag 240
atattggtgg aaatgtggac ctagatcttc tctctctgtt taccctcgaa aactagcaag 300
aatccatgga gggattagag agttagcaag ctcgaagaag gtcaacaatg gaggaagaac 360
acgagctcaa aggattaggg tcattgcgga agaagacccc cttttatagg agggggaaaa 420
tccaaccggt aagtgctcag cccgcacacg agcggttagt ccannnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn nnnnnnnnnn nnnnnnnnnn nnnnnnnnnn nnnnnnnnnn nnnnnnnnnn 600
nnnnnnnnnn nnnnnnnnnn nnnnnnnnnn nnnnnnnnnn nnnnnnnnnn nnnnnnnnnn 660
nnnnnnnnnn nnnnnnnnnn nnnnnnnnnn nnnnnnnnnn nnnnnnnnnn nnnnnnnnnn 720
nnnnnnnnnn nnnnnnnnnn nntctttcgc ttgctggatc actgaatatc tgagctcggt 780
cgcttggatg ctgcgagcgg cattaatctt actcaaaggc tgcaatacgc gaattccc 838

<210> 125
<211> 511
<212> DNA
<213> TRITICUM TAUSCHII

<220>
<223> n means a, g, c or t

<400> 125
attgtgaaaa caaaacccat ttgcgcaaca cctacttgtt cggtgatcag ccttccggcc 60
cggggcacac acacacacac acacacacac acacacacac acacaactat atattccggc 120
accatttgct accttcatcg attttctttt cttccaaagg cactgaagct tcgtgcttga 180
cgcacgcaag caacatcgaa gaaaagagta agaaggaata aatcataggg ccctataatg 240
catggcatgg ttagtccann nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420

nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn n 511

<210> 126
<211> 520
<212> DNA
<213> TRITICUM TAUSCHII

<400> 126
ctctcgctta cgcctccgtg cgtcgtgtag cagctccacc ggcaccggcg caggcgctgc 60
cgagctggag cagacggtgc actgcagggt gcggggaggg agtcagggag atgacgcatc 120
gggcgccagt cgctggaggt gcgggagctt gctggcaagg tgggtgcggc agctccggag 180
tggagccggg agcagtcgga gctccgaagg aattagggat tgggattcag gctttcagcg 240
gcggcggcgg cggcggcttc gagctcgagt gggctggacg acctggacga cagagagagg 300
cagacgtggg caatgggctg gaatgaatgc ctgggcttag tcatgagtgg gctttttcct 360
agcttattaa acgagattcc ctttctaaaa aaaaatccaa cggagaattc tcgaagaaaa 420
aaactcgtcc aagggagctg caagtttggg cgcaaaacag cgagatgaga aaatcattaa 480
gccaaccaca atactgcacc gaaaaaaaaa atacaggtta 520

<210> 127
<211> 439
<212> DNA
<213> TRITICUM TAUSCHII

<220>
<223> n means a, g, c or t

<400> 127
taggaagagc tcagtctacc agagcatgtg tgtctcctcg tgggagctcc ggccgcctgg 60
gttggtcaat gacgatggcg caggggcggc ggcgttgctg ccgaggctcc gatcccatac 120
ccatgggatg cccaagctct caggctgcgg tgttggtgtt gatgatggct agcatgaagg 180
cgatggccgg cagggctaat cgagattccg gtctcctagg cggcggcggc ggcggtcgcc 240
aagacgagga gtgaggagca ctgctagcga gggcagctcc tttttttttt ttttttttga 300
gaattgcgag gggagatcct atacgacgct tattgcgtcc aggggcagca cggctgggcc 360
gccggcccat tagttagtcc annnnnnnnn nnnnnnnnnn nnnnnnnnnn nnnnnnnnnn 420
nnnnnnnnnn nnnnnnnnn 439

<210> 128
<211> 495
<212> DNA
<213> TRITICUM TAUSCHII

<400> 128
ttcgccgcgc taccgacgag gtcactcgcg cacgaatcca tgctcgtcgt gtcggacgcg 60
gagccacgac caccgtccac cgcagtcgcc atggtttgac acacactaca tttctcatcc 120
ctgttcctct gcctcagcgc tcctccgcac aaactttctc tgcggcggcg atgctagcaa 180
taaattcgcc atgacaccgg caagaacgct cgcccgagac tttatgcaat atgtgtgtgt 240
gtgtgtgtgt gtgtgtgtgt gtgtgtgtgt gtgtgtgtaa caatgtgcag acgtcgacct 300
tttgcctgtt ttacttcaac caggctgagc tagccaggcc cggctcctac ggcaaccaaa 360
cacgcccccta gagttatatc cgattttggt aaaaaggttt gttgttcttg aatatttctt 420
gtccctagct gtttgttctt gtatctgtaa tattagttga atctgatcac tgatgttttt 480
gtttttggta tgtat 495

<210> 129
<211> 548
<212> DNA
<213> TRITICUM TAUSCHII

<400> 129
cccgtggtct tgggttcgaa ccccaccctc ccaaactttg ttctcttttt ctcatgtgcc 60
ccttacaaat gggataggga tagtttcgtc atctaacttc agtcaacgtg tattgaccaa 120
acggtaacgg tgaaaaatgg tttttttgag cttgatctaa tagaacagtg gcatttttga 180
gtagtgaaaa tatttagtgg caaaactgat tagcgttata cagccgatgg caaaactgat 240
aaaaacccta aaacaaaaag aaacagaaaa catctggact gtagcaactc ctgtagcgga 300
cgccacgtga cggttcttga ttggccgggc gcgtgtcctc acccaaaaca gaggaaaacg 360
acggcggcgg ctcgggcgtc ggcggtggcg ctcctccggc gactctccgg cgatggggga 420
agtgacgggc ggatgcgtcg gtgagtggag gaggttgtgg tggtatcgaa cggcgtcgat 480
acgtcctctg taggcgacgg cggcgaggtg aacctcggtg ctccggcggc ggcagacgtc 540
ggtggcgg 548

<210> 130
<211> 520
<212> DNA
<213> TRITICUM TAUSCHII

<400> 130
caggagaaag ctacagaata aaactaaagt tgttgcatct tcttggtttc atgcttactg 60
cttcttcatc acatatatgt tcttgagttc atactatatc tgaaaagact gaattttaca 120
ggtgaatagc agtgcgaatg aggatatgca agatatgtgc attggaaatt tggaagtcaa 180
gaagatgcgt tcatgcaggt caaagggagg gatgaaaaat tgattcatat tttttatacc 240
ttgaaacttg tgtcgaaact gacagagaaa tatttatgtg agtgaaagag ccaaacttct 300
cttgtgtgtg tgtgtgtgtg tgtgtgtgtg tgtgtgtgtg tttattgcta gacttataaa 360
tgaatctata agacatatgc agaataaacg atctgctaaa gatggaaaac ttaagcacta 420
gggaaagctc ccaaagactc aacaagttct ccagagtcgt tgggcctgat agtaatcttc 480
atagaggtgt cgacccttag gttcttctcc atgagaaatt 520

<210> 131
<211> 1038
<212> DNA
<213> TRITICUM TAUSCHII

<220>
<223> n means a, g, c or t

<400> 131
nggggattt atgaagagga tcatattgaa gctgatggga agcctgctgc tgctgaacgg 60
catgtagctc tgctttgcta tgtttttagc cgctctatat cttgtcaatg cttgtcttct 120
gtaataagtg acaccaaatg gaagttgtgg tttcaagaga gagcttagag atgccttgca 180
atgggagctc tatgcacaaa ggatgtgcct ccaactctga aggacagaac gtttttccat 240
tatgagatta ctctanatcc cttatgacat ttctgtccgt gatcttgcta ttgtagctta 300
acattgctat atagattgct attttgcaat caaagatggt tgaggangcc acccacaatc 360
caggggtgta ttctggtata agcacaaatt tccttagaaa agaaacaaag ataatgcata 420
acatgaagtc gaataacaac ctcatgttca aacaaaagac gagctaactg cagacccgat 480
atgatgatgc attattgtca atattaaaac acaaacacac acacacacac acacacatgc 540
actaaaatag actggtttat ttgaccagat ttcaattgat cgtgtgaaca tgtgcnaagc 600
ttacgaaagg ccatggctgc accatccact ggccttgggt gtggaaggta attgatctcc 660
gcatcaggat tgagtcactc ccatctaaaa caattagtta gtccannnnn nnnnnnnnnn 720
nnnnnnnnnn nnnnnnnnnn nnnnnnnanc cnngcgnnnc cccnnncccn nccntttnnn 780
nnnntntttt tttnttnnnt nnnccccccc ccnncnnncg ngccgggagc cnaaggggna 840
aggcgnnnnn nnnnnnnnnn nnnnnnnnnn nnnnnnnnnn nnnnnnnnnn nnnnnnnnnn 900
nnnnnnnnnn nnnnnnnnnn nnnnnnnnnn nnnnnnnnnn nnnnnnnnnn nnnnnnnnnn 960
nnnnnnnnnn nnnnnnnnnn tnnnnnnnnn nnnnnnnnnn nnnnnnnnnn nnnnnnnnnn 1020
nnnnnnnnnn nnnnnnnn 1038

<210> 132
<211> 509
<212> DNA
<213> TRITICUM TAUSCHII

<220>
<223> n means a, g, c or t

<400> 132
ctaatctaag caagggccgg ctacacactc acacacacac acagacacac acagggtcac 60
acacacacac acacacacac tgcacacatt caggcgcgca cacacagcac acactcaggc 120
acacacacac acgggagcag cgtagagcag tggcaatgtc cgcaacacag cagcggtatc 180
cagtggcagc ggcaacacga cgcagcggga ggtagcaact cgcagtgcag taaacagcag 240
cagttagtcc annnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnn 509

<210> 133
<211> 504
<212> DNA
<213> TRITICUM TAUSCHII

<400> 133
ctgaatttgg gtatgaaagg ctgaagggac aacatatcat ttagggaaga ataattagat 60
gagcttgacc aaacctattg ctccgtcaat tgtttgcgtc gcaccaccat gggatactgg 120
ccttcatatg agcttgacca aaccaacaca cacacacaca cacacacaca cacacacaca 180
cacacacaga gagagcatgt cactaaagtg ttgaatagtt gccatggaca aatacccttg 240
acatggtcaa gcgttgaata gtcgcgatgg cccgttcggt tcccactgga ttattctttc 300
ttttacacca tgaccgagca caacaatttt actccctctg tcctaaaata catggcatat 360
aaactaagtt aaaaaaatca atgctttcta agttcgacca aatgtatata caaaaataac 420
actaccaagt caatgccaat atattcatca ttagacattc aatattatag tgatatttta 480
ttccatttag ttggccaaac ttag 504

<210> 134
<211> 541
<212> DNA
<213> TRITICUM TAUSCHII

<400> 134
catgtatata tatcggccta tggcctcatg ggaatacaag ttgcatattt cctaacagtg 60
cagaggacaa cgagcaggga gatcgctcga gttctccccc cacttgcttt gcctgctgag 120
cttggcagag gctaacatgg actcttggca agcacattat ccccctttg gtctgctgta 180
aatgtgtcct ccaacctttc ccataccatc acacacacac acacacacac acacacacac 240
acacacacac atacatatat tttatttcaa tggtcaggga gggcgaacag cagagacgag 300
tgtgtttagg ccctgaagct gttcttcgat gctgatacca ttagagtgag ggctagaagg 360
cacactggca gaccttccat gcttggcgca gaagctctgg tgctattatt tgtttcagat 420
ggaaaggaag agaaggggaa catttggcta ccctcatata tagtgcaaaa gtagaagagc 480
ctacagacaa caggaatctt gatcagttca agtcctggga gcttgtcacc catcatcaag 540
g 541

<210> 135
<211> 480
<212> DNA
<213> TRITICUM TAUSCHII

<400> 135
gtggagctca aatgatttca ggtaaccact agagtatgtc cttatcacac acacacacac 60
acacacacac gcacgcacgc gaacacacac acacacacac acacacacac acacaccttg 120
agaacatggc acctctacat gacattgcac gcacgcgcgt gcacacgcac atgccggtta 180
gcctctataa tggtaaatta ctgagtgctt catattttcc aggctcaaga gaatacataa 240
ctttcatgac agtggatggg ttgtgggtct cgcatgtttg gtgggatcag gaaggtaatc 300
aatatttcta gttcttatta gttttcttac ttccccttttg tagcatgctt agctatatag 360
atggtgtggt catttacatg attacatcct tttcgtttcc tatgattata gattagtaaa 420
agcatgtgtt cgtttatcta tctgcaggct tgtagttgca cacaaaactg gagtgtcgtt 480

<210> 136
<211> 550
<212> DNA
<213> TRITICUM TAUSCHII

<220>

<400> 136
ctcaaaagtg tagctagggc aaccggtgct ttcatagata aaatgacttc agccgaagaa 60
ggatttctgc aataacaaac gaccatgtta gggatcacat acttgaagct ccatgagaga 120
gagagggaga gagagagaga gagagagaga gagagagaga gagagagaga tgatatttgc 180
atacctttat atcatcaact gtttctacta cttcctgctg acttgtgcga actcccctaa 240
gttcctgcct cattagtgcc acttcctcat gtagtaattc aatgtagtcc annnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn 550

<210> 137
<211> 513
<212> DNA
<213> TRITICUM TAUSCHII

<220>
<223> n means a, g, c or t

<400> 137
cggtatggga gatgggatac aactcacgct gctcttgtag ctcgctggct tcttgttttg 60
gaagaagtcg gtcgaggaag atatatgaag cctgagggga ggaggtcgct ggcggatggc 120
agcggctggg ttaggtgaaa ggttccctcc ctctcgctct ggtgggagga ttcatgttgt 180
tgtctgcaaa gaagaagaaa agggtggtct catccaatcc ggcgtagtgc agcttgagga 240
ggaggagatc atgtcgtctt gtggggcgag gaagaccacg gcggccaagc ggcggtgatg 300
aacgtgcgat tccaggccgg cggcgaacag gtcaggcgag tcccaaaatt aatttgattg 360
ggtcggagct cggcctaatt ctacaaaaaa aaggaaaaga aaaagaaaag gagaagacga 420
ttcgcctagc ctagctacgg gggagagaga gagagagaga gagagagaga gagagagaga 480
gagacgngcn gctccaagga gaccatcgac acg 513

<210> 138
<211> 472
<212> DNA
<213> TRITICUM TAUSCHII

<400> 138
ccctagttgg aagttaccct gtccgtcccc aactcgggac ggagacggaa gcgccgccgc 60
cgccctcgac ctgcccaccc cgctctggat ccagccccaa cggctaccca ctaactccac 120
tttccttcta tacccgccgc acgccacctc gccgaagccg cgcccggcaa ggacaagccc 180
ccggctgccg cgaccgcctc tgccccgact ataaaggcgc gccctttggc cggccagctc 240
ctcctctctt cgccgaggcc actcctcctc tcttcgtccg ggccatgcct ctcgctctcg 300
cctcgattcg tcctcgactg gtgcctcgcc tcctctcgcg tccggtttca tcttgctcat 360
ccccgctcga ttcgtccgct cggattagaa gaatcagatt tggttttgct cgtctgcggg 420
gttctgattt atttggttct gctcgttcgt cctcgccgtc gttcgttcag aa 472

<210> 139
<211> 551
<212> DNA
<213> TRITICUM TAUSCHII

<220>
<223> n means a, g, c or t

<400> 139
cccattgcca tctcagtctt atccatctcg ctccgcacct gcgcctgact tctctgagga 60
gctctcccat tggcggcccc ctccctccac cagccaaact gctacttctc caccctatct 120
cgcccttctc tttctccttc tccaaagaca tcttcatcca ttctctctct ctctctctct 180
ctctctctct ctctctctct ctctttctct ctctctcatt ccaagccatc gatgccatcg 240
ctttgggcct actatcgttc tacacaggcg gcacatcgtc ccccttcctg ctcttcatcc 300
cccttcaggc ttcgacggca acaaattgga gtagcggaaa atatcctaga gttgtttttg 360
ccttttctgt tttagttagg gtgttctcta caaaagtggg cagttgtctc gtatctggat 420
gtagtccann nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn n 551

<210> 140

<211> 541
<212> DNA
<213> TRITICUM TAUSCHII

<220>
<223> n means a, g, c or t

<400> 140
ctcccttgct ccaggaaaca acttctgaac ccttgatttt ccgctgtttt ttaacaaagg 60
gggtatctat ctgagatgta aagaaaaaga gggtgagctc agacaggtca attagtaaaa 120
atgaaaatta ataatcatga aatattgtca cgcaaaaata tacagtgtct actataccct 180
gtgctacaca cacacacacc cacacacaca cacacacaca cacagaaaga gagatctaca 240
tttactgaac aatagatgag ctaagacatg ataccagtta ccatcatgcc actaatacag 300
ttctggcttg ccagtaagac gaagtagtcc annnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
n 541

<210> 141
<211> 449
<212> DNA
<213> TRITICUM TAUSCHII

<220>
<223> n means a, g, c or t

<400> 141
tttgtcttta tttcatcgct ttggcccgaa aaaccatgat cgacagaagt ctaaccatct 60
ctgcacccct acgggtatcc tattttcgtc agttgtggca acttttcct gtcctttcat 120
ttgttcgtgc gtgcgtgcgt gcatgtgtat gtgtgtgtgt gtgtgtgtgt gtgtgtgtgg 180
aggctanctg gacaggttag tccannnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnn 449

<210> 142
<211> 327
<212> DNA
<213> TRITICUM TAUSCHII

<400> 142
ctgtttttca attcgcccta acttaagttt tctggaatct tcagatgaca catggacatt 60
caactttttt ttgatttcat gaaaaatcat ttttagcgtg aaatctactt tcagggcacc 120
tcggacatgg caatttctgg cggactgccg catagccttg gatttcgaac gagtgaatga 180
gattataagt gtttctagtc acacacacac acacacacac acacacacac acacacacac 240
aaatagatga gacacatgtt ttcgtgttga ttatatatgt gtgattacaa cgtctagaga 300
gattacaggg aggtttggag agataga 327

<210> 143
<211> 400
<212> DNA
<213> TRITICUM TAUSCHII

<400> 143
ccttcttaat gaccggctcg gagctgccct tgtccttctt cctcttgggc tcgctcccat 60
cctcgtcctc ctcgccttct tcgtcgtcgt catcctcccc ctcctcgtcg ctatactctt 120
cgtcatcttc gtcgtcgtcc tcgtcaccat cttcctcgtc gtccccgtcg tccatgtcca 180
cgtcttcgtt tggcttgggg gcagggccgc cgtcggcgta gatctcgggg tcgtggatga 240
ggagggcgca gaggccattg gcgaggcgga ggaggcggta ggaacggttg tcgctggggg 300
acttgaccac cagctcgtcg tcgcgcgaca ccgccgccgc catctcggcc gtccgccgcc 360
gccttgtaga tacgaaggag gggggaggt ggtgggagat 400

<210> 144
<211> 486
<212> DNA
<213> TRITICUM TAUSCHII

<220>
<223> n means a, g, c or t

<400> 144
cctttggata gcttatttcg tctaacaatt gatacgatgc gaagtattca gtgcgtctca 60
tacccataca aacaatagtg cttttggtat gtgcatgatg attgtctcat gtgaaattac 120
attatgcttg cctagagacc ataaaaactt gtgaactgta aataatccat aaacaaatac 180
atgtgtgtag tggtagaatt gtatgataca cacacacaca cacacacaca cacacacaca 240
cacacacaca cacacattct tgtataactg ttctctgcat gcatacatag cattctgagt 300
gctgatggtc ttgaagaaat ccgttagtcc annnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnn 486

<210> 145
<211> 551

<212> DNA
<213> TRITICUM TAUSCHII

<400> 145
aagatgaaag ctcctccctc ccatagcctg gcagagaaag tgaaggaaag cacacacaca 60
cacacacaca cccacacgga gcaaaacggg aatttggttc ggttcggtta cctatactag 120
tcagttcggt ttcatcttaa ctaactagac agcaaacggc gcaaacccga gatgaagcca 180
gatcttcgac ccagttcact tcagggttcg ccctgaaaaa ggagcctctc tctctctctc 240
tctctctctc cctctgtatg ttaaacgccg gtcgattagc tctcggcagc catggcggcg 300
ggcactatac aacggcgact gcaggtgcca ggggaaactc catggcgggc ggcgggtcgg 360
gccagcaaaa ctcgaagggg gcttcgatcg cgggggcctg ggccgccggg agggtgatct 420
ctctggggca tgcctcggtg tagtggaact cgatctggag gcggtggtgc agcggcgtcg 480
acgccatgag ccccttcttg atgtcggcct gcagctcccg gatcgggatg gcggccagga 540
agctcttgat g 551

<210> 146
<211> 518
<212> DNA
<213> TRITICUM TAUSCHII

<220>
<223> n means a, g, c or t

<400> 146
ccgtcccagg cgggacagct ccggcttgtg cttcaggggt ttgccgaagc cgggtgccaa 60
ctctatcaag tggaaacacg agttcttctt cctctcgtcg ccggagccgt ggccctgcgc 120
cgtggagtgg cgcgagcggt ccaaccgctc ctcctgcaac ccggtgctca ccgttgagga 180
aagccagtcg atgctgaagc tgctaagtgc tcatggtggc gccgctgttg atctcaggaa 240
cctgcttcct cctacgtgct caaggccccg cagccgcctt actgccgcat ccccgccgcc 300
gccgccttcc tcaactcgta ttattctgtt ccaaatccct ctgattcccc tgtttcatcg 360
agcccttagg atggtgttgc gtgctatcta ggttagtcca nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnn 518

<210> 147
<211> 385
<212> DNA
<213> TRITICUM TAUSCHII

<400> 147
caggtcaact tcatttgaca ttttcaattc tcagtaaaaa aaagacagtt cgagaaagaa 60
gaaaaattgt cggctgctcc tcttgcttga tgttcatgtg tttagttctt taccgctttt 120
ttcttactgt tttgttcaaa aagtttgtgt ctccagggac ctacatgccg ctggtgctca 180
ctacccgcat gaacaaagag aggaagaagg tcaccatcag gcaggggaga aggtcacgtg 240
aggtgcagaa agaaggtcgg agttcagaac ggtgtgtgtg tgtgtgtgtg tgtgtgtgtg 300
tgtgtgtgtg atttttgtgg agttcagaac ggtttgtgca gaaagaaggt gtgattttcg 360
tgctggtttt ttggggtgca ttcca 385

<210> 148
<211> 542
<212> DNA
<213> TRITICUM TAUSCHII

<220>
<223> n means a, g, c or t

<400> 148
ctaatacgct atggaagtct tcttccggga aggcgaggaa agagaactag ctaagcatcg 60
gcttgtgcat aagtgtataa ttttttactc acgtgaattt cctagatgtc tcgccaggct 120
agtttatttt cgtggccatg tccgaaagtg acaatggtta ctgcgtgctt gcaccaaaat 180
gaatatggga gccaattgga aggccacgat acactataaa gtgtgcattg actaagcatt 240
gaatgtgttg gagattcatg atctcggatt ctgttcatat acgacgaagc attaacatgg 300
gctttgaaac acacacacac acacacacac acacacacac acacacacac acacacacac 360
acacacacac acaatctcat aaagctagat aaaacccgac gggtgcactc tcgttttgtt 420
ggttagtcca nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nn 542

<210> 149
<211> 518
<212> DNA
<213> TRITICUM TAUSCHII

<220>
<223> n means a, g, c or t

<400> 149
tagtcccaga ttttcagttt aaactgtttt gtcaaactga aaattccatt gttgtagcaa 60
ctttgaaaac agtccgagat ttttgacagt gtaaatggac ttcacagtcc cagatttttg 120
acaactctaa atggcttctc taaatggcca tcaagcaatg aaatttggtc aatatgacaa 180
gtggcattac ttcacagtag tctgaaggcg tttgatttaa catatcgcac gcgggcacac 240
acacacacac acacacacac acacacacac acacactcag ggaggcatta gttcacaagc 300
tacataggtt cagacttgaa gcatcaaaat gttagtccan nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnn 518

<210> 150
<211> 550
<212> DNA
<213> TRITICUM TAUSCHII

<220>
<223> n means a, g, c or t

<400> 150
cggtctttgt ttgctctaaa cctaacttga tgagagagag agagagagag agagagagag 60
agagagagag agagagagag agagagagag atcaatgcgt tcttgacatc actacaaata 120
tgaatattac tgtctgaccg aatagtatga tcattgacgt gcaagtggac catcagtgcc 180
tccgagtcag tgataacttt gacaccacaa ccgagcctct aggtcggcaa gtgatagagc 240
ttcatcatag atggccggtc ggtcagtgat aatcaatagt ttgttgtagg ctgaatttgc 300
tccttttatt gcttggcttt atttatgttg gttagtccan nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn 550

<210> 151
<211> 542
<212> DNA
<213> TRITICUM TAUSCHII

<220>
<223> n means a, g, c or t

<400> 151
ttaggattcg gagctcaatt tgttttttcg aaaaggagag ctcgatttgg tgctagccaa 60
tttagtgtcc agtccacctg tattacctgg gtgtgcaagt gtggcttctc catgctggca 120
tcggcgacaa aagcaataga aagagtgctc acctacctgg caccgcagaa gaaccgactc 180
aagcggcgca tcaggggtgc gccttaacca ctagtcaaac atcaaggagc aggttgcacg 240
acctacaaag catgtttcct cgttgaactc gcaactggag agagagagag agagagagag 300
agagagagag agagagagag agagagagcg cgctttcgca aaaaaacaaa ataaccaaga 360
aagatgataa gcccgatagt gagtgagcga tgtaagagga aggcggagag tggacttttt 420
atattagacc caactattac ataattacta atcctctcaa cacgttggtg aaacattaat 480
cactactccc tccgtctgaa actagtgaag tagttagtcc annnnnnnnn nnnnnnnnnn 540
nn 542

<210> 152
<211> 520
<212> DNA
<213> TRITICUM TAUSCHII

<220>
<223> n means a, g, c or t

<400> 152
ctacgtggat cgattggggt gctactcttg ttttacgggg acagtattcg gcaaggttcc 60
gccatgcata tgcattttac agggacggac ggggcggttg aaccaatttt gttagttggg 120
atatggttgc atgctcatac tgctctccct ctctctctct ctctctctct ctctctctct 180
ctctctctcc cggctttgag cgtgcacgtt tgcacaactc cctctccgca tcgagtgtct 240
tctctttcac atattcacac gtagtccann nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn 520

<210> 153
<211> 550
<212> DNA
<213> TRITICUM TAUSCHII

<220>
<223> n means a, g, c or t

<400> 153
gtttttacgc gatagccatg ttgagctttg attctacact tcttcggctt tctgcctaaa 60
tctaaatgcc cgatttcagt ttcactatgc taatgcgaat ttatggaagt tgctgccagt 120
gagacagtgt caagtattca cacacacacg cacacacaca cacacacaca cacacacaca 180
cacaccggca cacaatcgtt ttttaacggt gaacatgcac attatcctct caaagaagaa 240
atataatcac aagaagacta tttacattag gttagtccan nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn 550

<210> 154
<211> 543
<212> DNA
<213> TRITICUM TAUSCHII

<220>
<223> n means a, g, c or t

<400> 154
cttggaagtt gtcacgcgaa ggcagccact tccggtgatt gtcagggagg ccacgcaggg 60
tgctgctgat gtttgttgct tgctagctag ggtgatgatg ttctcgatgt gaaagactgt 120
tatgtggtgg tccaactctg gcagaaggcc caaggcctcc tgatgcaacc gatagttgga 180
ctaggccttg aagaagaccg cagatgcgta agaatggaag gcggacatag aggagcaagg 240
atacatatag agagcagaga ggtgaggaga ggttgtcgaa gagagagaga gagagagaga 300
gagagagaga gagagagtaa aatctagatc tagagcataa aatcccgttg tatctctcgc 360
cttatactag tggctcgatc tatacatgtg agtgcataac ataggcagtg gtcgtgtgct 420
attatggctg ttgtggaggc tatggcgata tatagttcca gagtcgaggg tgtgccctcg 480
agttgtgcgg ngtgagtggc tttggggcct aatgcacata cctaggcatg tagcacattg 540
acc 543

<210> 155
<211> 544
<212> DNA
<213> TRITICUM TAUSCHII

<400> 155
ggtcgcaaag caccgcgaag accgggacga agcgcagacc aaaccgtgac ccctcgctgc 60
caccggcgca gccgaaggta acgtgcacca agccacgacc cctggctgcc attggcgcag 120
ccggaggcaa cgtgccgccg agcctgacgc acgttccctc cctccgtcct cctcctgtat 180
actatttgtc ttgtctcgtt ccctcctcgg tctccctcca cccacctacc catcatcatc 240
ccctacataa cccgatctag atcgcgggaa gtgcatccat ccatccatcc agggccgcac 300
accgccgcct cgcctccttt gtccccctcc cgccagcccc acgagaaaaa gggagaccac 360
gccgccgcct ccttcccacc gccgacgact gccccgacac ctccggcaag gtaagctcct 420
catgcatccc tccatgatcg ccatctgagt ctgcttgggt gtatgtatgt atgaactatg 480
aactgcctcg tagtaatttg catgtatgta ttgcttggtg cacgattgct gctgctatgg 540
attg 544

<210> 156
<211> 400
<212> DNA
<213> TRITICUM TAUSCHII

<400> 156
cactgcgaga agggcattga gctcaccttg gagctcgggt agtagccggg agagggctgc 60
gcctcagccc aacgaccctg ctgcagcagc agcagcatca cggctcctac caccaccatg 120
agatgagaga tggcaatatg aggcctaatc tgcatcatca tcgtcatcat cttctgctcc 180
agtggagtgg agttgtgtgt gtgtgtgtgt gtgtgtgtgt gtgtgtgtgt gtggttctac 240
tccgattgag ctgccttcac gcatggggct atatgtaatg tttgtaggag tggggtgggt 300
cccgcatggg tgctaatctc aggagtggtt ggttgctaat tggagttagt tatacacttg 360
tgatcgctga gactgatgat gagctttcat ttattactag 400

<210> 157
<211> 542
<212> DNA
<213> TRITICUM TAUSCHII

<220>
<223> n means a, g, c or t

<400> 157
aactggtgaa gtctagtatg gtctctcata tcaacatatt gagaaacaag atagaggttg 60
tgaaattaat tccaacatac ttttattgtg atttgtgaaa tgcacatacg tttcaatctc 120
gaagatatat actagtttca atagacacaa tcccagtttg cggttgtgca tgatggcttc 180
aatctttttt tcctctctct ctctctctct ctctctctct ctctctctct ctctctctct 240
ctctctttgn ggagctagga cttgagatct tggatgagtg gttggtcagt gttctggggc 300
cttcaagcct tgaggatttt gttttgattt ataatctgtt aagcagcgga tcattaggat 360
tggaggaaag caaaagatta aaagtgacat ttacttctat agagatcatt gaaccccaca 420
cacgtgttag tccannnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nn 542

<210> 158
<211> 456
<212> DNA
<213> TRITICUM TAUSCHII

<220>
<223> n means a, g, c or t

<400> 158
ccctgacttt ctcaatcttg cactgtcaaa gaattttgtt acaacccagg gggcagttga 60
gaaagaaaga aacctataag gagagtttgt ttttggtcct gaataacaaa acagagatga 120
tcagctgcag taatgagctc ctaaatcgcc tctgtgtgtg tgtgtgtgtg tgtgtgtgtg 180
tgtgcgcgcg cgctttccta gagttactcc aaagatgaaa ttatttcctg tttctttcgc 240
cacacaacat tgtgcctgaa aaaaattacc ccctgagagg gaacttaagt agttagtcca 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnn 456

<210> 159

<211> 489
<212> DNA
<213> TRITICUM TAUSCHII

<400> 159
cgcacaactt cctctcaagc ggcccgcaat gacggcccca atcactagtg ttaattaagg 60
acccacatca cgagcacacc taagcaacaa cagttgccgg cgaagagaag cgtatatcat 120
gattcatgaa tgagtatcta atagattact cacaccgtgt ttgtttcatc attggaatgc 180
actgctcgac tttataaacg aacttgagag gttttctgag tccaattttc aaatagattt 240
ctctcctatg tttgtttcgt cttcaaaatg cactgttcta ctttaaaaac caacttgaga 300
ggtgtttgag tctaacttca aaatagattt agtatagtgt tctatataca attaaacacc 360
catcaatttc agttttcaac acacacacac acacacacac acacacacac acacacacac 420
accaattcaa tgcatgattt attctgttct aattgtctac taacgcacct ttgtaagcta 480
ccaaaggaa 489

<210> 160
<211> 488
<212> DNA
<213> TRITICUM TAUSCHII

<220>
<223> n means a, g, c or t

<400> 160
ctaatatcga taggccgctt caagaattaa tatacatggg ctaataggag ggacaacctt 60
gatgagtata aaagataaag aggcatataa gggccaagtg acgtaggacg aatacactcc 120
tctatggagc ctatgtcgaa agaggctacg gttgccgcac acaggtagac cataagagac 180
ggacaactac gagggttgca gtgggtggca gtgaggaaga gagcctgaaa gttgttgatc 240
cttgatggga ggagtgcctc tctagagatg cgtgcattct gaccgatgag atgcttagat 300
tctgatcagt atttgtgggc ggcaatgtcc tttccttggt ctctctctct ctctctctct 360
ctctctctct ctttgaaaga acgtaaacac aatgatgaat acaatacatt agtcagctca 420
tgttagtcca nnnnnnnnnn nnnnnnnnnn nnnnnnnnnn nnnnnnnnnn nnnnnnnnnn 480
nnnnnnnn 488

<210> 161
<211> 551
<212> DNA
<213> TRITICUM TAUSCHII

<400> 161
aacgctcatg gtataaaagg tgcccgatcc aataaaaaga ctgcacacat acgattgtgt 60
aaggtttggg caattgaaaa tgaaggttcc cgtgtcacta tgacaatacg gggatactcg 120
actaatatga ggggtataag ttgcacattt tactcatatt gtctggtgat aatttgtgtt 180
tcctcctatg ccaactctat aaacttatgt tttgagtgag ttttaccacg tcaagggttg 240
gtgcctagat tgaattatag tttgtccatc cctaggacaa aacacacaca cacacacaga 300
gagagagaga gggaggacgg gaatatatga attattggga gcaattgagg actttcaaag 360
gagcgactag aaaaggatga tgtcccaatg ggaaatgaag gttcatgtgt cattatgacg 420
atacaagacc accccgaggg gtgtaacttg catgtttcaa ttacgattgt tggtcattat 480
tgttgtttcc tcttaagcga gcttaactac gtaagtgttg gcaccttgat gcacacaaag 540
aacacacaca c 551

<210> 162
<211> 458
<212> DNA
<213> TRITICUM TAUSCHII

<220>
<223> n means a, g, c or t

<400> 162
tctacttctt gtagcctcta agtatgctat caggatagca cttttgctgg caagtaacat 60
gaacggaaga gagagagaga gagagagaga gagagaggga gagagagaga gagagagaga 120
gagagagaga gcaattgtaa caggttggac cattggagtg gttgtgaatg gaaggcatac 180
ggaaatgcat catcttcgcc gttgtggagt tcagagaaga gaaggcaggg gcaacatgaa 240
tgacatcgag gaaaagctaa caaatgaaca tagcagttag tccannnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnn 458

<210> 163
<211> 408
<212> DNA
<213> TRITICUM TAUSCHII

<400> 163
ctttttcaca atttcatttg caattcggat gatttttcat ccgatgatga ggagattgtg 60
gctgctgctt tggtcgtcca tgaccacatt agtaggaagc ggctgatgtt caggggctca 120
atcctgggac atactccggc gttgaatcgc aacacacaca cacacacaca cacacacaca 180
cacacacaca cacacacaca cacacagaga gagagagagg agcattgcct actcaggcgg 240
gactacttcg aatccgctga cccactcttc aaacacaacc tattccggcg ccgcttccag 300
atggctagac atgtgtttaa ccgtatccgg gatacggtgg tcgcatacga caactatttc 360
gtgtgcaaga aggatgtcct gggaaagatt ggcttcttct cttatcag 408

<210> 164
<211> 528

<212> DNA
<213> TRITICUM TAUSCHII

<400> 164
atactcaagg aaatctccgt ctaacgcata tttatccttg aatgaaggta aatacaccta 60
caatggagag caatacgacg ctatagctcc accacaagga gcagcatatg tcaagtgcag 120
agaggacgtc accaaagcac taaagttgaa tgcaccctgt gaaacaaaga actgcacctt 180
caacggcgtg tggagtggcg gcggtggagc tggcctggcc gacctctaca tcaccaccag 240
cttctactac acggcatcac aggttaaagg gaaccactaa ccaattaatt aatctggatg 300
catgtgaatt agtgttggct acaagaactg atgaactctc tctctctctc tctctctctc 360
tctctgtgtg tgtgtgtgtg tgtgtgtgtg tgtggccaac aggttggctt gatcgacagc 420
gaggctagca gtgcaaagac cacccctgcg gcgtggaggg acgcggcaga gaagatttgc 480
ccgctgagct tcaaggaagc gaaggatgca tacccaaggg tccgagct 528

<210> 165
<211> 352
<212> DNA
<213> TRITICUM TAUSCHII

<400> 165
catcaaaatg cttaagctca taatataact ggcatcgact tcctcctcct acctccttct 60
ttgatgctca agctccgccg ccgccgccgt cgaactcctc gtcttcaccg actcaggccg 120
ctgcatcacc ctgattccga ccagaaaacg atggcgaccc tccgcagctc ttccgcatct 180
gtgaggtcct ctttttcccc cttctcagat ctgcattgaa actgtcttga gttcgttggc 240
gttcttcgcc gcccgacgca gacctccatt agttttcacc tccaatgcct tgttcagatt 300
ataaaacaac atagaactgc tgcggaaaat atttgcgctt atcctgtata ag 352

<210> 166
<211> 471
<212> DNA
<213> TRITICUM TAUSCHII

<220>
<223> n means a, g, c or t

<400> 166
cccacgaatt gatctgtcaa agcaataaat taattatctt ggatggattt gtgagctata 60
ctagtagcaa attgaagcaa tctgtaaatt tcatcaggcc ctgcactcat cacacagatt 120
ccacacacac acacacacac acacatacac acacacacac acacacacac acatggtttg 180
ttgcagctaa tgaaggagaa ggaagttagt ccannnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn n 471

<210> 167
<211> 471
<212> DNA
<213> TRITICUM TAUSCHII

<220>
<223> n means a, g, c or t

<400> 167
cccacgaatt gatctgtcaa agcaataaat taattatctt ggatggattt gtgagctata 60
ctagtagcaa attgaagcaa tctgtaaatt tcatcaggcc ctgcactcat cacacagatt 120
ccacacacac acacacacac acacatacac acacacacac acacacacac acatggtttg 180
ttgcagctaa tgaaggagaa ggaagttagt ccannnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn n 471

<210> 168
<211> 533
<212> DNA
<213> TRITICUM TAUSCHII

<400> 168
caatcaatta agtgcaaata ggcattgaac aattagttgt atcaagtaat actcctatta 60
tgagagaaat tgatacttag tggaagggat ccatgaatgt ttacagcaat ctacaaatca 120
gattcagact tgggggaaaa ttatgtctag aaactacatt ttataccaat ttatttaagg 180
aagaccatac gtgtccaaga ttgtctatta tttaagcact aagcaagttt ggggcatgat 240
tttgccgaga cctccacaga tgtcatttca tcacaaaaat tggcacgcat gtgaaagata 300
cataagtgtt tgttgccaaa aagtttcaga tttttttgat ttctaaaaag attgatttta 360
ctgtagcaaa gggtgcatgt gagctcgagt tcacatactc catgccccta tacacacaca 420
cacacacaca cacacacaca cacacatata tataacttct agagttgtaa tcacaccagg 480
aataaataaa gtaaacaaca gcgacgtaac atgcccatgt tttttttttc ttc 533

<210> 169
<211> 496
<212> DNA
<213> TRITICUM TAUSCHII

<220>

<223> n means a, g, c or t

<400> 169
ctcatcatat cagtctgcaa tccactttgc gccatgcata tgcgtctctc tctctctctc 60
tctctctctc tctctctctc tctctctctc gctctctccc tctctctacc cacatgcatg 120
catgtatgac aatacctgtc ctcccttcct ctcaaaatcc atcgctttag tttccttttc 180
ataatttaga tggtagtcca nnnnnnnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnt 480
cttccgcttc ctcgct 496

<210> 170
<211> 344
<212> DNA
<213> TRITICUM TAUSCHII

<400> 170
ctgctaagac ggggtctcta acctctgatc tgttataaat tgtatcccaa actgtgagaa 60
ttgttgaaga ttgcaagaaa attgaataga tcagttgatt ttcgctgaat ttgcgcttac 120
aaaggtatca aagcgccagg ttgttcctcg gcgacatcgg atgtcggcga ttcgggcagc 180
ggagagcagg aggttgggtt ctcccgtaaa atccctcccg tccctccctg ttcacggcgg 240
cggtgtgatc cggtggcggc gacgcggcgg cggtgaaatt cgagcgtaga tatctgagtc 300
cgagacggtt gctgggagat aatctcctgg tagtgaaatt ccgg 344

<210> 171
<211> 456
<212> DNA
<213> TRITICUM TAUSCHII

<400> 171
ctatggtccc caactatggc atcatgagct ttctcagagt agatacaggt aggatctaga 60
gcacatgtga tgtgcggtca ttgcatattt gtttgtttgt gtgtgtgtgt gtgtgtgtgt 120
gttggtatta gattggatta tacttgatgt gttggatcct ttggcactcg aagtcctctc 180
attctccaaa gaggtgccaa tgcgccgctt caagtttttg accctctggg attttatcaa 240
gaatggggtt ctgaggatgt ctaggtttgg tgtcgtacta gtgccctccg ccatcatgaa 300
ggccttactc gcagggtcgg agctggcgga gctttctctg catttgttcc ggtccaagtc 360
gaggacggca aacaaagtca accaccccat gaagcgggcg gtcgtgtcat tgcccattgt 420
ctggacaggg ctcccacgtg gctccttgcc ccgcct 456

<210> 172
<211> 408
<212> DNA
<213> TRITICUM TAUSCHII

<220>
<223> n means a, g, c or t

<400> 172
ctagctggac gaagccagca ccatgttaaa ctcataatct gtgcaaacca acctcattga 60
ccccaagaca aacannnacc tccnnnnnag caaaacacac acacacacac acacacacac 120
acacctcccc attcatctcc ctccatggag ccaggatcca aacaagatgg agctgtgaac 180
atgacgaggg caatggaggc tttgtcccct tggttgaacc acccaaggag gacgatcgtc 240
caggttgaag ttctggtggt tagtccannn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnn 408

<210> 173
<211> 351
<212> DNA
<213> TRITICUM TAUSCHII

<400> 173
agtaataaat ggaacaccaa agtaatctgc cgagtcactt cattgactgg ggggtagtct 60
ctgtcgactg catcgctggg tgtgtgtgtg tgttttaatt atttggattg cttaccacat 120
gtgtcatgtg gtgtgtgtgt gtgtgtgtgt gtgtgtgtgt gtgtgtgtgt gttttgagaa 180
gaagaagacc agattaccct ggagcagaat acactttgtt tctgcacttc ttttggactg 240
gccttgtcac ggatgagggg ataggccagc ccatgaacac gaataaaact gtgtgggtag 300
gtaactggcc cccctcgaaa aaaaaggtgg gtaatgggtt ttgcaatccg g 351

<210> 174
<211> 536
<212> DNA
<213> TRITICUM TAUSCHII

<400> 174
ccaggccgac ggatcactaa cgtgggccca catgtcggcc tggcaacagt ggcagcaaat 60
ttatttcgag caaatcgcaa cagtagcaag tggcaacact ggggcacggc ttggcttgct 120
cgctgcccct tcactcctca ctgtggtgtg gggcgcggtg agtgagctat agctgtagct 180
ttgcatgcat gaacacacaa cacaagcaag cagggaggaa cagaggaggg cagcaggcag 240
gcgcaagcga tgtggacttt ttaatactgc tgtgccggat ggcaaagaga ggagaaaagc 300
aaacagcgac gtgacgacgg acggacggct ccttcatcgc ctcacctcac ctatcgctcg 360
ctgctgctgc cttgctccca agtttgcact ctccagtgat cgatccagtc cagtagcgtc 420
tgctgctgct gctgctcaaa gatctttgct ccacgccagc gtgctgcgcc atggaagatg 480

ctactgtggt gtggtgtcag cgtgtggcta gctagctagc atccggaatt catggc 536

<210> 175
<211> 448
<212> DNA
<213> TRITICUM TAUSCHII

<220>
<223> n means a, g, c or t

<400> 175
gaatctgacc gaacaaaatc acacacatca attcttatga agtatacaca cacacgaata 60
ctcagacaca cgaacacaca cattcacaca cacacacaca cacacacaaa tacgactatg 120
gtactacagg gcactgttct aatactctcc gatgaacaaa aacagttcta ctgttagtcc 180
annnnnnnnn nnnnnnnnnn nnnnnnnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnn 448

<210> 176
<211> 542
<212> DNA
<213> TRITICUM TAUSCHII

<400> 176
ctttagctag tgcagcatgc attttagctt tagggtttct tcctttttt cctttctttt 60
ttgcacttta tgattaagct ggctagagag tgcagcatgc attttagcta gtgcagcatg 120
catgatgcat cttttggtcg agcccctgaa acagatatac ccctatctat atatcgatcc 180
atccatgtat atgtatgtat gttacctaaa tcatgttgat gagaagaaaa ggattcagaa 240
aagtcagatt attgtttctg ttttgctctg gtgaacaaat aagtgcggca cgtggaagtg 300
agcccctctg ctctgcaaat tgcagggttg cttgctttat tttatgtatt tttgttttgg 360
tttttggcat gtgcactgca tatatatgat ctgacatgag ctgctgcagc tgacaaccac 420
gaacagcaag aagaagcaag caagcaaccc tttccttccg tcctctatat cacaagcttc 480
ttgctgggct ggtggtggca tcgcatcgca caggagagga aggaaggaag aaggcgacct 540
cc 542

<210> 177
<211> 543
<212> DNA
<213> TRITICUM TAUSCHII

<220>
<223> n means a, g, c or t

<400> 177
ctgtgtagag gaagcatgaa gttagtggga aaaatgaaca tgacctcggc cgaaatacat 60
ataatttgca tcagatgaca ggtcataata ttttgcatca atagaagatg aaccaaaata 120
gtcttaaagc tccggtttga gcatcaacag tagatgaacc aaaatagcta agctccagtt 180
taagcaacac acatcaggct ctagtttggc catcaggctc tagtttgagc atcacacaca 240
cacacacaca aacacacaca cacacacaca cacaagttca acctctagtt tgagcatcac 300
acgcatacaa gttccagctc cagtttgcgc atcacacaca catacatggt ccagttcaag 360
caaattcatc ccccaagcca ataaagcaga tttttcttgg ttcctagagt tggcttgaga 420
acagcatcac tggtagatgg tgtaacaaac tgcagttttt tgctcttctt ccctgcagnt 480
ttctcctttc tagttagtcc annnnnnnnn nnnnnnnnnn nnnnnnnnnn nnnnnnnnnn 540
nnn 543

<210> 178
<211> 392
<212> DNA
<213> TRITICUM TAUSCHII

<400> 178
ctgttaaaca accttacaga aaccctcact ttggacagat taaacaacca gttcaagatc 60
tagtggacaa acaagggtag ccatagatgg ccctacctgt ccaacaacaa caacaacaac 120
aacaacaaca acaacctgat caagtggaag tccctgtaga aggccagaga agacttgcta 180
aagatccact gctaactggc agaagaaacc aaactcaggt caagcctgct aaaatgaaca 240
tgcctgaatt tgaaggagat aacacagact cctggattca aaccatggac ccttactttg 300
acactgctag aaccccacct gaaactaaaa ctgagataat agtcacctac ttgaaaggac 360
ctgccattga gtggtggaga ggttagtcca cg 392

<210> 179
<211> 216
<212> DNA
<213> TRITICUM TAUSCHII

<400> 179
actgaatgca cgtgacttct actataatag gtaatattca ggttaccgca catgcatgtg 60
tgtgtgcgcg tgtgcgtgtg ggtgtgggtg tgggtgtggg tgggtgtgtt ggtgtgtgtg 120
tgtgtgtgtg tgtgtgtgtg tgtgtgtgtg tgtgtgtgtg tgtgtgtgtg tgctagccca 180
caacaattta taactttacc cttgtttttc ttttgt 216

<210> 180
<211> 415

<212> DNA
<213> TRITICUM TAUSCHII

<400> 180
caaaagttat aatttaaaat tctagaattc actttgacaa ggagaactat atacaaaatt 60
tcaaataatc tgggtatgct actgtcgagc acagacattt atattgccga aaagcttaat 120
gcttctgtag ttacatcata attcggcagc ttaatatttt catagaagat cctatttaag 180
cattcatgtc atcaccaatt atgtgtggag tttaatatta gttgcaatta tatagtgttg 240
gatacaatag gtgcctccgc ccagttagaa aactctgcat aaattctgtg acttatggtc 300
taactatttg cgtgtgtgtg tgtgtgtgcg tgcgtgcgtg cgtgcgtgtg tgtgtgtgtg 360
tgtgtgtgtt attatattga tttcaaacag aagcatttac ataatggtag tccac 415

<210> 181
<211> 531
<212> DNA
<213> TRITICUM TAUSCHII

<220>
<223> n means a, g, c or t

<400> 181
ctattttca attattgata aagttcaaca aaataaatac tcttatattg ataagcatac 60
attgaatttt ttggttactt tgtcggacaa tataacaaca atgtggagat ggcacaatat 120
aagataattt attttgttta acttttatca atataacaag acttgagagg aactctgggt 180
ttgtgtgtgt gtgtgtgtgt gtgtgtgtgt gtgtgttctc acattgaaac taggcacttt 240
ttgaacgatc aatggaagtg cagctggtag actagtagtc cannnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn n 531

<210> 182
<211> 464
<212> DNA
<213> TRITICUM TAUSCHII

<220>
<223> n means a, g, c or t

<400> 182
cagaataatg gccatgtaat taaggcacat tacaaaatgc ataaactaat aatatgtcat 60
tattatcatc ttacacacac acacacacac acacacacac acacacacac acacacacac 120
atatatatat ataggactcc tcttctgtac tcctgggagt atgtagtcca nnnnnnnnnn 180
nnnnnnnnnn nnnnnnnnnn nnnnnnnnnn nnnnnnnnnn nnnnnnnnnn nnnnnnnnnn 240
nnnnnnnnnn nnnnnnnnnn nnnnnnnnnn nnnnnnnnnn nnnnnnnnnn nnnnnnnnnn 300
nnnnnnnnnn nnnnnnnnnn nnnnnnnnnn nnnnnnnnnn nnnnnnnnnn nnnnnnnnnn 360
nnnnnnnnnn nnnnnnnnnn nnnnnnnnnn nnnnnnnnnn nnnnnnnnnn nnnnnnnnnn 420
nnnnnnnnnn nnnnnnnnnn nnnnnnnnnt cttccgcttc ctcg 464

<210> 183
<211> 224
<212> DNA
<213> TRITICUM TAUSCHII

<400> 183
actaccaaat ataaagccgc ctcgtcttct ccctattcta ctgtcacagc ctctgcaagt 60
ctttaccgtg tgcgcgagtg tgtgttacgg agacgatgaa tgctgttaag ctgaaggaga 120
cgggcaaggg tggcgacgcg gcggcggcgg cggcggcggc ggtgaccctg gaggagctga 180
ggaagaagat ggccgacttc gccagggaga gggactggga gaag 224

<210> 184
<211> 518
<212> DNA
<213> TRITICUM TAUSCHII

<220>
<223> n means a, g, c or t

<400> 184
acttatagaa tcatacttga tactaccacg gcgctaatcc agtgactcaa gccattcatt 60
catcgttggt tagtagccag agccatgccc tccacccctc ttggaaccgc ctccttgtcc 120
gccgccttgc ccgagtggac cgtttatccg taagcatata gcccgacacc atggccccgg 180
ctaaacccaa gaaatcgttg agtcgcttcg agaaccactt cgaccgtctg cattggttgt 240
ccatagggca gaacagaatt catcagcgca cacacacaca cacacacaca cacacacaca 300
cacacacaaa tggatcagca gcataggcac agagtaaaat accacaatgg gtcggcgggc 360
ctaggatgga tctgcacggc aagctgctgg agcatgcgtt agtccannnn nnnnnnnnnn 420
nnnnnnnnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnn 518

<210> 185
<211> 550
<212> DNA
<213> TRITICUM TAUSCHII

<400> 185

```
ctgtgggact ttgttggatt tgtttagtag ttgtagctag cagcaagtag ttgcatatcg 60
agaatgatat gttcgtaaga ttttgaaaac ttctgaatcg acatgccaaa ttatagtcag 120
ttatgaaatt ccctgactac aactagtaaa acacacacac acacacacac acacacacac 180
acacacacac aaagcagtct agatgaatta atgttttttt ttgtgaaatg ctgacggctc 240
agcttttcat tgcaatagaa ggagcgaacc aatacaaaca agggcggcta aactgctgtt 300
aaaaagcggc taacaacagg aattaatgtt attatcaatt aaatatagca acaaacaagt 360
ccaatagttt taagtaactg tttaaaatta tgatttgaaa ttatttttc cttctaagaa 420
aataataagc ggttttaatt taatcccttt gtatttatta tttatgttaa gaacttaaaa 480
catcagtttt gcgtacttgt gtaaattata attttaaata ctttttcatt caaacctggc 540
atggatgtct                                                        550
```

```
<210> 186
<211> 20
<212> DNA
<213> TRITICUM TAUSCHII

<400> 186
accaaagaac ttgcctggtg                                              20


<210> 187
<211> 20
<212> DNA
<213> TRITICUM TAUSCHII

<400> 187
ggttgcagtt tccaccttgt                                              20


<210> 188
<211> 19
<212> DNA
<213> TRITICUM TAUSCHII

<400> 188
gcaccaacac acggagaag                                               19


<210> 189
<211> 20
<212> DNA
<213> TRITICUM TAUSCHII

<400> 189
tgctccgtct ccgagtagat                                              20


<210> 190
<211> 19
<212> DNA
<213> TRITICUM TAUSCHII

<400> 190
tgccctgtcc acagtgaag                                               19


<210> 191
<211> 20
<212> DNA
<213> TRITICUM TAUSCHII

<400> 191
actctccccc tcgttgctat                                              20


<210> 192
<211> 20
<212> DNA
<213> TRITICUM TAUSCHII

<400> 192
agctaccagc ctagcagcag                                              20


<210> 193
<211> 20
<212> DNA
<213> TRITICUM TAUSCHII

<400> 193
accaccgtca tgtcactgag                                              20


<210> 194
<211> 20
<212> DNA
<213> TRITICUM TAUSCHII

<400> 194
```

ttgcacgcac ctaaactctg 20

<210> 195
<211> 21
<212> DNA
<213> TRITICUM TAUSCHII

<400> 195
cgttctatga cgtgtcatgc t 21

<210> 196
<211> 20
<212> DNA
<213> TRITICUM TAUSCHII

<400> 196
ggatttgcct tggatttgaa 20

<210> 197
<211> 20
<212> DNA
<213> TRITICUM TAUSCHII

<400> 197
gttacccaaa cctgcccttt 20

<210> 198
<211> 20
<212> DNA
<213> TRITICUM TAUSCHII

<400> 198
ccactaacca agctgccatt 20

<210> 199
<211> 20
<212> DNA
<213> TRITICUM TAUSCHII

<400> 199
ccaccggcca gagtagtatt 20

<210> 200
<211> 20
<212> DNA
<213> TRITICUM TAUSCHII

<400> 200
ctcccgtatt gagcaggaag 20

<210> 201
<211> 20
<212> DNA
<213> TRITICUM TAUSCHII

<400> 201
ggatccaagg gaatccaaat 20

<210> 202
<211> 20
<212> DNA
<213> TRITICUM TAUSCHII

<400> 202
agcacagaag gggttagggt 20

<210> 203
<211> 20
<212> DNA
<213> TRITICUM TAUSCHII

<400> 203
catccaacag caccaagaga 20

<210> 204
<211> 20
<212> DNA
<213> TRITICUM TAUSCHII

<400> 204

tacgcaggtt tgctgcttct 20

<210> 205
<211> 20
<212> DNA
<213> TRITICUM TAUSCHII

<400> 205
tgatgggaag gtaatgggag 20

<210> 206
<211> 20
<212> DNA
<213> TRITICUM TAUSCHII

<400> 206
cctccatgta ggcggaaata 20

<210> 207
<211> 20
<212> DNA
<213> TRITICUM TAUSCHII

<400> 207
ggttgcaaac cgtcttgttt 20

<210> 208
<211> 20
<212> DNA
<213> TRITICUM TAUSCHII

<400> 208
tagcagtagc agcagcagga 20

<210> 209
<211> 20
<212> DNA
<213> TRITICUM TAUSCHII

<400> 209
ggcgcaatct gaaagaaaag 20

<210> 210
<211> 20
<212> DNA
<213> TRITICUM TAUSCHII

<400> 210
catcgctcat gctaaggtca 20

<210> 211
<211> 20
<212> DNA
<213> TRITICUM TAUSCHII

<400> 211
tcaagatcgt gccaaatcaa 20

<210> 212
<211> 20
<212> DNA
<213> TRITICUM TAUSCHII

<400> 212
gcagcaagat caaatcgaca 20

<210> 213
<211> 23
<212> DNA
<213> TRITICUM TAUSCHII

<400> 213
tgcatcttat tactggaggc att 23

<210> 214
<211> 21
<212> DNA
<213> TRITICUM TAUSCHII

<400> 214

ggttgtcagg caggatattt g 21

<210> 215
<211> 20
<212> DNA
<213> TRITICUM TAUSCHII

<400> 215
aatcgcacaa caatggttca 20

<210> 216
<211> 20
<212> DNA
<213> TRITICUM TAUSCHII

<400> 216
gcaccaacct tgatagggaa 20

<210> 217
<211> 20
<212> DNA
<213> TRITICUM TAUSCHII

<400> 217
caacacaacc acaatttccg 20

<210> 218
<211> 20
<212> DNA
<213> TRITICUM·TAUSCHII

<400> 218
taccgcaata atcacaccca 20

<210> 219
<211> 20
<212> DNA
<213> TRITICUM TAUSCHII

<400> 219
ggaagaaccg caacagacat 20

<210> 220
<211> 20
<212> DNA
<213> TRITICUM TAUSCHII

<400> 220
gggatgacac ataacggaca 20

<210> 221
<211> 20
<212> DNA
<213> TRITICUM TAUSCHII

<400> 221
gcaaagtgta gccgaggaag 20

<210> 222
<211> 20
<212> DNA
<213> TRITICUM TAUSCHII

<400> 222
gcttcttttg ctgcttttgc 20

<210> 223
<211> 20
<212> DNA
<213> TRITICUM TAUSCHII

<400> 223
tggccattcg atattcaaaa 20

<210> 224
<211> 23
<212> DNA
<213> TRITICUM TAUSCHII

<400> 224

ccacagctac atcatctttc ctt 23

<210> 225
<211> 21
<212> DNA
<213> TRITICUM TAUSCHII

<400> 225
gcgacaagta attcagaacg g 21

<210> 226
<211> 20
<212> DNA
<213> TRITICUM TAUSCHII

<400> 226
taaagtctca ggcgacccac 20

<210> 227
<211> 20
<212> DNA
<213> TRITICUM TAUSCHII

<400> 227
aggttctagg gggcatgtct 20

<210> 228
<211> 20
<212> DNA
<213> TRITICUM TAUSCHII

<400> 228
aacaaaagtc ggtgcagtcc 20

<210> 229
<211> 20
<212> DNA
<213> TRITICUM TAUSCHII

<400> 229
aaacccaatg gctctcacac 20

<210> 230
<211> 20
<212> DNA
<213> TRITICUM TAUSCHII

<400> 230
tctctccagt tgctcctcgt 20

<210> 231
<211> 21
<212> DNA
<213> TRITICUM TAUSCHII

<400> 231
tggtggtata gtcgttggag c 21

<210> 232
<211> 25
<212> DNA
<213> TRITICUM TAUSCHII

<400> 232
tgaccatgtc atgttttata ccact 25

<210> 233
<211> 20
<212> DNA
<213> TRITICUM TAUSCHII

<400> 233
atggttgatg gtgggtgttt 20

<210> 234
<211> 20
<212> DNA
<213> TRITICUM TAUSCHII

<400> 234

tgagttcttc tggtgaggca 20

<210> 235
<211> 20
<212> DNA
<213> TRITICUM TAUSCHII

<400> 235
ttctgcaaca ttttgtccca 20

<210> 236
<211> 20
<212> DNA
<213> TRITICUM TAUSCHII

<400> 236
ggaggcttct ctatgggagg 20

<210> 237
<211> 20
<212> DNA
<213> TRITICUM TAUSCHII

<400> 237
agacggtgtt ggttcctgag 20

<210> 238
<211> 20
<212> DNA
<213> TRITICUM TAUSCHII

<400> 238
ccctatttcc cccatgtctt 20

<210> 239
<211> 20
<212> DNA
<213> TRITICUM TAUSCHII

<400> 239
tcgttccaaa atgcatgaaa 20

<210> 240
<211> 20
<212> DNA
<213> TRITICUM TAUSCHII

<400> 240
ccagtagccg gccctactat 20

<210> 241
<211> 21
<212> DNA
<213> TRITICUM TAUSCHII

<400> 241
ttgcataatt acttgccctc c 21

<210> 242
<211> 20
<212> DNA
<213> TRITICUM TAUSCHII

<400> 242
atcgccgtta acataggcag 20

<210> 243
<211> 21
<212> DNA
<213> TRITICUM TAUSCHII

<400> 243
aatgggcctt taagagcaaa a 21

<210> 244
<211> 20
<212> DNA
<213> TRITICUM TAUSCHII

<400> 244

tcacctggaa aatggtcaca 20

<210> 245
<211> 20
<212> DNA
<213> TRITICUM TAUSCHII

<400> 245
tgaccggcat tcagtatcaa 20

<210> 246
<211> 20
<212> DNA
<213> TRITICUM TAUSCHII

<400> 246
attcaaatgc aacgcaaaca 20

<210> 247
<211> 20
<212> DNA
<213> TRITICUM TAUSCHII

<400> 247
caagagctga ccaatgtgga 20

<210> 248
<211> 20
<212> DNA
<213> TRITICUM TAUSCHII

<400> 248
tcctgaggat gttgaggacc 20

<210> 249
<211> 20
<212> DNA
<213> TRITICUM TAUSCHII

<400> 249
acagtgttgt tgcccctttc 20

<210> 250
<211> 20
<212> DNA
<213> TRITICUM TAUSCHII

<400> 250
agacgatgag aaggaagcca 20

<210> 251
<211> 20
<212> DNA
<213> TRITICUM TAUSCHII

<400> 251
aggtcttggt ggttttggtg 20

<210> 252
<211> 20
<212> DNA
<213> TRITICUM TAUSCHII

<400> 252
gcggacaaat tgagccttag 20

<210> 253
<211> 20
<212> DNA
<213> TRITICUM TAUSCHII

<400> 253
tttgcagcat cacacgtttt 20

<210> 254
<211> 23
<212> DNA
<213> TRITICUM TAUSCHII

<400> 254

aaataccttg aattgtgagc tgc 23

<210> 255
<211> 20
<212> DNA
<213> TRITICUM TAUSCHII

<400> 255
gtcggcatag tcgcacatac 20

<210> 256
<211> 20
<212> DNA
<213> TRITICUM TAUSCHII

<400> 256
caataagtag gccgggacaa 20

<210> 257
<211> 20
<212> DNA
<213> TRITICUM TAUSCHII

<400> 257
ctccttggaa tctcaccgaa 20

<210> 258
<211> 20
<212> DNA
<213> TRITICUM TAUSCHII

<400> 258
gatagatcaa tgtgggccgt 20

<210> 259
<211> 20
<212> DNA
<213> TRITICUM TAUSCHII

<400> 259
tcaaaaccac accaggcata 20

<210> 260
<211> 20
<212> DNA
<213> TRITICUM TAUSCHII

<400> 260
gcataaactt ggaccctgga 20

<210> 261
<211> 20
<212> DNA
<213> TRITICUM TAUSCHII

<400> 261
gcaatttcac acgcgactta 20

<210> 262
<211> 20
<212> DNA
<213> TRITICUM TAUSCHII

<400> 262
ctgcttcagg gattggagag 20

<210> 263
<211> 20
<212> DNA
<213> TRITICUM TAUSCHII

<400> 263
atgaaatcct tgccctcaga 20

<210> 264
<211> 20
<212> DNA
<213> TRITICUM TAUSCHII

<400> 264

tctggttctt gggaggaaga 20

<210> 265
<211> 21
<212> DNA
<213> TRITICUM TAUSCHII

<400> 265
ataggggttt tgaatcactc c 21

<210> 266
<211> 20
<212> DNA
<213> TRITICUM TAUSCHII

<400> 266
tatccccaat cccctctttc 20

<210> 267
<211> 20
<212> DNA
<213> TRITICUM TAUSCHII

<400> 267
gctgatgctg ctgtaagtgc 20

<210> 268
<211> 20
<212> DNA
<213> TRITICUM TAUSCHII

<400> 268
aaggatggag aggaccccta 20

<210> 269
<211> 20
<212> DNA
<213> TRITICUM TAUSCHII

<400> 269
gttgcctcgg tgtcgtttat 20

<210> 270
<211> 21
<212> DNA
<213> TRITICUM TAUSCHII

<400> 270
ttaatgagcg tcagtactcc c 21

<210> 271
<211> 20
<212> DNA
<213> TRITICUM TAUSCHII

<400> 271
taggcatagt tttgggcctg 20

<210> 272
<211> 25
<212> DNA
<213> TRITICUM TAUSCHII

<400> 272
ttagcagata agagctaagc tatgg 25

<210> 273
<211> 20
<212> DNA
<213> TRITICUM TAUSCHII

<400> 273
cttgttgatc tccttcccca 20

<210> 274
<211> 23
<212> DNA
<213> TRITICUM TAUSCHII

<400> 274

aatcctgact ttaaagcctt tcc 23

<210> 275
<211> 20
<212> DNA
<213> TRITICUM TAUSCHII

<400> 275
tcgaccgaag agagcagaat 20

<210> 276
<211> 21
<212> DNA
<213> TRITICUM TAUSCHII

<400> 276
ttgtggaagg gtttgatgaa g 21

<210> 277
<211> 19
<212> DNA
<213> TRITICUM TAUSCHII

<400> 277
acgggtggtt ttgctcagt 19

<210> 278
<211> 20
<212> DNA
<213> TRITICUM TAUSCHII

<400> 278
tttgcccatt acaacaagca 20

<210> 279
<211> 20
<212> DNA
<213> TRITICUM TAUSCHII

<400> 279
taaacaccag ggaggtccac 20

<210> 280
<211> 18
<212> DNA
<213> TRITICUM TAUSCHII

<400> 280
caaatgctaa tccccgcc 18

<210> 281
<211> 18
<212> DNA
<213> TRITICUM TAUSCHII

<400> 281
ggatctcggg gatgtcct 18

<210> 282
<211> 20
<212> DNA
<213> TRITICUM TAUSCHII

<400> 282
cgtaaagatc cgagagggtg 20

<210> 283
<211> 20
<212> DNA
<213> TRITICUM TAUSCHII

<400> 283
ttctccatgg gcagctactt 20

<210> 284
<211> 20
<212> DNA
<213> TRITICUM TAUSCHII

<400> 284

tggaagtctg gaaccactcc 20

<210> 285
<211> 20
<212> DNA
<213> TRITICUM TAUSCHII

<400> 285
agcagtgtaa taaaagggcg 20

<210> 286
<211> 20
<212> DNA
<213> TRITICUM TAUSCHII

<400> 286
ccactactgc ggctaggtct 20

<210> 287
<211> 20
<212> DNA
<213> TRITICUM TAUSCHII

<400> 287
gtaaggcatc ttcgcgtctc 20

<210> 288
<211> 20
<212> DNA
<213> TRITICUM TAUSCHII

<400> 288
tttccttgga tccactcacc 20

<210> 289
<211> 20
<212> DNA
<213> TRITICUM TAUSCHII

<400> 289
cttcgcaaat cgaggatgat 20

<210> 290
<211> 17
<212> DNA
<213> TRITICUM TAUSCHII

<400> 290
ctgtcggacg acgacga 17

<210> 291
<211> 20
<212> DNA
<213> TRITICUM TAUSCHII

<400> 291
tgtcggggac actctctctt 20

<210> 292
<211> 24
<212> DNA
<213> TRITICUM TAUSCHII

<400> 292
acttgcactt gctatactta cgaa 24

<210> 293
<211> 20
<212> DNA
<213> TRITICUM TAUSCHII

<400> 293
aatggcttca ctgtttgcct 20

<210> 294
<211> 20
<212> DNA
<213> TRITICUM TAUSCHII

<400> 294

gctaaagcca cataggacgg 20

<210> 295
<211> 20
<212> DNA
<213> TRITICUM TAUSCHII

<400> 295
caatcagaag cgccattgtt 20

<210> 296
<211> 19
<212> DNA
<213> TRITICUM TAUSCHII

<400> 296
gctgccgcta ctgtctgtc 19

<210> 297
<211> 20
<212> DNA
<213> TRITICUM TAUSCHII

<400> 297
acaagaccac acctccaagg 20

<210> 298
<211> 20
<212> DNA
<213> TRITICUM TAUSCHII

<400> 298
agaagactgc acgcaaggat 20

<210> 299
<211> 20
<212> DNA
<213> TRITICUM TAUSCHII

<400> 299
gaggtggttg ctgcctagat 20

<210> 300
<211> 20
<212> DNA
<213> TRITICUM TAUSCHII

<400> 300
ggcccatctg tcattgactt 20

<210> 301
<211> 22
<212> DNA
<213> TRITICUM TAUSCHII

<400> 301
aagagctaca atgaccagca ga 22

<210> 302
<211> 25
<212> DNA
<213> TRITICUM TAUSCHII

<400> 302
ccacaaactt tgatgaatga ttact 25

<210> 303
<211> 20
<212> DNA
<213> TRITICUM TAUSCHII

<400> 303
gaatttttgg tggcctgtgt 20

<210> 304
<211> 20
<212> DNA
<213> TRITICUM TAUSCHII

<400> 304

gttgaggagg acaaagaggc 20

<210> 305
<211> 20
<212> DNA
<213> TRITICUM TAUSCHII

<400> 305
ctctcgttct ctccaggctc 20

<210> 306
<211> 20
<212> DNA
<213> TRITICUM TAUSCHII

<400> 306
ccagtttgca gcagtcacat 20

<210> 307
<211> 20
<212> DNA
<213> TRITICUM TAUSCHII

<400> 307
gaacatgaag acgaggaggc 20

<210> 308
<211> 20
<212> DNA
<213> TRITICUM TAUSCHII

<400> 308
aagtcgacag gtcggttgag 20

<210> 309
<211> 20
<212> DNA
<213> TRITICUM TAUSCHII

<400> 309
tctcaacttg caactgccac 20

<210> 310
<211> 20
<212> DNA
<213> TRITICUM TAUSCHII

<400> 310
gaaaacaaaa cccatttgcg 20

<210> 311
<211> 19
<212> DNA
<213> TRITICUM TAUSCHII

<400> 311
gtgcgtcgtg tagcagctc 19

<210> 312
<211> 20
<212> DNA
<213> TRITICUM TAUSCHII

<400> 312
agcatgtgtg tctcctcgtg 20

<210> 313
<211> 20
<212> DNA
<213> TRITICUM TAUSCHII

<400> 313
tctcatccct gttcctctgc 20

<210> 314
<211> 17
<212> DNA
<213> TRITICUM TAUSCHII

<400> 314

cccgtggtct tgggttc 17

<210> 315
<211> 20
<212> DNA
<213> TRITICUM TAUSCHII

<400> 315
tcaagaagat gcgttcatgc 20

<210> 316
<211> 20
<212> DNA
<213> TRITICUM TAUSCHII

<400> 316
gacgagctaa ctgcagaccc 20

<210> 317
<211> 19
<212> DNA
<213> TRITICUM TAUSCHII

<400> 317
ctaatctaag caagggccg 19

<210> 318
<211> 20
<212> DNA
<213> TRITICUM TAUSCHII

<400> 318
tgggtatgaa aggctgaagg 20

<210> 319
<211> 20
<212> DNA
<213> TRITICUM TAUSCHII

<400> 319
ccccttttgg tctgctgtaa 20

<210> 320
<211> 24
<212> DNA
<213> TRITICUM TAUSCHII

<400> 320
tcaaatgatt tcaggtaacc acta 24

<210> 321
<211> 20
<212> DNA
<213> TRITICUM TAUSCHII

<400> 321
cagccgaaga aggatttctg 20

<210> 322
<211> 20
<212> DNA
<213> TRITICUM TAUSCHII

<400> 322
taatttgatt gggtcggagc 20

<210> 323
<211> 20
<212> DNA
<213> TRITICUM TAUSCHII

<400> 323
tggaagttac cctgtccgtc 20

<210> 324
<211> 20
<212> DNA
<213> TRITICUM TAUSCHII

<400> 324

cccattgcca tctcagtctt 20

<210> 325
<211> 20
<212> DNA
<213> TRITICUM TAUSCHII

<400> 325
tctgaaccct tgattttccg 20

<210> 326
<211> 20
<212> DNA
<213> TRITICUM TAUSCHII

<400> 326
cgaaaaacca tgatcgacag 20

<210> 327
<211> 20
<212> DNA
<213> TRITICUM TAUSCHII

<400> 327
tctactttca gggcacctcg 20

<210> 328
<211> 20
<212> DNA
<213> TRITICUM TAUSCHII

<400> 328
ccttcttaat gaccggctcg 20

<210> 329
<211> 20
<212> DNA
<213> TRITICUM TAUSCHII

<400> 329
tgcgaagtat tcagtgcgtc 20

<210> 330
<211> 20
<212> DNA
<213> TRITICUM TAUSCHII

<400> 330
cctggcagag aaagtgaagg 20

<210> 331
<211> 20
<212> DNA
<213> TRITICUM TAUSCHII

<400> 331
gctgctaagt gctcatggtg 20

<210> 332
<211> 20
<212> DNA
<213> TRITICUM TAUSCHII

<400> 332
tcactacccg catgaacaaa 20

<210> 333
<211> 20
<212> DNA
<213> TRITICUM TAUSCHII

<400> 333
attggaaggc cacgatacac 20

<210> 334
<211> 20
<212> DNA
<213> TRITICUM TAUSCHII

<400> 334

taaatggcca tcaagcaatg 20

<210> 335
<211> 22
<212> DNA
<213> TRITICUM TAUSCHII

<400> 335
cggtctttgt ttgctctaaa cc 22

<210> 336
<211> 20
<212> DNA
<213> TRITICUM TAUSCHII

<400> 336
gttgcacgac ctacaaagca 20

<210> 337
<211> 20
<212> DNA
<213> TRITICUM TAUSCHII

<400> 337
tttacgggga cagtattcgg 20

<210> 338
<211> 20
<212> DNA
<213> TRITICUM TAUSCHII

<400> 338
tgcctaaatc taaatgcccg 20

<210> 339
<211> 20
<212> DNA
<213> TRITICUM TAUSCHII

<400> 339
gaatggaagg cggacataga 20

<210> 340
<211> 20
<212> DNA
<213> TRITICUM TAUSCHII

<400> 340
tcatccccta cataacccga 20

<210> 341
<211> 20
<212> DNA
<213> TRITICUM TAUSCHII

<400> 341
cttggagctc gggtagtagc 20

<210> 342
<211> 20
<212> DNA
<213> TRITICUM TAUSCHII

<400> 342
ttgtgcatga tggcttcaat 20

<210> 343
<211> 19
<212> DNA
<213> TRITICUM TAUSCHII

<400> 343
tttgttacaa cccaggggg 19

<210> 344
<211> 21
<212> DNA
<213> TRITICUM TAUSCHII

<400> 344

tttcgtcttc aaaatgcact g 21

<210> 345
<211> 20
<212> DNA
<213> TRITICUM TAUSCHII

<400> 345
ttgatccttg atgggaggag 20

<210> 346
<211> 20
<212> DNA
<213> TRITICUM TAUSCHII

<400> 346
gggttggtgc ctagattgaa 20

<210> 347
<211> 20
<212> DNA
<213> TRITICUM TAUSCHII

<400> 347
tggcaagtaa catgaacgga 20

<210> 348
<211> 20
<212> DNA
<213> TRITICUM TAUSCHII

<400> 348
gacatactcc ggcgttgaat 20

<210> 349
<211> 20
<212> DNA
<213> TRITICUM TAUSCHII

<400> 349
acggcatcac aggttaaagg 20

<210> 350
<211> 20
<212> DNA
<213> TRITICUM TAUSCHII

<400> 350
gcatcgactt cctcctccta 20

<210> 351
<211> 20
<212> DNA
<213> TRITICUM TAUSCHII

<400> 351
tcttggatgg atttgtgagc 20

<210> 352
<211> 20
<212> DNA
<213> TRITICUM TAUSCHII

<400> 352
ttcttcagtt gttttggggg 20

<210> 353
<211> 20
<212> DNA
<213> TRITICUM TAUSCHII

<400> 353
tagcaaaggg tgcatgtgag 20

<210> 354
<211> 20
<212> DNA
<213> TRITICUM TAUSCHII

<400> 354

cagtctgcaa tccactttgc 20

<210> 355
<211> 20
<212> DNA
<213> TRITICUM TAUSCHII

<400> 355
agagcaggag gttgggttct 20

<210> 356
<211> 20
<212> DNA
<213> TRITICUM TAUSCHII

<400> 356
acatgtgatg tgcggtcatt 20

<210> 357
<211> 20
<212> DNA
<213> TRITICUM TAUSCHII

<400> 357
caaaccaacc tcattgaccc 20

<210> 358
<211> 20
<212> DNA
<213> TRITICUM TAUSCHII

<400> 358
tctgccgagt cacttcattg 20

<210> 359
<211> 20
<212> DNA
<213> TRITICUM TAUSCHII

<400> 359
ggagaaaagc aaacagcgac 20

<210> 360
<211> 22
<212> DNA
<213> TRITICUM TAUSCHII

<400> 360
aatctgaccg aacaaaatca ca 22

<210> 361
<211> 20
<212> DNA
<213> TRITICUM TAUSCHII

<400> 361
tgcagcatgc attttagctt 20

<210> 362
<211> 20
<212> DNA
<213> TRITICUM TAUSCHII

<400> 362
ttggccatca ggctctagtt 20

<210> 363
<211> 20
<212> DNA
<213> TRITICUM TAUSCHII

<400> 363
agccatagat ggccctacct 20

<210> 364
<211> 23
<212> DNA
<213> TRITICUM TAUSCHII

<400> 364

ggtaatattc aggttaccgc aca 23

<210> 365
<211> 20
<212> DNA
<213> TRITICUM TAUSCHII

<400> 365
gatacaatag gtgcctccgc 20

<210> 366
<211> 20
<212> DNA
<213> TRITICUM TAUSCHII

<400> 366
acaatgtgga gatggcacaa 20

<210> 367
<211> 20
<212> DNA
<213> TRITICUM TAUSCHII

<400> 367
ggccatgtaa ttaaggcaca 20

<210> 368
<211> 20
<212> DNA
<213> TRITICUM TAUSCHII

<400> 368
gcctctgcaa gtctttaccg 20

<210> 369
<211> 20
<212> DNA
<213> TRITICUM TAUSCHII

<400> 369
ggaccgttta tccgtaagca 20

<210> 370
<211> 21
<212> DNA
<213> TRITICUM TAUSCHII

<400> 370
tgggactttg ttggatttgt t 21

<210> 371
<211> 20
<212> DNA
<213> TRITICUM TAUSCHII

<400> 371
aagcctgacc tagcccaaat 20

<210> 372
<211> 20
<212> DNA
<213> TRITICUM TAUSCHII

<400> 372
catctattgc caaaatcgca 20

<210> 373
<211> 20
<212> DNA
<213> TRITICUM TAUSCHII

<400> 373
ttgagaggag ggcttggtta 20

<210> 374
<211> 20
<212> DNA
<213> TRITICUM TAUSCHII

<400> 374

gggaaggaga gatgggaaac 20

<210> 375
<211> 20
<212> DNA
<213> TRITICUM TAUSCHII

<400> 375
ttgccagttc caaggagaat 20

<210> 376
<211> 20
<212> DNA
<213> TRITICUM TAUSCHII

<400> 376
atttaaggga gacatcgggc 20

<210> 377
<211> 21
<212> DNA
<213> TRITICUM TAUSCHII

<400> 377
tcagacacgt ctcctgacaa a 21

<210> 378
<211> 20
<212> DNA
<213> TRITICUM TAUSCHII

<400> 378
gtgaagacga caagacgcaa 20

<210> 379
<211> 16
<212> DNA
<213> TRITICUM TAUSCHII

<400> 379
caagtgtgag cgtcgg 16

<210> 380
<211> 21
<212> DNA
<213> TRITICUM TAUSCHII

<400> 380
tccattttca aaaacaccct g 21

<210> 381
<211> 20
<212> DNA
<213> TRITICUM TAUSCHII

<400> 381
ttgcatctca cgcacctaag 20

<210> 382
<211> 20
<212> DNA
<213> TRITICUM TAUSCHII

<400> 382
ctacgagtcg ggatcagcat 20

<210> 383
<211> 20
<212> DNA
<213> TRITICUM TAUSCHII

<400> 383
tttttggcat tgatctgctg 20

<210> 384
<211> 22
<212> DNA
<213> TRITICUM TAUSCHII

<400> 384

tcctggtcta acaacgagaa ga 22

<210> 385
<211> 20
<212> DNA
<213> TRITICUM TAUSCHII

<400> 385
ggcaggtgtg gtgatgatct 20

<210> 386
<211> 20
<212> DNA
<213> TRITICUM TAUSCHII

<400> 386
tccttcggtt cccatatcac 20

<210> 387
<211> 20
<212> DNA
<213> TRITICUM TAUSCHII

<400> 387
agctgcggtg tgagctaaat 20

<210> 388
<211> 24
<212> DNA
<213> TRITICUM TAUSCHII

<400> 388
gctactacta tttcattgcg acca 24

<210> 389
<211> 20
<212> DNA
<213> TRITICUM TAUSCHII

<400> 389
ggagttcaca agcatgggtt 20

<210> 390
<211> 20
<212> DNA
<213> TRITICUM TAUSCHII

<400> 390
atccagttct cgtccaaagc 20

<210> 391
<211> 20
<212> DNA
<213> TRITICUM TAUSCHII

<400> 391
tgtgtcccat tcactaaccg 20

<210> 392
<211> 20
<212> DNA
<213> TRITICUM TAUSCHII

<400> 392
agtcgagttg cgaccaaagt 20

<210> 393
<211> 20
<212> DNA
<213> TRITICUM TAUSCHII

<400> 393
gcaaggaaga gtgttcagcc 20

<210> 394
<211> 19
<212> DNA
<213> TRITICUM TAUSCHII

<400> 394

ccaggtccca ctttcgtct 19

<210> 395
<211> 20
<212> DNA
<213> TRITICUM TAUSCHII

<400> 395
cgtgtctgtt agctgggtgg 20

<210> 396
<211> 20
<212> DNA
<213> TRITICUM TAUSCHII

<400> 396
actccaagct gagcacgttt 20

<210> 397
<211> 20
<212> DNA
<213> TRITICUM TAUSCHII

<400> 397
actgaggact tggtgccatc 20

<210> 398
<211> 20
<212> DNA
<213> TRITICUM TAUSCHII

<400> 398
cgcatgccct tataccaact 20

<210> 399
<211> 20
<212> DNA
<213> TRITICUM TAUSCHII

<400> 399
tattgataga tcagggcgca 20

<210> 400
<211> 20
<212> DNA
<213> TRITICUM TAUSCHII

<400> 400
gcctctcctc tctgctcctt 20

<210> 401
<211> 20
<212> DNA
<213> TRITICUM TAUSCHII

<400> 401
gtgcctgatg attttacccg 20

<210> 402
<211> 20
<212> DNA
<213> TRITICUM TAUSCHII

<400> 402
ctcagggagg tcatgcagag 20

<210> 403
<211> 20
<212> DNA
<213> TRITICUM TAUSCHII

<400> 403
ggtcgatgga ctgtccctaa 20

<210> 404
<211> 20
<212> DNA
<213> TRITICUM TAUSCHII

<400> 404

gcatcttctc ctccctcctc 20

<210> 405
<211> 20
<212> DNA
<213> TRITICUM TAUSCHII

<400> 405
atcagcggcg ctatagtacg 20

<210> 406
<211> 20
<212> DNA
<213> TRITICUM TAUSCHII

<400> 406
ttagagtttt gcagcgcctt 20

<210> 407
<211> 20
<212> DNA
<213> TRITICUM TAUSCHII

<400> 407
cccccacata cagaggctaa 20

<210> 408
<211> 20
<212> DNA
<213> TRITICUM TAUSCHII

<400> 408
gtgagttgag gcgcatgata 20

<210> 409
<211> 20
<212> DNA
<213> TRITICUM TAUSCHII

<400> 409
caaagtttga acagcagcca 20

<210> 410
<211> 20
<212> DNA
<213> TRITICUM TAUSCHII

<400> 410
cgcttcggta aagtttttgc 20

<210> 411
<211> 20
<212> DNA
<213> TRITICUM TAUSCHII

<400> 411
agtgatagac ggatggcacc 20

<210> 412
<211> 20
<212> DNA
<213> TRITICUM TAUSCHII

<400> 412
gctctcaatg actgcactgg 20

<210> 413
<211> 21
<212> DNA
<213> TRITICUM TAUSCHII

<400> 413
ccaaaaacat ggttaaaggg g 21

<210> 414
<211> 20
<212> DNA
<213> TRITICUM TAUSCHII

<400> 414

atggcccaat tatgcaactc 20

<210> 415
<211> 20
<212> DNA
<213> TRITICUM TAUSCHII

<400> 415
atgtggaacc ggtctactcg 20

<210> 416
<211> 20
<212> DNA
<213> TRITICUM TAUSCHII

<400> 416
ccacacacac acaccatcaa 20

<210> 417
<211> 22
<212> DNA
<213> TRITICUM TAUSCHII

<400> 417
tggactacat gtcaagcaca aa 22

<210> 418
<211> 20
<212> DNA
<213> TRITICUM TAUSCHII

<400> 418
atgtatcgat gaagggccaa 20

<210> 419
<211> 20
<212> DNA
<213> TRITICUM TAUSCHII

<400> 419
gaatcggttc acaagggaaa 20

<210> 420
<211> 20
<212> DNA
<213> TRITICUM TAUSCHII

<400> 420
cgtatgatcc taacgagggc 20

<210> 421
<211> 20
<212> DNA
<213> TRITICUM TAUSCHII

<400> 421
tgcatcttat cctgtgcagc 20

<210> 422
<211> 20
<212> DNA
<213> TRITICUM TAUSCHII

<400> 422
tagtggccgg tggaattaaa 20

<210> 423
<211> 20
<212> DNA
<213> TRITICUM TAUSCHII

<400> 423
aaggagggca catatcgttg 20

<210> 424
<211> 20
<212> DNA
<213> TRITICUM TAUSCHII

<400> 424

aagggccaga aatctgtgtg 20

<210> 425
<211> 20
<212> DNA
<213> TRITICUM TAUSCHII

<400> 425
gcacgagata cggacaatca 20

<210> 426
<211> 20
<212> DNA
<213> TRITICUM TAUSCHII

<400> 426
ctggtccaac ttccatccat 20

<210> 427
<211> 20
<212> DNA
<213> TRITICUM TAUSCHII

<400> 427
tcactgctgt atttgctccg 20

<210> 428
<211> 20
<212> DNA
<213> TRITICUM TAUSCHII

<400> 428
aggggtgaaa ggttggagac 20

<210> 429
<211> 20
<212> DNA
<213> TRITICUM TAUSCHII

<400> 429
aagaaggcta gggttcaggc 20

<210> 430
<211> 20
<212> DNA
<213> TRITICUM TAUSCHII

<400> 430
tggtcacttt gatgagcagg 20

<210> 431
<211> 20
<212> DNA
<213> TRITICUM TAUSCHII

<400> 431
gttagccaag gacccctttc 20

<210> 432
<211> 16
<212> DNA
<213> TRITICUM TAUSCHII

<400> 432
acggcggtga gatgag 16

<210> 433
<211> 20
<212> DNA
<213> TRITICUM TAUSCHII

<400> 433
gagagaggcg aaacatggac 20

<210> 434
<211> 20
<212> DNA
<213> TRITICUM TAUSCHII

<400> 434

cccatgttac agctttgggt 20

<210> 435
<211> 20
<212> DNA
<213> TRITICUM TAUSCHII

<400> 435
cctcccttgt ttttgggatt 20

<210> 436
<211> 20
<212> DNA
<213> TRITICUM TAUSCHII

<400> 436
ttttcacatg cccacagttg 20

<210> 437
<211> 20
<212> DNA
<213> TRITICUM TAUSCHII

<400> 437
tgtgcgtgtg tgtgtgtttt 20

<210> 438
<211> 20
<212> DNA
<213> TRITICUM TAUSCHII

<400> 438
aaaattgtat cccccgtggt 20

<210> 439
<211> 20
<212> DNA
<213> TRITICUM TAUSCHII

<400> 439
tctgttcatc cccaaagtcc 20

<210> 440
<211> 20
<212> DNA
<213> TRITICUM TAUSCHII

<400> 440
actatgccaa ggggagtgtg 20

<210> 441
<211> 20
<212> DNA
<213> TRITICUM TAUSCHII

<400> 441
tgtgccagtt gagtttgctc 20

<210> 442
<211> 20
<212> DNA
<213> TRITICUM TAUSCHII

<400> 442
tccttgggaa tatgcctcct 20

<210> 443
<211> 20
<212> DNA
<213> TRITICUM TAUSCHII

<400> 443
aactgttctg ccatctgagc 20

<210> 444
<211> 20
<212> DNA
<213> TRITICUM TAUSCHII

<400> 444

aagtggtggg gagtgtgtgt 20

<210> 445
<211> 20
<212> DNA
<213> TRITICUM TAUSCHII

<400> 445
gctaagccac gctaccactc 20

<210> 446
<211> 20
<212> DNA
<213> TRITICUM TAUSCHII

<400> 446
cgctcgacaa catgacactt 20

<210> 447
<211> 20
<212> DNA
<213> TRITICUM TAUSCHII

<400> 447
gtttcctggg ctaaaccaca 20

<210> 448
<211> 20
<212> DNA
<213> TRITICUM TAUSCHII

<400> 448
tgagatcatc gccaatcaga 20

<210> 449
<211> 19
<212> DNA
<213> TRITICUM TAUSCHII

<400> 449
catccaacaa tttgcccat 19

<210> 450
<211> 20
<212> DNA
<213> TRITICUM TAUSCHII

<400> 450
ttggatttgc agagccttct 20

<210> 451
<211> 20
<212> DNA
<213> TRITICUM TAUSCHII

<400> 451
gtcaattgtg gcttgtccct 20

<210> 452
<211> 21
<212> DNA
<213> TRITICUM TAUSCHII

<400> 452
tgaagaatac aatggcagca a 21

<210> 453
<211> 20
<212> DNA
<213> TRITICUM TAUSCHII

<400> 453
ggaggtggag caacctatca 20

<210> 454
<211> 20
<212> DNA
<213> TRITICUM TAUSCHII

<400> 454

tcctcgaggt ccaaaacatc 20

<210> 455
<211> 20
<212> DNA
<213> TRITICUM TAUSCHII

<400> 455
gcaaccatgt ttaagccgat 20

<210> 456
<211> 20
<212> DNA
<213> TRITICUM TAUSCHII

<400> 456
ggtagaagga agcttcggga 20

<210> 457
<211> 20
<212> DNA
<213> TRITICUM TAUSCHII

<400> 457
gcagtctttt tcctgcgttc 20

<210> 458
<211> 20
<212> DNA
<213> TRITICUM TAUSCHII

<400> 458
ttctctcatg acggcaacac 20

<210> 459
<211> 25
<212> DNA
<213> TRITICUM TAUSCHII

<400> 459
catctgtatg atattttgga ggtca 25

<210> 460
<211> 20
<212> DNA
<213> TRITICUM TAUSCHII

<400> 460
agaatgcgtc cttttcttgc 20

<210> 461
<211> 20
<212> DNA
<213> TRITICUM TAUSCHII

<400> 461
tgcaggacca aacatagctg 20

<210> 462
<211> 20
<212> DNA
<213> TRITICUM TAUSCHII

<400> 462
actccaccag cggagaaata 20

<210> 463
<211> 20
<212> DNA
<213> TRITICUM TAUSCHII

<400> 463
caagcagcac ctcatgacag 20

<210> 464
<211> 20
<212> DNA
<213> TRITICUM TAUSCHII

<400> 464

acctacgatc gacgaaatgg 20

<210> 465
<211> 20
<212> DNA
<213> TRITICUM TAUSCHII

<400> 465
tgtaaacaag gtcgcaggtg 20

<210> 466
<211> 20
<212> DNA
<213> TRITICUM TAUSCHII

<400> 466
taagcacctt cttcatgggg 20

<210> 467
<211> 20
<212> DNA
<213> TRITICUM TAUSCHII

<400> 467
tccgaggtgc tacctaccag 20

<210> 468
<211> 18
<212> DNA
<213> TRITICUM TAUSCHII

<400> 468
actacttgcg gacggctg 18

<210> 469
<211> 20
<212> DNA
<213> TRITICUM TAUSCHII

<400> 469
gcaaccagac cacactctca 20

<210> 470
<211> 20
<212> DNA
<213> TRITICUM TAUSCHII

<400> 470
gtattcgcac cagaatccgt 20

<210> 471
<211> 20
<212> DNA
<213> TRITICUM TAUSCHII

<400> 471
cttttccgtg tctccctagc 20

<210> 472
<211> 20
<212> DNA
<213> TRITICUM TAUSCHII

<400> 472
ccatgataga tttggacggg 20

<210> 473
<211> 20
<212> DNA
<213> TRITICUM TAUSCHII

<400> 473
gaaacaaccc agggacaaga 20

<210> 474
<211> 20
<212> DNA
<213> TRITICUM TAUSCHII

<400> 474

ttcacgccca gtattaaggc 20

<210> 475
<211> 20
<212> DNA
<213> TRITICUM TAUSCHII

<400> 475
catcctcttg acgccgccgc 20

<210> 476
<211> 20
<212> DNA
<213> TRITICUM TAUSCHII

<400> 476
accaatggga tgcttctttg 20

<210> 477
<211> 20
<212> DNA
<213> TRITICUM TAUSCHII

<400> 477
gtgtgtcggt gtgtggaaag 20

<210> 478
<211> 20
<212> DNA
<213> TRITICUM TAUSCHII

<400> 478
aaatggtccc agcattcaag 20

<210> 479
<211> 20
<212> DNA
<213> TRITICUM TAUSCHII

<400> 479
gcacaagatt ttgcaaggct 20

<210> 480
<211> 22
<212> DNA
<213> TRITICUM TAUSCHII

<400> 480
ccctgatgtt ttcttttct cc 22

<210> 481
<211> 20
<212> DNA
<213> TRITICUM TAUSCHII

<400> 481
ggcacactca cacaccacac 20

<210> 482
<211> 20
<212> DNA
<213> TRITICUM TAUSCHII

<400> 482
cggtttgggt tttgtgatct 20

<210> 483
<211> 22
<212> DNA
<213> TRITICUM TAUSCHII

<400> 483
tgcactaaag catcttcgtg tt 22

<210> 484
<211> 20
<212> DNA
<213> TRITICUM TAUSCHII

<400> 484

tgcaaggacc gaataagagg 20

<210> 485
<211> 20
<212> DNA
<213> TRITICUM TAUSCHII

<400> 485
cagcttgtgt tgctcgctta 20

<210> 486
<211> 22
<212> DNA
<213> TRITICUM TAUSCHII

<400> 486
gcagtgtatg tcaggagaag ca 22

<210> 487
<211> 20
<212> DNA
<213> TRITICUM TAUSCHII

<400> 487
gcgctatgct tcatgacatt 20

<210> 488
<211> 20
<212> DNA
<213> TRITICUM TAUSCHII

<400> 488
atcactgcac cgacttttgg 20

<210> 489
<211> 20
<212> DNA
<213> TRITICUM TAUSCHII

<400> 489
gatacgagcg agcccataaa 20

<210> 490
<211> 20
<212> DNA
<213> TRITICUM TAUSCHII

<400> 490
gagaggagag cttgccattg 20

<210> 491
<211> 20
<212> DNA
<213> TRITICUM TAUSCHII

<400> 491
cagaccttaa cggggttgaa 20

<210> 492
<211> 20
<212> DNA
<213> TRITICUM TAUSCHII

<400> 492
aaagcatctt tgcttctcgc 20

<210> 493
<211> 20
<212> DNA
<213> TRITICUM TAUSCHII

<400> 493
tttacgcagg atacggctct 20

<210> 494
<211> 20
<212> DNA
<213> TRITICUM TAUSCHII

<400> 494

ccctccatgg attcttgcta 20

<210> 495
<211> 20
<212> DNA
<213> TRITICUM TAUSCHII

<400> 495
aagcttcagt gcctttggaa 20

<210> 496
<211> 20
<212> DNA
<213> TRITICUM TAUSCHII

<400> 496
ctctctgtcg tccaggtcgt 20

<210> 497
<211> 20
<212> DNA
<213> TRITICUM TAUSCHII

<400> 497
ctcctcactc ctcgtcttgg 20

<210> 498
<211> 20
<212> DNA
<213> TRITICUM TAUSCHII

<400> 498
gtcgacgtct gcacattgtt 20

<210> 499
<211> 20
<212> DNA
<213> TRITICUM TAUSCHII

<400> 499
agttttgcca tcggctgtat 20

<210> 500
<211> 20
<212> DNA
<213> TRITICUM TAUSCHII

<400> 500
gggagctttc cctagtgctt 20

<210> 501
<211> 20
<212> DNA
<213> TRITICUM TAUSCHII

<400> 501
ctcaatcctg atgcggagat 20

<210> 502
<211> 20
<212> DNA
<213> TRITICUM TAUSCHII

<400> 502
ttactgcact gcgagttgct 20

<210> 503
<211> 20
<212> DNA
<213> TRITICUM TAUSCHII

<400> 503
atcgcgacta ttcaacgctt 20

<210> 504
<211> 20
<212> DNA
<213> TRITICUM TAUSCHII

<400> 504

cagtgtgcct tctagccctc 20

<210> 505
<211> 20
<212> DNA
<213> TRITICUM TAUSCHII

<400> 505
ttcctgatcc caccaaacat 20

<210> 506
<211> 20
<212> DNA
<213> TRITICUM TAUSCHII

<400> 506
gaggcaggaa cttaggggag 20

<210> 507
<211> 19
<212> DNA
<213> TRITICUM TAUSCHII

<400> 507
cgtgtcgatg gtctccttg 19

<210> 508
<211> 20
<212> DNA
<213> TRITICUM TAUSCHII

<400> 508
gaagagagga ggagtggcct 20

<210> 509
<211> 20
<212> DNA
<213> TRITICUM TAUSCHII

<400> 509
atagtaggcc caaagcgatg 20

<210> 510
<211> 21
<212> DNA
<213> TRITICUM TAUSCHII

<400> 510
ttactggcaa gccagaactg t 21

<210> 511
<211> 20
<212> DNA
<213> TRITICUM TAUSCHII

<400> 511
acctgtccag ctagcctcca 20

<210> 512
<211> 21
<212> DNA
<213> TRITICUM TAUSCHII

<400> 512
tctctccaaa cctccctgta a 21

<210> 513
<211> 20
<212> DNA
<213> TRITICUM TAUSCHII

<400> 513
aaacgaagac gtggacatgg 20

<210> 514
<211> 21
<212> DNA
<213> TRITICUM TAUSCHII

<400> 514

tcaagaccat cagcactcag a 21

<210> 515
<211> 20
<212> DNA
<213> TRITICUM TAUSCHII

<400> 515
agtcgccgtt gtatagtgcc 20

<210> 516
<211> 20
<212> DNA
<213> TRITICUM TAUSCHII

<400> 516
tgaaacaggg gaatcagagg 20

<210> 517
<211> 20
<212> DNA
<213> TRITICUM TAUSCHII

<400> 517
ttctgcacaa accgttctga 20

<210> 518
<211> 20
<212> DNA
<213> TRITICUM TAUSCHII

<400> 518
cccgtcgggt tttatctagc 20

<210> 519
<211> 21
<212> DNA
<213> TRITICUM TAUSCHII

<400> 519
gcttgtgaac taatgcctcc c 21

<210> 520
<211> 20
<212> DNA
<213> TRITICUM TAUSCHII

<400> 520
accggccatc tatgatgaag 20

<210> 521
<211> 20
<212> DNA
<213> TRITICUM TAUSCHII

<400> 521
atcgctcact cactatcggg 20

<210> 522
<211> 20
<212> DNA
<213> TRITICUM TAUSCHII

<400> 522
aagacactcg atgcggagag 20

<210> 523
<211> 21
<212> DNA
<213> TRITICUM TAUSCHII

<400> 523
ggataatgtg catgttcacc g 21

<210> 524
<211> 20
<212> DNA
<213> TRITICUM TAUSCHII

<400> 524

gcctccacaa cagccataat 20

<210> 525
<211> 20
<212> DNA
<213> TRITICUM TAUSCHII

<400> 525
atcgtgcacc aagcaataca 20

<210> 526
<211> 20
<212> DNA
<213> TRITICUM TAUSCHII

<400> 526
aaggcagctc aatcggagta 20

<210> 527
<211> 20
<212> DNA
<213> TRITICUM TAUSCHII

<400> 527
ccaatcctaa tgatccgctg 20

<210> 528
<211> 20
<212> DNA
<213> TRITICUM TAUSCHII

<400> 528
ttgtgtggcg aaagaaacag 20

<210> 529
<211> 20
<212> DNA
<213> TRITICUM TAUSCHII

<400> 529
tggtagctta caaaggtgcg 20

<210> 530
<211> 24
<212> DNA
<213> TRITICUM TAUSCHII

<400> 530
catcattgtg tttacgttct ttca 24

<210> 531
<211> 20
<212> DNA
<213> TRITICUM TAUSCHII

<400> 531
tcaaggtgcc aacacttacg 20

<210> 532
<211> 20
<212> DNA
<213> TRITICUM TAUSCHII

<400> 532
atgtcattca tgttgcccct 20

<210> 533
<211> 20
<212> DNA
<213> TRITICUM TAUSCHII

<400> 533
ttcccaggac atccttcttg 20

<210> 534
<211> 20
<212> DNA
<213> TRITICUM TAUSCHII

<400> 534

ggtctttgca ctgctagcct 20

<210> 535
<211> 20
<212> DNA
<213> TRITICUM TAUSCHII

<400> 535
tccgcagcag ttctatgttg 20

<210> 536
<211> 22
<212> DNA
<213> TRITICUM TAUSCHII

<400> 536
ttccttctcc ttcattagct gc 22

<210> 537
<211> 20
<212> DNA
<213> TRITICUM TAUSCHII

<400> 537
tttggtcgac aagcaaatca 20

<210> 538
<211> 20
<212> DNA
<213> TRITICUM TAUSCHII

<400> 538
gcatgttacg tcgctgttgt 20

<210> 539
<211> 22
<212> DNA
<213> TRITICUM TAUSCHII

<400> 539
aaaaggaaac taaagcgatg ga 22

<210> 540
<211> 20
<212> DNA
<213> TRITICUM TAUSCHII

<400> 540
ccggaatttc actaccagga 20

<210> 541
<211> 20
<212> DNA
<213> TRITICUM TAUSCHII

<400> 541
tcctcagaac cccattcttg 20

<210> 542
<211> 20
<212> DNA
<213> TRITICUM TAUSCHII

<400> 542
ccaccagaac ttcaacctgg 20

<210> 543
<211> 20
<212> DNA
<213> TRITICUM TAUSCHII

<400> 543
gacaaggcca gtccaaaaga 20

<210> 544
<211> 20
<212> DNA
<213> TRITICUM TAUSCHII

<400> 544

cagtagcatc ttccatggcg 20

<210> 545
<211> 23
<212> DNA
<213> TRITICUM TAUSCHII

<400> 545
tcggagagta ttagaacagt gcc 23

<210> 546
<211> 20
<212> DNA
<213> TRITICUM TAUSCHII

<400> 546
tgccgcactt atttgttcac 20

<210> 547
<211> 20
<212> DNA
<213> TRITICUM TAUSCHII

<400> 547
gtgatgctgt tctcaagcca 20

<210> 548
<211> 20
<212> DNA
<213> TRITICUM TAUSCHII

<400> 548
cactcaatgg caggtccttt 20

<210> 549
<211> 25
<212> DNA
<213> TRITICUM TAUSCHII

<400> 549
ggtaaagtta taaattgttg tgggc 25

<210> 550
<211> 25
<212> DNA
<213> TRITICUM TAUSCHII

<400> 550
ccattatgta aatgcttctg tttga 25

<210> 551
<211> 20
<212> DNA
<213> TRITICUM TAUSCHII

<400> 551
taccagctgc acttccattg 20

<210> 552
<211> 22
<212> DNA
<213> TRITICUM TAUSCHII

<400> 552
ctcccaggag tacagaagag ga 22

<210> 553
<211> 20
<212> DNA
<213> TRITICUM TAUSCHII

<400> 553
aagtcggcca tcttcttcct 20

<210> 554
<211> 20
<212> DNA
<213> TRITICUM TAUSCHII

<400> 554

```
gcctatgctg ctgatccatt                                        20

<210> 555
<211> 20
<212> DNA
<213> TRITICUM TAUSCHII

<400> 555
cgcccttgtt tgtattggtt                                        20
```

## Description

The present invention relates to a map of the D genome of wheat bread, microsatellite markers and primers for detecting said markers and uses thereof for selection, detection of mutations, study of diversity, phylogeny, and evolution and for the identification of cultivars and hybrids of wheat bread species, Triticum species and related species of commercial interest.

Mapping many agronomically important genes and QTL (quantitative trait loci) requires a saturated map. Compared to other grass species, genetic mapping of wheat has been made more difficult because of its low level of polymorphism, the large size of its genome ($16.10^9$ bp) and its polyploid structure. Wheat is an allohexaploid species made of three diploid genomes: A, B and D. It results from two successive intercrossings, which involved at least three different species (Kihara 1950). The D genome has been introduced during the last intercrossing, between the amphiploid AABB and *Triticum tauschii* (DD), involving probably only a limited number of both genotypes of species (Breiman et al. 1995). Genetic mapping on bread wheat started with RFLP markers (Chao et al. 1989, Liu and Tsunewaki 1991, Gale et al. 1995, Cadalen et al. 1997, Gupta et al. 1999), but these show a rather low level of polymorphism. At the end of the 1980's, a new class of molecular markers : microsatellites, were developed (Weber and May 1989; Litt and Lutty 1989; Beckmann and Sollers 1990). The first mapping studies using microsatellites were carried out on animal species (cattle, Bishop et al. 1994; human, Dib et al. 1996) and more recently on plants (rice, Wu et al. 1993; barley, Shanghai-Maroof et al. 1994; wheat, Röder *et al.* 1998). Microsatellites are tandemly repeated sequences between of one to six nucleotides long. They can be amplified using PCR and specific primer pairs designed from the flanking regions of the repeat motif. Microsatellites appear to be very polymorphic in length, due mainly to polymerase slippage during replication (Levinson and Gutman 1987). Weber (1990) divided microsatellites into three categories: perfect repeats, imperfect repeats with one or more interruptions and compound repeats with adjacent tandem simple repeats of different motifs. Microsatellites combine a number of advantages. They are codominant and show Mendelian inheritance (Lagercrantz et al. 1993 ; Morgante et al. 1993). The generally high level of polymorphism of microsatellites makes them suitable for the genetic mapping of species which show little intraspecific polymorphism, such as most inbreeding species. In addition, microsatellites have good potential of automation and require only a small amount of DNA.

In wheat, the D genome is known to be less polymorphic than the A and B genomes (Nelson et al. 1995a,b, c ; Cadalen et al. 1997). Thus, genetic maps of the D genome are very difficult to elaborate especially within a narrow genetic base. Despite the fact that microsatellites have been shown to be twice as polymorphic as RFLP markers (Röder, 1995), only 25% of the wheat microsatellite locus developed by Röder et al. (1995) mapped to the D genome while 30% and 45 % were mapped to the A and B genome respectively. Previous results obtained in our laboratory (Tavaud 1998) have shown that microsatellites, which mapped onto the D genome of bread wheat, also amplified the D genome ancestral diploid donor: *T.tauschii*. To enhance the probability of developing microsatellites which map onto the D genome, we explore the possibility of using a library enriched for various microsatellite motifs, constructed from *T. tauschii* genomic DNA.

In connection with the present invention, we show that microsatellites developed from *T.tauschii* can be transferred to hexaploid wheat. We further show that most of these microsatellites map to the D genome of bread wheat. Therefore, despite the above mentioned problems, we provide here with a map the D genome of wheat bread, microsatellite markers and primers for detecting said markers.

We have analysed our enrichment results in terms of motif and number of repeats and the influence which motif length and type has on the ability to detect polymorphisms. Furthermore, primers generated from the flanking region of the microsatellites were used successfully to amplify the D genome of bread wheat. Additional amplification sometimes also occurred from the A and B genomes. A total of 185 markers were developed. The majority of these contained $(GA)_n$ and $(GT)_n$ motifs. GA and GT repeats appeared to be both more abundant in our library and more polymorphic than other type of repeats. The distribution of both types of dinucleotides repeats fitted a Poisson's distribution. Deviance analysis showed that GA and GT were more polymorphic than other motifs in bread wheat. Within each motif type (di-, tri-, tetranucleotide repeats), stretch length has no influence on polymorphism. The microsatellites map along the D chromosomes in the *T.aestivum* Courtot x Chinese Spring map.

For polyploid species, isolation of microsatellites from an ancestral diploid donor seems to be an efficient way of developing markers from a particular elementary genome.

## DESCRIPTION

Thus, in a first embodiment, the present invention is aimed at map of the wheat D genome comprising the genome location of a microsatellite marker selected from the group consisting of sequences SEQ ID No 1 to 185. For example, this map comprises the genome location of at least 25, 50, 100, 150 or preferably 185 microsatellite markers selected from the sequences SEQ ID No 1 to 185. A preferred map according to the invention comprises the genome location of at least 10, 20, 30, 40 or preferably 45 microsatellite markers as depicted in figure 1 selected from the microsatellite markers of sequences SEQ ID No 3, 5, 7, 8, 9, 11, 12, 14, 17, 18, 19, 21, 23, 26, 27, 29, 31, 32, 34, 35, 36, 37, 38, 40, 41, 43, 44, 45, 46, 47, 53, 55, 56, 57, 58, 60, 62, 63, 64, 66, 68, 69, 70, 71, 79, 81. Figure 1 shows the wheat D-genome molecular linkage map of Courtot x Chinese Spring population. Kosambi distances are expressed in centimorgans and are written on the left of chromosomes for anchor markers. Distances written on the right of chromosomes are distances between the markers and the anchor marker above. Double bars mean a lack of linkage between two linkage groups with thresholds of LOD3 and 50cM using Mapmaker.

In a second embodiment, the invention is directed to microsatellite markers selected from sequences SEQ ID No 1 to 185. It also relates to the left primer (SEQ ID No 186-370) and right primers (SEQ ID No 371-555) of said microsatellite markers, see Table 1 hereinafter. For each microsatellite marker, Table 1 gives the corresponding left and right primers.

Maps of microsatellite markers selected from sequences SEQ ID No 1 to 185, microsatellite markers selected from sequences SEQ ID No 1 to 185, primers selected from sequences SEQ ID No 186-555 or sequence complementary thereto are useful for identifying genes responsible for a phenotypic trait of interest, notably QTLs in wheat, more particularly for analyzing diseases, for identifying the implicated genes of said diseases and their alleles, for identifying development factors, quality factors and factors conferring resistance to pathogens and xenobiotics.

In a third embodiment, the invention concerns the use of left primers selected from sequences SEQ ID No 186-370 and right primers selected from sequences SEQ ID No 371-555 or sequence complementary thereto to amplify and detect microsatellite markers selected from the sequences SEQ ID No 1 to 185 respectively (see Table 1 below). These primers are also useful for assessing the interspecific or intraspecific polymorphism of microsatellite microsatellite markers selected from the sequences SEQ ID No 1 to 185 respectively.

Table 1 gives the correspondence between each microsatellite marker and its respective left and right primers:

| Sequence | Micro-satellite SEQ ID N° | Labo. code | Inter code | Left primer | SEQ ID N° | Right primer | SEQ ID N° | Tm | Motif | Size | notes | Location |
|---|---|---|---|---|---|---|---|---|---|---|---|---|
| XBG0AD001ZF09F1 | 1 | CFD01F09 | cfd1 | ACCAAAGAACTTGCCTGGTG | 186 | AAGCCTGACCTAGCCCAAAT | 371 | 60 | (GCC)6 | 222 | perfect | 6A |
| BG0AD002ZB01R1 | 2 | CFD02B01 | cfd2 | GGTTGCAGTTTCCACCTTGT | 187 | CATCTATTGCCAAAATCGCA | 372 | 60 | (CA)21 | 288 | imperfect | 2A/2D/3A/3D/4A/5B/5D |
| BG0AD002ZB02F1 | 3 | CFD02B02 | cfd3 | GCACCAACACACGGAGAAG | 188 | TTGAGAGGAGGGCTTGGTTA | 373 | 60 | (CA)16 | 163 | perfect | 5D |
| BG0AD002ZD01R1 | 4 | CFD02D01 | cfd4 | TGCTCCGTCTCCGAGTAGAT | 189 | GGGAAGGAGAGATGGGAAAC | 374 | 60 | (TC)37 | 262 | perfect | 3B/3D |
| BG0AD003ZG07F1 | 5 | CFD03G07 | cfd5 | TGCCCTGTCCACAGTGAAG | 190 | TTGCCAGTTCCAAGGAGAAT | 375 | 60 | (GT)17 | 195 | perfect | 6D |
| BG0AD003ZG09F1 | 6 | CFD03G09 | cfd6 | ACTCTCCCCCTCGTTGCTAT | 191 | ATTTAAGGGAGACATCGGGC | 376 | 60 | (GA)6(GCTA)4 | 236 | imperfect | 7A |
| BG0AD004ZA06R1 | 7 | CFD04A06 | cfd7 | AGCTACCAGCCTAGCAGCAG | 192 | TCAGACACGTCTCCTGACAAA | 377 | 60 | (TC)27 | 250 | perfect | 5B/5D |
| BG0AD004ZD06R1 | 8 | CFD04D06 | cfd8 | ACCACCGTCATGTCACTGAG | 193 | GTGAAGACGACAAGACGCAA | 378 | 60 | (CT)19(TCCT)4 | 162 | imperfect | 5D |
| BG0AD004ZD08R1 | 9 | CFD04D08 | cfd9 | TTGCACGCACCTAAACTCTG | 194 | CAAGTGTGAGCGTCGG | 379 | 60 | (TC)29 | 209 | perfect | 3D |
| BG0AD004ZD11F1 | 10 | CFD04D11 | cfd10 | CGTTCTATGACGTGTCATGCT | 195 | TCCATTTTCAAAAACACCCTG | 380 | 60 | (CA)16 | 288 | perfect | 5D |
| BG0AD004ZE03F1 | 11 | CFD04E03 | cfd11 | GGATTTGCCTTGGATTTGAA | 196 | TTGCATCTCACGCACCTAAG | 381 | 60 | (CA)31 | 167 | imperfect | 2D/2B |
| BG0AD004ZF06F1 | 12 | CFD04F06 | cfd12 | GTTACCCAAACCTGCCCTTT | 197 | CTACGAGTCGGGATCAGCAT | 382 | 60 | (CTT)6 | 192 | imperfect | 5D |
| BG0AD004ZG01R1 | 13 | CFD04G01 | cfd13 | CCACTAACCAAGCTGCCATT | 198 | TTTTTGGCATTGATCTGCTG | 383 | 60 | (CT)20(TGTA)3 | 254 | imperfect | 6B/6D |
| BG0AD004ZG03F1 | 14 | CFD04G03 | cfd14 | CCACCGGCCAGAGTAGTATT | 199 | TCCTGGTCTAACAACGAGAAGA | 384 | 60 | (TC)28 | 152 | perfect | 7D |
| BG0AD005ZB06F1 | 15 | CFD05B06 | cfd15 | CTCCCGTATTGAGCAGGAAG | 200 | GGCAGGTGTGGTGATGATCT | 385 | 60 | (CT)20(TGTA)3 | 179 | perfect | 1A/1D |
| BG0AD005ZB10F1 | 16 | CFD05B10 | cfd16 | GGATCCAAGGGAATCCAAAT | 201 | TCCTTCGGTTCCCATATCAC | 386 | 60 | (GGC)5 | 241 | perfect | 4A |
| BG0AD005ZC10F1 | 17 | CFD05C10 | cfd17 | AGCACAGAAGGGGTTAGGGT | 202 | AGCTGCGGTGTGAGCTAAAT | 387 | 60 | (CA)18 | 205 | perfect | 2D |
| BG0AD005ZC11F1 | 18 | CFD05C11 | cfd18 | CATCCAACAGCACCAAGAGA | 203 | GCTACTACTATTTCATTGCGACCA | 388 | 60 | (GA)25 | 169 | imperfect | 5D |
| BG0AD005ZE07F1 | 19 | CFD05E07 | cfd19 | TACGCAGGTTTGCTGCTTCT | 204 | GGAGTTCACAAGCATGGGTT | 389 | 60 | (GA)18 | 267 | perfect | 1D/5D/6D |
| BG0AD005ZE09F1 | 20 | CFD05E09 | cfd20 | TGATGGGAAGGTAATGGGAG | 205 | ATCCAGTTCTCGTCCAAAGC | 390 | 60 | (GGAA)3(CTAC)3 | 296 | imperfect | 1B |
| BG0AD006ZE12F1 | 21 | CFD06E12 | cfd21 | CCTCCATGTAGGCGGAAATA | 206 | TGTGTCCCATTCACTAACCG | 391 | 60 | (GA)21 | 281 | perfect | 1D/7D |
| BG0AD006ZF04R1 | 22 | CFD06F04 | cfd22 | GGTTGCAAACCGTCTTGTTT | 207 | AGTCGAGTTGCGACCAAAGT | 392 | 60 | (GA)28 | 254 | imperfect | 4B |
| BG0AD006ZG09R1 | 23 | CFD06G09 | cfd23 | TAGCAGTAGCAGCAGCAGGA | 208 | GCAAGGAAGAGTGTTCAGCC | 393 | 60 | (AGT)23(GCA)3(GTA)8 | 187 | imperfect | 4D |
| BG0AD007ZA01F1 | 24 | CFD07A01 | cfd24 | GGCGCAATCTGAAAGAAAAG | 209 | CCAGGTCCCACTTTCGTCT | 394 | 60 | (A)n(T)n | 273 | perfect | 4A |
| BG0AD007ZC01F1 | 25 | CFD07C01 | cfd25 | CATCGCTCATGCTAAGGTCA | 210 | CGTGTCTGTTAGCTGGGTGG | 395 | 60 | (GA)35 | 203 | perfect | 2B/5D/7D |
| BG0AD008ZB09R1 | 26 | CFD08B09 | cfd26 | TCAAGATCGTGCCAAATCAA | 211 | ACTCCAAGCTGAGCACGTTT | 396 | 60 | (GAGAA)2(GA)37 | 271 | perfect | 5D |
| BG0AD008ZF07F1 | 27 | CFD08F07 | cfd27 | GCAGCAAGATCAAATCGACA | 212 | ACTGAGGACTTGGTGCCATC | 397 | 60 | (CA)20 | 161 | perfect | 1D |
| BG0AD008ZH09F1 | 28 | CFD08H09 | cfd28 | TGCATCTTATTACTGGAGGCA | 213 | CGCATGCCCTTATACCAACT | 398 | 60 | (CAA)22 | 190 | perfect | 1D |

| | | | | | | | | | | | | |
|---|---|---|---|---|---|---|---|---|---|---|---|---|
| | | | | TT | | | | | | | | |
| BG0AD009ZB08F1 | 29 | CFD09B08 | cfd29 | GGTTGTCAGGCAGGATATTTG | 214 | TATTGATAGATCAGGGCGCA | 399 | 60 | (TC)18 | 153 | perfect | 5D |
| BG0AD009ZC02R1 | 30 | CFD09C02 | cfd30 | AATCGCACAACAATGGTTCA | 215 | GCCTCTCCTCTCTGCTCCTT | 400 | 60 | (GA)33(GCA)4 | 236 | imperfect | 6A |
| BG0AD009ZD07F1 | 31 | CFD09D07 | cfd31 | GCACCAACCTTGATAGGGAA | 216 | GTGCCTGATGATTTTACCCG | 401 | 60 | (GA)51 | 276 | perfect | 4A/7D |
| BG0AD009ZE01R1 | 32 | CFD09E01 | cfd32 | CAACACAACCACAATTTCCG | 217 | CTCAGGGAGGTCATGCAGAG | 402 | 60 | (CA)18 | 176 | perfect | 1D |
| BG0AD009ZE03F1 | 33 | CFD09E03 | cfd33 | TACCGCAATAATCACACCCA | 218 | GGTCGATGGACTGTCCCTAA | 403 | 60 | (CA)18 | 170 | imperfect | 6D |
| BG0AD009ZF12F1 | 34 | CFD09F12 | cfd34 | GGAAGAACCGCAACAGACAT | 219 | GCATCTTCTCCTCCCTCCTC | 404 | 60 | (GGA)5(G)13 | 203 | imperfect | 3D |
| BG0AD009ZG04R1 | 35 | CFD09G04 | cfd35 | GGGATGACACATAACGGACA | 220 | ATCAGCGGCGCTATAGTACG | 405 | 60 | (GT)13(CT)20 | 191 | perfect | 3D |
| BG0AD009ZH12F1 | 36 | CFD09H12 | cfd36 | GCAAAGTGTAGCCGAGGAAG | 221 | TTAGAGTTTTGCAGCGCCTT | 406 | 60 | (CA)25(CCCG)3 | 192 | perfect | 2A/2D |
| BG0AD010ZA11R1 | 37 | CFD10A11 | cfd37 | GCTTCTTTTGCTGCTTTTGC | 222 | CCCCCACATACAGAGGCTAA | 407 | 60 | (CT)22 | 218 | perfect | 6D |
| BG0AD010ZB03R1 | 38 | CFD10B03 | cfd38 | TGGCCATTCGATATTCAAAA | 223 | GTGAGTTGAGGCGCATGATA | 408 | 60 | (GA)32 | 215 | perfect | 6D |
| BG0AD010ZC09F1 | 39 | CFD10C09 | cfd39 | CCACAGCTACATCATCTTTCCT T | 224 | CAAAGTTTGAACAGCAGCCA | 409 | 60 | (GA)26 | 175 | perfect | 4B/4D/5A |
| BG0AD010ZF09R1 | 40 | CFD10F09 | cfd40 | GCGACAAGTAATTCAGAACGG | 225 | CGCTTCGGTAAAGTTTTTGC | 410 | 60 | (TAA)5 | 223 | perfect | 5D |
| BG0AD011ZC11R1 | 41 | CFD11C11 | cfd41 | TAAAGTCTCAGGCGACCCAC | 226 | AGTGATAGACGGATGGCACC | 411 | 60 | (CA)17 | 286 | perfect | 7D |
| BG0AD011ZF05F1 | 42 | CFD11F05 | cfd42 | AGGTTCTAGGGGGCATGTCT | 227 | GCTCTCAATGACTGCACTGG | 412 | 60 | (GA)24 | 177 | perfect | 6D |
| BG0AD012ZA06F1 | 43 | CFD12A06 | cfd43 | AACAAAAGTCGGTGCAGTCC | 228 | CCAAAAACATGGTTAAAGGGG | 413 | 60 | (CA)20 | 150 | perfect | 2D |
| BG0AD012ZB08R1 | 44 | CFD12B08 | cfd44 | AAACCCAATGGCTCTCACAC | 229 | ATGGCCCAATTATGCAACTC | 414 | 60 | (CT)24 | 238 | perfect | 2D |
| BG0AD012ZC01R1 | 45 | CFD12C01 | cfd45 | TCTCTCCAGTTGCTCCTCGT | 230 | ATGTGGAACCGGTCTACTCG | 415 | 60 | (GCC)7 | 187 | perfect | 6D |
| BG0AD012ZC08F1 | 46 | CFD12C08 | cfd46 | TGGTGGTATAGTCGTTGGAGC | 231 | CCACACACACACACCATCAA | 416 | 60 | (GT)29 | 175 | perfect | 7D |
| BG0AD012ZF08F1 | 47 | CFD12F08 | cfd47 | TGACCATGTCATGTTTTATACC ACT | 232 | TGGACTACATGTCAAGCACAA A | 417 | 60 | (GT)25 | 184 | perfect | 6D |
| BG0AD012ZF11F1 | 48 | CFD12F11 | cfd48 | ATGGTTGATGGTGGGTGTTT | 233 | ATGTATCGATGAAGGGCCAA | 418 | 60 | (GT)22 | 250 | imperfect | 1B |
| BG0AD013ZA04R1 | 49 | CFD13A04 | cfd49 | TGAGTTCTTCTGGTGAGGCA | 234 | GAATCGGTTCACAAGGGAAA | 419 | 60 | (GA)33 | 214 | imperfect | 6D |
| BG0AD013ZD02F1 | 50 | CFD13D02 | cfd50 | TTCTGCAACATTTTGTCCCA | 235 | CGTATGATCCTAACGAGGGC | 420 | 60 | (CA)16 | 261 | perfect | 2A/2B/2D |
| BG0AD013ZD03F1 | 51 | CFD13D03 | cfd51 | GGAGGCTTCTCTATGGGAGG | 236 | TGCATCTTATCCTGTGCAGC | 421 | 60 | (GA)45 | 172 | perfect | 2D |
| BG0AD013ZD10F1 | 52 | CFD13D10 | cfd52 | AGACGGTGTTGGTTCCTGAG | 237 | TAGTGGCCGGTGGAATTAAA | 422 | 60 | ? | 275 | imperfect | 5D |
| BG0AD013ZF06F1 | 53 | CFD13F06 | cfd53 | CCCTATTTCCCCCATGTCTT | 238 | AAGGAGGGCACATATCGTTG | 423 | 60 | (CT)22(CA)10 | 233 | perfect | 2D |
| BG0AD013ZF09F1 | 54 | CFD13F09 | cfd54 | TCGTTCCAAAATGCATGAAA | 239 | AAGGGCCAGAAATCTGTGTG | 424 | 60 | (GA)42 | 200 | imperfect | 4B/4D |
| BG0AD013ZG03F1 | 55 | CFD13G03 | cfd55 | CCAGTAGCCGGCCCTACTAT | 240 | GCACGAGATACGGACAATCA | 425 | 60 | (GT)23 | 263 | perfect | 3D |
| BG0AD014ZC06F1 | 56 | CFD14C06 | cfd56 | TTGCATAATTACTTGCCCTCC | 241 | CTGGTCCAACTTCCATCCAT | 426 | 60 | (GA)31 | 221 | perfect | 2D |
| BG0AD015ZC07F1 | 57 | CFD15C07 | cfd57 | ATCGCCGTTAACATAGGCAG | 242 | TCACTGCTGTATTTGCTCCG | 427 | 60 | (GA)29 | 291 | perfect | 5D |
| BG0AD015ZD11R1 | 58 | CFD15D11 | cfd58 | AATGGGCCTTTAAGAGCAAAA | 243 | AGGGGTGAAAGGTTGGAGAC | 428 | 60 | (CT)5(CA)10 | 179 | perfect | 1D |
| BG0AD015ZG11R1 | 59 | CFD15G11 | cfd59 | TCACCTGGAAAATGGTCACA | 244 | AAGAAGGCTAGGGTTCAGGC | 429 | 60 | (GC)6(GA)23 | 296 | perfect | 1B/1D/4B/6 B |

| | | | | | | | | | | | | |
|---|---|---|---|---|---|---|---|---|---|---|---|---|
| BG0AD015ZH01R1 | 60 | CFD15H01 | cfd60 | TGACCGGCATTCAGTATCAA | 245 | TGGTCACTTTGATGAGCAGG | 430 | 60 | (CA)25 | 218 | perfect | 6D |
| BG0AD015ZH05R1 | 61 | CFD15H05 | cfd61 | ATTCAAATGCAACGCAAACA | 246 | GTTAGCCAAGGACCCCTTTC | 431 | 60 | (CACAA)3(AC)22 | 218 | imperfect | 1D |
| BG0AD016ZC09F1 | 62 | CFD16C09 | cfd62 | CAAGAGCTGACCAATGTGGA | 247 | ACGGCGGTGAGATGAG | 432 | 60 | (CT)27 | 220 | perfect | 2D/7A |
| BG0AD016ZF12R1 | 63 | CFD16F12 | cfd63 | TCCTGAGGATGTTGAGGACC | 248 | GAGAGAGGCGAAACATGGAC | 433 | 60 | (CA)26 | 275 | perfect | 1D |
| BG0AD016ZG07R1 | 64 | CFD16G07 | cfd64 | ACAGTGTTGTTGCCCCTTTC | 249 | CCCATGTTACAGCTTTGGGT | 434 | 60 | (CT)9(CA)22 | 263 | perfect | 3D |
| BG0AD016ZG10F1 | 65 | CFD16G10 | cfd65 | AGACGATGAGAAGGAAGCCA | 250 | CCTCCCTTGTTTTTGGGATT | 435 | 60 | (CT)32 | 199 | imperfect | 1B/1D |
| BG0AD017ZA01R1 | 66 | CFD17A01 | cfd66 | AGGTCTTGGTGGTTTTGGTG | 251 | TTTTCACATGCCCACAGTTG | 436 | 60 | (GC)9(AG)60 | 202 | perfect | 7D |
| BG0AD017ZD08F1 | 67 | CFD17D08 | cfd67 | GCGGACAAATTGAGCCTTAG | 252 | TGTGCGTGTGTGTGTGTTTT | 437 | 60 | (CA)21 | 188 | imperfect | 5D |
| BG0AD017ZE03F1 | 68 | CFD17E03 | cfd68 | TTTGCAGCATCACACGTTTT | 253 | AAAATTGTATCCCCCGTGGT | 438 | 60 | (GT)16 | 204 | imperfect | 7D |
| BG0AD017ZG09R1 | 69 | CFD17G09 | cfd69 | AAATACCTTGAATTGTGAGCT GC | 254 | TCTGTTCATCCCCAAAGTCC | 439 | 60 | (CA)46 | 240 | imperfect | 7D |
| BG0AD017ZH01F1 | 70 | CFD17H01 | cfd70 | GTCGGCATAGTCGCACATAC | 255 | ACTATGCCAAGGGGAGTGTG | 440 | 60 | (CA)19 | 172 | perfect | 3D |
| BG0AD017ZH08F1 | 71 | CFD17H08 | cfd71 | CAATAAGTAGGCCGGGACAA | 256 | TGTGCCAGTTGAGTTTGCTC | 441 | 60 | (CA)10(GA)30 | 216 | perfect | 4A/4D |
| BG0AD017ZH10F1 | 72 | CFD17H10 | cfd72 | CTCCTTGGAATCTCACCGAA | 257 | TCCTTGGGAATATGCCTCCT | 442 | 60 | (GA)25 | 223 | perfect | 1D |
| BG0AD018ZA12R1 | 73 | CFD18A12 | cfd73 | GATAGATCAATGTGGGCCGT | 258 | AACTGTTCTGCCATCTGAGC | 443 | 60 | (CT)19 | 242 | perfect | 2B/2D |
| BG0AD018ZB12F1 | 74 | CFD18B12 | cfd74 | TCAAAACCACACCAGGCATA | 259 | AAGTGGTGGGGAGTGTGTGT | 444 | 60 | (ATG)3(CA)15 | 338 | imperfect | 5D/7B |
| BG0AD018ZD03F1 | 75 | CFD18D03 | cfd75 | GCATAAACTTGGACCCTGGA | 260 | GCTAAGCCACGCTACCACTC | 445 | 60 | (TGGA)3(GA)23 | 297 | imperfect | 6D |
| BG0AD018ZF12F1 | 76 | CFD18F12 | cfd76 | GCAATTTCACACGCGACTTA | 261 | CGCTCGACAACATGACACTT | 446 | 60 | (GA)26 | 166 | imperfect | 6D |
| BG0AD018ZG03F1 | 77 | CFD18G03 | cfd77 | CTGCTTCAGGGATTGGAGAG | 262 | GTTTCCTGGGCTAAACCACA | 447 | 60 | (GGC)6 | 209 | perfect | 2D |
| BG0AD019ZB10R1 | 78 | CFD19B10 | cfd78 | ATGAAATCCTTGCCCTCAGA | 263 | TGAGATCATCGCCAATCAGA | 448 | 60 | (GA)19 | 182 | perfect | 5D |
| BG0AD019ZC05F1 | 79 | CFD19C05 | cfd79 | TCTGGTTCTTGGGAGGAAGA | 264 | CATCCAACAATTTGCCCAT | 449 | 60 | (GA)26 | 284 | perfect | 3D/3B |
| BG0AD019ZC11F1 | 80 | CFD19C11 | cfd80 | ATAGGGGTTTTGAATCACTCC | 265 | TTGGATTTGCAGAGCCTTCT | 450 | 60 | (GA)27 | 179 | perfect | 6D |
| BG0AD019ZE05F1 | 81 | CFD19E05 | cfd81 | TATCCCCAATCCCCTCTTTC | 266 | GTCAATTGTGGCTTGTCCCT | 451 | 60 | (GT)14(GA)19 | 283 | perfect | 4D/5D/7B |
| BG0AD020ZB02F1 | 82 | CFD20B02 | cfd82 | GCTGATGCTGCTGTAAGTGC | 267 | TGAAGAATACAATGGCAGCAA | 452 | 60 | (TGC)5 | 242 | perfect | 6A |
| BG0AD020ZC09F1 | 83 | CFD20C09 | cfd83 | AAGGATGGAGAGGACCCCTA | 268 | GGAGGTGGAGCAACCTATCA | 453 | 60 | (TG)14 | 233 | perfect | 1D |
| BG0AD020ZG07F1 | 84 | CFD20G07 | cfd84 | GTTGCCTCGGTGTCGTTTAT | 269 | TCCTCGAGGTCCAAAACATC | 454 | 60 | (GA)26 | 193 | perfect | 4D |
| XBG0AD001ZA11R 1 | 85 | CFD01A11 | cfd86 | TTAATGAGCGTCAGTACTCCC | 270 | GCAACCATGTTTAAGCCGAT | 455 | 60 | GT | 274 | perfect | 5B/5D |
| XBG0AD001ZE09F 1 | 86 | CFD01E09 | cfd88 | TAGGCATAGTTTTGGGCCTG | 271 | GGTAGAAGGAAGCTTCGGGA | 456 | 60 | (CCG)5 | 238 | perfect | 4A |
| XBG0AD001ZG02F 1 | 87 | CFD01G02 | cfd89 | TTAGCAGATAAGAGCTAAGCT ATGG | 272 | GCAGTCTTTTTCCTGCGTTC | 457 | 60 | (GAAA)3 | 155 | perfect | 4D |
| BG0AD002ZB12R1 | 88 | CFD02B12 | cfd92 | CTTGTTGATCTCCTTCCCCA | 273 | TTCTCTCATGACGGCAACAC | 458 | 60 | (CT)21(A)19 | 253 | imperfect | 1D |
| BG0AD002ZC11F1 | 89 | CFD02C11 | cfd95 | AATCCTGACTTTAAAGCCTTTC C | 274 | CATCTGTATGATATTTTGGAG GTCA | 459 | 60 | (GT)18 | 150 | perfect | 6D |
| BG0AD002ZF06F1 | 90 | CFD02F06 | cfd10 1 | TCGACCGAAGAGAGCAGAAT | 275 | AGAATGCGTCCTTTTCTTGC | 460 | 60 | (CGG)5 | 299 | perfect | 5D |

| | | | | | | | | | | | | |
|---|---|---|---|---|---|---|---|---|---|---|---|---|
| BG0AD002ZG03R1 | 91 | CFD02G03 | cfd102 | TTGTGGAAGGGTTTGATGAAG | 276 | TGCAGGACCAAACATAGCTG | 461 | 60 | (CT)12(CA)16(CT)27 | 299 | perfect | 5D |
| BG0AD003ZA03R1 | 92 | CFD03A03 | cfd106 | ACGGGTGGTTTTGCTCAGT | 277 | ACTCCACCAGCGGAGAAATA | 462 | 60 | (GCC)5(TTG)3 | 187 | imperfect | 4D |
| BG0AD003ZG02F1 | 93 | CFD03G02 | cfd116 | TTTGCCCATTACAACAAGCA | 278 | CAAGCAGCACCTCATGACAG | 463 | 60 | (GA)15 | 225 | imperfect | 2D |
| BG0AD004ZC10F1 | 94 | CFD04C10 | cfd127 | TAAACACCAGGGAGGTCCAC | 279 | ACCTACGATCGACGAAATGG | 464 | 60 | (GCC)6 | 150 | imperfect | 3D |
| BG0AD004ZE06F1 | 95 | CFD04E06 | cfd132 | CAAATGCTAATCCCCGCC | 280 | TGTAAACAAGGTCGCAGGTG | 465 | 60 | (CT)6(CA)19 | 150 | perfect | 6D |
| BG0AD004ZF07F1 | 96 | CFD04F07 | cfd135 | GGATCTCGGGGATGTCCT | 281 | TAAGCACCTTCTTCATGGGG | 466 | 60 | (GA)35 | 213 | imperfect | 6D |
| BG0AD005ZA05F1 | 97 | CFD05A05 | cfd141 | CGTAAAGATCCGAGAGGGTG | 282 | TCCGAGGTGCTACCTACCAG | 467 | 60 | (TCAA)4 | 155 | perfect | 3D |
| BG0AD005ZB03F1 | 98 | CFD05B03 | cfd143 | TTCTCCATGGGCAGCTACTT | 283 | ACTACTTGCGGACGGCTG | 468 | 60 | (CCG)7 | 262 | imperfect | 3B/6D |
| BG0AD006ZA11F1 | 99 | CFD06A11 | cfd152 | TGGAAGTCTGGAACCACTCC | 284 | GCAACCAGACCACACTCTCA | 469 | 60 | (TC)24 | 288 | perfect | 3D |
| BG0AD006ZD09F1 | 100 | CFD06D09 | cfd156 | AGCAGTGTAATAAAAGGGCG | 285 | GTATTCGCACCAGAATCCGT | 470 | 60 | (CACGCA)3 | 300 | perfect | 5B/5D |
| BG0AD006ZH01R1 | 101 | CFD06H01 | cfd160 | CCACTACTGCGGCTAGGTCT | 286 | CTTTTCCGTGTCTCCCTAGC | 471 | 60 | (CCCT)6 | 200 | perfect | 2D |
| BG0AD007ZA02F1 | 102 | CFD07A02 | cfd161 | GTAAGGCATCTTCGCGTCTC | 287 | CCATGATAGATTTGGACGGG | 472 | 60 | (G)n(A)n | 179 | perfect | 2D |
| BG0AD007ZC11F1 | 103 | CFD07C11 | cfd165 | TTTCCTTGGATCCACTCACC | 288 | GAAACAACCCAGGGACAAGA | 473 | 60 | (GT)24 | 250 | perfect | 5D |
| BG0AD007ZG03R1 | 104 | CFD07G03 | cfd168 | CTTCGCAAATCGAGGATGAT | 289 | TTCACGCCCAGTATTAAGGC | 474 | 60 | (CTG)20 | 197 | imperfect | 2D |
| BG0AD007ZG12F1 | 105 | CFD07G12 | cfd170 | CTGTCGGACGACGACGA | 290 | CATCCTCTTGACGCCGCCGC | 475 | 60 | (GGC)4 | 268 | imperfect | 1B |
| BG0AD008ZD04F1 | 106 | CFD08D04 | cfd175 | TGTCGGGGACACTCTCTCTT | 291 | ACCAATGGGATGCTTCTTTG | 476 | 60 | (CT)28 | 281 | imperfect | 2D |
| BG0AD009ZA07R1 | 107 | CFD09A07 | cfd183 | ACTTGCACTTGCTATACTTACGAA | 292 | GTGTGTCGGTGTGTGGAAAG | 477 | 60 | (CA)22 | 179 | perfect | 5D |
| BG0AD009ZF02R1 | 108 | CFD09F02 | cfd188 | AATGGCTTCACTGTTTGCCT | 293 | AAATGGTCCCAGCATTCAAG | 478 | 60 | (CA)28 | 248 | perfect | 6D |
| BG0AD009ZH08R1 | 109 | CFD09H08 | cfd189 | GCTAAAGCCACATAGGACGG | 294 | GCACAAGATTTTGCAAGGCT | 479 | 60 | (GT)28 | 280 | perfect | 5D |
| BG0AD010ZC07F1 | 110 | CFD10C07 | cfd190 | CAATCAGAAGCGCCATTGTT | 295 | CCCTGATGTTTTCTTTTTCTCC | 480 | 60 | (CA)20 | 191 | imperfect | 6A |
| BG0AD010ZG08R1 | 111 | CFD10G08 | cfd193 | GCTGCCGCTACTGTCTGTC | 296 | GGCACACTCACACACCACAC | 481 | 60 | (GT)16 | 199 | perfect | 4D |
| BG0AD011ZE06F1 | 112 | CFD11E06 | cfd201 | ACAAGACCACACCTCCAAGG | 297 | CGGTTTGGGTTTTGTGATCT | 482 | 60 | (GCC)6 | 218 | perfect | 3D |
| BG0AD012ZD08F1 | 113 | CFD12D08 | cfd211 | AGAAGACTGCACGCAAGGAT | 298 | TGCACTAAAGCATCTTCGTGTT | 483 | 60 | (GA)17 | 283 | perfect | 3D |

| | | | | | | | | | | | | | |
|---|---|---|---|---|---|---|---|---|---|---|---|---|---|
| BG0AD012ZG04R1 | 114 | CFD12G04 | cfd213 | GAGGTGGTTGCTGCCTAGAT | 299 | TGCAAGGACCGAATAAGAGG | 484 | 60 | (CCG)5 | 229 | imperfect | 3D/6D |
| BG0AD013ZE06F1 | 115 | CFD13E06 | cfd219 | GGCCCATCTGTCATTGACTT | 300 | CAGCTTGTGTTGCTCGCTTA | 485 | 60 | (CA)13 | 296 | perfect | 3D/5B |
| BG0AD013ZH10F1 | 116 | CFD13H10 | cfd223 | AAGAGCTACAATGACCAGCAGA | 301 | GCAGTGTATGTCAGGAGAAGCA | 486 | 60 | (TG)18 | 153 | perfect | 3D |
| BG0AD014ZB07F1 | 117 | CFD14B07 | cfd226 | CCACAAACTTTGATGAATGATTACT | 302 | GCGCTATGCTTCATGACATT | 487 | 60 | (TG)39 | 266 | imperfect | 5D |
| BG0AD015ZC12R1 | 118 | CFD15C12 | cfd233 | GAATTTTTGGTGGCCTGTGT | 303 | ATCACTGCACCGACTTTTGG | 488 | 60 | (GA)38 | 289 | perfect | 2D |
| BG0AD015ZG05F1 | 119 | CFD15G05 | cfd238 | GTTGAGGAGGACAAAGAGGC | 304 | GATACGAGCGAGCCCATAAA | 489 | 60 | (GGGA)3 | 151 | perfect | 2B |
| BG0AD015ZH10F1 | 120 | CFD15H10 | cfd239 | CTCTCGTTCTCTCCAGGCTC | 305 | GAGAGGAGAGCTTGCCATTG | 490 | 60 | (GCC)5 | 222 | perfect | 2D |
| BG0AD016ZB03R1 | 121 | CFD16B03 | cfd242 | CCAGTTTGCAGCAGTCACAT | 306 | CAGACCTTAACGGGGTTGAA | 491 | 60 | (GTT)15(AGC)5 | 227 | perfect | 7A |
| BG0AD016ZG11F1 | 122 | CFD16G11 | cfd251 | GAACATGAAGACGAGGAGGC | 307 | AAAGCATCTTTGCTTCTCGC | 492 | 60 | (CCTT)3 | 169 | imperfect | 1B |
| BG0AD017ZA06F1 | 123 | CFD17A06 | cfd255 | AAGTCGACAGGTCGGTTGAG | 308 | TTTACGCAGGATACGGCTCT | 493 | 60 | (GA)7 | 235 | imperfect | 2D |
| BG0AD017ZC05F1 | 124 | CFD17C05 | cfd257 | TCTCAACTTGCAACTGCCAC | 309 | CCCTCCATGGATTCTTGCTA | 494 | 60 | (GAA)6 | 287 | imperfect | 4A/4D |
| BG0AD018ZA05F1 | 125 | CFD18A05 | cfd266 | GAAAACAAAACCCATTTGCG | 310 | AAGCTTCAGTGCCTTTGGAA | 495 | 60 | (CA)20 | 192 | perfect | 5D |
| BG0AD018ZB01F1 | 126 | CFD18B01 | cfd267 | GTGCGTCGTGTAGCAGCTC | 311 | CTCTCTGTCGTCCAGGTCGT | 496 | 60 | (GGC)6 | 280 | perfect | 2A/2B/2D |
| BG0AD018ZC04F1 | 127 | CFD18C04 | cfd270 | AGCATGTGTGTCTCCTCGTG | 312 | CTCCTCACTCCTCGTCTTGG | 497 | 60 | (GGC)5 | 236 | perfect | 2D |
| BG0AD019ZC02F1 | 128 | CFD19C02 | cfd282 | TCTCATCCCTGTTCCTCTGC | 313 | GTCGACGTCTGCACATTGTT | 498 | 60 | (TG)23 | 186 | perfect | 1D |
| BG0AD019ZC12F1 | 129 | CFD19C12 | cfd283 | CCCGTGGTCTTGGGTTC | 314 | AGTTTTGCCATCGGCTGTAT | 499 | 60 | (CCCA)3 | 237 | imperfect | 4B/5D |
| BG0AD019ZF09R1 | 130 | CFD19F09 | cfd287 | TCAAGAAGATGCGTTCATGC | 315 | GGGAGCTTTCCCTAGTGCTT | 500 | 60 | (GT)19 | 256 | perfect | 6D |
| XBG0AA001ZH08F1 | 131 | CFA01H08 | cfa2019 | GACGAGCTAACTGCAGACCC | 316 | CTCAATCCTGATGCGGAGAT | 501 | 60 | (CA)14 | 217 | perfect | 7A |
| BG0AA002ZB08R1 | 132 | CFA02B08 | cfa2026 | CTAATCTAAGCAAGGGCCG | 317 | TTACTGCACTGCGAGTTGCT | 502 | 60 | (CA)24 | 233 | imperfect | 4A |
| BG0AA002ZB12F1 | 133 | CFA02B12 | cfa2028 | TGGGTATGAAAGGCTGAAGG | 318 | ATCGCGACTATTCAACGCTT | 503 | 60 | (CA)21 | 261 | perfect | 7A |
| BG0AA002ZF02F1 | 134 | CFA02F02 | cfa2037 | CCCCTTTTGGTCTGCTGTAA | 319 | CAGTGTGCCTTCTAGCCCTC | 504 | 60 | (CA)23 | 206 | imperfect | 3D |
| BG0AA002ZF10F1 | 135 | CFA02F10 | cfa2040 | TCAAATGATTTCAGGTAACCACTA | 320 | TTCCTGATCCCACCAAACAT | 505 | 60 | (CA)16 | 286 | perfect | 7A/7D |
| BG0AA002ZG01F1 | 136 | CFA02G01 | cfa2043 | CAGCCGAAGAAGGATTTCTG | 321 | GAGGCAGGAACTTAGGGGAG | 506 | 60 | (GA)28 | 202 | imperfect | 2A/2B |

| | | | | | | | | | | | | |
|---|---|---|---|---|---|---|---|---|---|---|---|---|
| BG0AA003ZB06R1 | 137 | CFA03B06 | cfa2049 | TAATTTGATTGGGTCGGAGC | 322 | CGTGTCGATGGTCTCCTTG | 507 | 60 | (GA)21 | 164 | perfect | 7A |
| BG0AA003ZD03R1 | 138 | CFA03D03 | cfa2056 | TGGAAGTTACCCTGTCCGTC | 323 | GAAGAGAGGAGGAGTGGCCT | 508 | 60 | (CGC)4 | 268 | perfect | 1A/7A |
| BG0AA003ZD07R1 | 139 | CFA03D07 | cfa2058 | CCCATTGCCATCTCAGTCTT | 324 | ATAGTAGGCCCAAAGCGATG | 509 | 60 | (TC)28 | 255 | imperfect | 2A |
| BG0AA003ZG08F1 | 140 | CFA03G08 | cfa2070 | TCTGAACCCTTGATTTTCCG | 325 | TTACTGGCAAGCCAGAACTGT | 510 | 60 | (CA)18 | 294 | imperfect | 5B |
| BG0AA004ZA04F1 | 141 | CFA04A04 | cfa2076 | CGAAAAACCATGATCGACAG | 326 | ACCTGTCCAGCTAGCCTCCA | 511 | 60 | (TG)18 | 172 | imperfect | 3D |
| BG0AA004ZD06F1 | 142 | CFA04D06 | cfa2086 | TCTACTTTCAGGGCACCTCG | 327 | TCTCTCCAAACCTCCCTGTAA | 512 | 60 | (CA)21 | 220 | perfect | 2A |
| BG0AA004ZF04F1 | 143 | CFA04F04 | cfa2091 | CCTTCTTAATGACCGGCTCG | 328 | AAACGAAGACGTGGACATGG | 513 | 60 | (CTT)4(TCC)5 | 191 | imperfect | 4B |
| BG0AA004ZH07F1 | 144 | CFA04H07 | cfa2099 | TGCGAAGTATTCAGTGCGTC | 329 | TCAAGACCATCAGCACTCAGA | 514 | 60 | (CA)24 | 277 | perfect | 2A/7D |
| BG0AA005ZA08R1 | 145 | CFA05A08 | cfa2104 | CCTGGCAGAGAAAGTGAAGG | 330 | AGTCGCCGTTGTATAGTGCC | 515 | 60 | (CA)13 | 294 | imperfect | 5A/5D |
| BG0AA005ZB07F1 | 146 | CFA05B07 | cfa2106 | GCTGCTAAGTGCTCATGGTG | 331 | TGAAACAGGGGAATCAGAGG | 516 | 60 | (CCG)5 | 159 | perfect | 7B |
| BG0AA005ZC05R1 | 147 | CFA05C05 | cfa2110 | TCACTACCCGCATGAACAAA | 332 | TTCTGCACAAACCGTTCTGA | 517 | 60 | (TG)19 | 166 | perfect | 7A |
| BG0AA005ZD10F1 | 148 | CFA05D10 | cfa2114 | ATTGGAAGGCCACGATACAC | 333 | CCCGTCGGGTTTTATCTAGC | 518 | 60 | (CA)32 | 209 | perfect | 6A |
| BG0AA005ZG01F1 | 149 | CFA05G01 | cfa2121 | TAAATGGCCATCAAGCAATG | 334 | GCTTGTGAACTAATGCCTCCC | 519 | 60 | (CA)20 | 160 | perfect | 2A/4A |
| BG0AA005ZG10F1 | 150 | CFA05G10 | cfa2123 | CGGTCTTTGTTTGCTCTAAACC | 335 | ACCGGCCATCTATGATGAAG | 520 | 60 | (GA)30 | 259 | perfect | 7A |
| BG0AA006ZB05R1 | 151 | CFA06B05 | cfa2129 | GTTGCACGACCTACAAAGCA | 336 | ATCGCTCACTCACTATCGGG | 521 | 60 | (GA)25 | 159 | perfect | 1A |
| BG0AA006ZC10F1 | 152 | CFA06C10 | cfa2134 | TTTACGGGGACAGTATTCGG | 337 | AAGACACTCGATGCGGAGAG | 522 | 60 | (TC)n | 210 | perfect | 3A |
| BG0AA006ZD02F1 | 153 | CFA06D02 | cfa2135 | TGCCTAAATCTAAATGCCCG | 338 | GGATAATGTGCATGTTCACCG | 523 | 60 | (CA23) | 175 | imperfect | 1A |
| BG0AA006ZE02R1 | 154 | CFA06E02 | cfa2141 | GAATGGAAGGCGGACATAGA | 339 | GCCTCCACAACAGCCATAAT | 524 | 60 | (GA)18 | 229 | perfect | 5A/5D |
| BG0AA006ZG10F1 | 155 | CFA06G10 | cfa2147 | TCATCCCCTACATAACCCGA | 340 | ATCGTGCACCAAGCAATACA | 525 | 60 | (CATC)4 | 291 | perfect | 1B/1D |
| BG0AA006ZH01R1 | 156 | CFA06H01 | cfa2149 | CTTGGAGCTCGGGTAGTAGC | 341 | AAGGCAGCTCAATCGGAGTA | 526 | 60 | (TG)20 | 231 | perfect | 4B |
| BG0AA007ZA08F1 | 157 | CFA07A08 | cfa2153 | TTGTGCATGATGGCTTCAAT | 342 | CCAATCCTAATGATCCGCTG | 527 | 60 | (CT)27 | 200 | perfect | 1A |
| BG0AA007ZB09F1 | 158 | CFA07B09 | cfa2155 | TTTGTTACAACCCAGGGGG | 343 | TTGTGTGGCGAAAGAAACAG | 528 | 60 | (TG)16 | 213 | perfect | 5A |
| BG0AA007ZC10R1 | 159 | CFA07C10 | cfa2158 | TTTCGTCTTCAAAATGCACTG | 344 | TGGTAGCTTACAAAGGTGCG | 529 | 60 | (CA)21 | 229 | perfect | 1A/1B/1D |

| | | | | | | | | | | | | |
|---|---|---|---|---|---|---|---|---|---|---|---|---|
| BG0AA007ZD12F1 | 160 | CFA07D12 | cfa2163 | TTGATCCTTGATGGGAGGAG | 345 | CATCATTGTGTTTACGTTCTTTCA | 530 | 60 | (TC)16 | 163 | perfect | 5A |
| BG0AA007ZE03F1 | 161 | CFA07E03 | cfa2164 | GGGTTGGTGCCTAGATTGAA | 346 | TCAAGGTGCCAACACTTACG | 531 | 60 | (CA)8(GA)9 | 295 | imperfect | 2A/3A |
| BG0AA007ZF08F1 | 162 | CFA07F08 | cfa2170 | TGGCAAGTAACATGAACGGA | 347 | ATGTCATTCATGTTGCCCCT | 532 | 60 | (GA)31 | 199 | imperfect | 3A/3B |
| BG0AA008ZA05F1 | 163 | CFA08A05 | cfa2173 | GACATACTCCGGCGTTGAAT | 348 | TTCCCAGGACATCCTTCTTG | 533 | 60 | (CA)28 | 258 | perfect | ?/4D |
| BG0AA008ZA09F1 | 164 | CFA08A09 | cfa2174 | ACGGCATCACAGGTTAAAGG | 349 | GGTCTTTGCACTGCTAGCCT | 534 | 60 | (CT)15(GT)14 | 191 | perfect | 3D/7A/7D |
| BG0AA008ZB08R1 | 165 | CFA08B08 | cfa2179 | GCATCGACTTCCTCCTCCTA | 350 | TCCGCAGCAGTTCTATGTTG | 535 | 60 | (CCG)5 | 295 | perfect | 4A |
| BG0AA008ZC09F1 | 166 | CFA08C09 | cfa2183 | TCTTGGATGGATTTGTGAGC | 351 | TTCCTTCTCCTTCATTAGCTGC | 536 | 60 | (CA)26 | 168 | imperfect | 3A |
| BG0AA008ZD01F1 | 167 | CFA08D01 | cfa2185 | TTCTTCAGTTGTTTTGGGGG | 352 | TTTGGTCGACAAGCAAATCA | 537 | 60 | (TG)29 | 226 | perfect | 5D |
| BG0AA008ZE12F1 | 168 | CFA08E12 | cfa2187 | TAGCAAAGGGTGCATGTGAG | 353 | GCATGTTACGTCGCTGTTGT | 538 | 60 | (CA)17 | 151 | perfect | ?/1D/5A |
| BG0AA008ZF08F1 | 169 | CFA08F08 | cfa2190 | CAGTCTGCAATCCACTTTGC | 354 | AAAAGGAAACTAAAGCGATGGA | 539 | 60 | (TC)31 | 169 | imperfect | 5A |
| BG0AA008ZF11F1 | 170 | CFA08F11 | cfa2191 | AGAGCAGGAGGTTGGGTTCT | 355 | CCGGAATTTCACTACCAGGA | 540 | 60 | (TCCC)4 | 162 | imperfect | 3B |
| BG0AA008ZG02R1 | 171 | CFA08G02 | cfa2193 | ACATGTGATGTGCGGTCATT | 356 | TCCTCAGAACCCCATTCTTG | 541 | 60 | (GT)12 | 195 | perfect | 3A |
| BG0AA009ZB02F1 | 172 | CFA09B02 | cfa2101 | CAAACCAACCTCATTGACCC | 357 | CCACCAGAACTTCAACCTGG | 542 | 60 | (CA)14 | 216 | perfect | 2A |
| BG0AA010ZE07F1 | 173 | CFA10E07 | cfa2219 | TCTGCCGAGTCACTTCATTG | 358 | GACAAGGCCAGTCCAAAAGA | 543 | 60 | (GT)21 | 223 | perfect | 1A |
| BG0AA010ZH04F1 | 174 | CFA10H04 | cfa2226 | GGAGAAAAGCAAACAGCGAC | 359 | CAGTAGCATCTTCCATGGCG | 544 | 60 | (GCT)6 | 196 | perfect | 1A/3B |
| BG0AA011ZC06F1 | 175 | CFA11C06 | cfa2234 | AATCTGACCGAACAAAATCACA | 360 | TCGGAGAGTATTAGAACAGTGCC | 545 | 60 | (CA)17 | 151 | imperfect* | 3A |
| BG0AA011ZF11F1 | 176 | CFA11F11 | cfa2240 | TGCAGCATGCATTTTAGCTT | 361 | TGCCGCACTTATTTGTTCAC | 546 | 60 | (TA)n | 280 | imperfect | 7A |
| BG0AA011ZF12R1 | 177 | CFA11F12 | cfa2241 | TTGGCCATCAGGCTCTAGTT | 362 | GTGATGCTGTTCTCAAGCCA | 547 | 60 | (CA)21 | 225 | imperfect | 1B |
| BG0AA012ZC05F1 | 178 | CFA12C05 | cfa2250 | AGCCATAGATGGCCCTACCT | 363 | CACTCAATGGCAGGTCCTTT | 548 | 60 | (CAA)11 | 295 | perfect* | 5A |
| BG0AA012ZD08F1 | 179 | CFA12D08 | cfa2256 | GGTAATATTCAGGTTACCGCACA | 364 | GGTAAAGTTATAAATTGTTGTGGGC | 549 | 60 | (TG)29 | 171 | perfect | 4A |
| BG0AA012ZE02F1 | 180 | CFA12E02 | cfa2257 | GATACAATAGGTGCCTCCGC | 365 | CCATTATGTAAATGCTTCTGTTTGA | 550 | 60 | (TG)28 | 167 | imperfect | 7A |
| BG0AA012ZH06F1 | 181 | CFA12H06 | cfa2262 | ACAATGTGGAGATGGCACAA | 366 | TACCAGCTGCACTTCCATTG | 551 | 60 | (TG)17 | 172 | perfect | 2D/3A |
| BG0AA012ZH09F1 | 182 | CFA12H09 | cfa2263 | GGCCATGTAATTAAGGCACA | 367 | CTCCCAGGAGTACAGAAGAGGA | 552 | 60 | (CA)24 | 150 | perfect | 2A |

| | | | | | | | | | | | |
|---|---|---|---|---|---|---|---|---|---|---|---|
| BG0AA013ZE11F1 | 183 | CFA13E11 | cfa22 78 | GCCTCTGCAAGTCTTTACCG | 368 | AAGTCGGCCATCTTCTTCCT | 553 | 60 | (GCG)8 | 151 | perfect | 2B |
| BG0AA014ZB02F1 | 184 | CFA14B02 | cfa22 92 | GGACCGTTTATCCGTAAGCA | 369 | GCCTATGCTGCTGATCCATT | 554 | 60 | (CA)20 | 192 | perfect | 1B |
| BG0AA014ZB09R1 | 185 | CFA14B09 | cfa22 93 | TGGGACTTTGTTGGATTTGTT | 370 | CGCCCTTGTTTGTATTGGTT | 555 | 60 | (CA)20 | 283 | perfect | 7A |

The invention also contemplate the use of microsatellite markers selected from sequences SEQ ID No 1 to 185 or primers selected from sequences SEQ ID No 186-555 or sequence complementary thereto for mapping and genotyping diploid and polyploid species of wheat bread and Triticum, more particularly *Aegilops, Triticum monococcum, T durum*, *T aestivum*, related species and progenies thereof. Markers and primers as described above are useful tools for selecting species of wheat bread, Triticum, progenies thereof, and related species of interest. Such markers and primers allows the study of diversity, phylogeny, and evolution of wheat bread species, Triticum species and related species of commercial interest from their respective wild species. Other examples include without limitation the assessment of the conformity or purity of a given Triticum line or wheat bread, the detection of mutations in Triticum, wheat bread and related species, the identification of cultivars and hybrids of wheat bread, Triticum and related species thanks to markers and primers provided herein.

In a further embodiment, the invention relates to the use of a sequence selected from sequences SEQ ID No 1 to 555 to screen BAC, YAC, cosmid and phage librairies for example for identifying genes associated with QTLs, to assess whether a product comprises wheat bread or related species and also to assess whether a product comprises genetically modified wheat bread or related species.

It shall be understood that any method or process using microsatellite markers and primers thereof as mentioned above for detecting, identifying QTLs and genes of interest, for selecting species of interest and for further mapping is contemplated by the present invention.

In order to complete bread wheat genetic map on the D genome, microsatellites were isolated from an enriched librairy constructed using total DNA of *T. tauschii.* Seventy five percent of clones from our enriched library contained at least one microsatellite. However, a significant number of the microsatellites developed (45 %) failed to show any polymorphism in the set of lines used. It is possible that our microsatellite definition was too wide and included stretch which are too short or too imperfect to detect polymorphism.

Twenty two percent of the clones contained compound microsatellites. This type of repeat sequences could be strongly selected by the enrichment procedure, which simultaneously enriched for ten microsatellite motifs. The enrichment procedure was not efficient for all motifs and non-selected motifs were present. Röder et al. (1995) estimated that there were 1.5 times more $(GA)_n$ repeats than $(GT)_n$ repeats per haploid wheat genome. In our library, we found as many $(GA)_n$ as $(GT)_n$ microsatellites. However, as Röder et al. (1995) developed microsatellites from an unenriched genomic library, their results could be more representative of the biological reality. This suggests that our library was particularly well enriched for $(GT)_n$ microsatellites.

Our library appeared to be particularly enriched for microsatellite motifs having a high GC content: $(GCC)_n$, $(GA)_n$ and $(GT)_n$. This could be due to the stringency of washing conditions employed during the enrichment procedure (filters were washed 20 times, 5 min per wash, in 0.5X SSC at 65°C). However, no enrichment was observed for palindromic microsatellites like $(GC)_n$ or $(AT)_n$ presumably due to self complementation, which prevents hybridisation to the membranes. $(AT)_n$ microsatellite are believed to be very abundant in plants (Wang et al. 1994, Lagercrantz et al. 1993, Morgante et al. 1993).

Transportability of microsatellites markers from *T.tauschii* to bread wheat was very good (92 %). Only 17 of the 293 primer pairs tested (6%) failed to amplify all genotypes including control. The lack of amplification could be due to either sequence errors or to problems of primer synthesis. Six other primer pairs amplified only the *T.tauschii* and W-7984 (a synthetic wheat) lines. Three primers were defined in the close vicinity of the microsatellite sequence. It is known that the proximal regions of microsatellite sequences are unstable (Karhu *et al.* 2000). This may explain that why no amplification was observed, as this region could be highly polymorphic between *T. tauschii* and *T. aestivum*, but not between T. tauschii and W-7984 (a synthetic wheat), whose D-genome comes from a *T.tauschii* accession. The three remaining primer pairs contained short tandem repeats (proto microsatellites) which could confer particular instability in the sequence. Feldman (1997) recently reported that formation of allopolyploid wheat was accompanied by the rapid non-random elimination of specific low-copy, non-coding DNA sequences. It is possible that microsatellite loci in the vicinity of low-copy DNA sequences could have be rearranged or deleted, thus preventing their amplification by PCR.

Dinucleotides appeared to be more polymorphic than other types of motifs as shown in humans by Chakraborty et al. (1997) or in Drosophile by Schug et al. (1998). Microsatellites mapped on Courtot x Chinese Spring population were mostly either $(GA)_n$ or $(GT)_n$ repeats (56 % of simple $(GA)_n$ or $(GT)_n$ and 20 % of compound microsatellites containing $(GA)_n$ or $(GT)_n$). Average PIC levels of the dinucleotide and trinucleotide motifs were in agreement with those described in Bryan et al. (1997). The proportion of polymorphic markers for $(GA)_n$ and $(GT)_n$ microsatellites was similar and difference in the average of allele number was not statistically significant.

We failed to observe any significant influence of the stretch length on the polymorphism within each class of motifs (di-, tri- and tetranucleotides). It is possible that this may be because information on the stretch length was from *T. tauschii* while polymorphism was visualised on hexaploid species. We noticed that in some cases, *T.tauschii* amplification product was either far smaller or larger than the alleles of the hexaploid lines. These differences in size certainly reflected a difference in stretch length. Influence of stretch length has been discussed in the literature. Some authors have found an influence (Weber 1990; Innan et al. 1997) and others have not (Plaschke et al. 1995; Chin et al. 1996). In our case, dinucleotides appeared to be far more polymorphic than other repeat classes. The repeat length of the dinucleotides developed in this study were far longer than the other microsatellites motifs. Therefore if we consider all microsatellites together our study would confirm that polymorphic levels are influenced by stretch length.

In simple microsatellites, the presence of interruptions seem to reduce the polymorphism (table 3) as described by Taylor et al. (1999) and Bell et Ecker (1994). However, we noticed that compound interrupted microsatellites seemed to be more polymorphic than non-interrupted microsatellites. Our classification into the four categories was based on *T. tauschii* sequences and interruptions often occur or change with evolution as it was already shown by Karhu et al. (2000). It would be interesting to sequence alleles from hexaploid wheat and to compare them to *T. tauschii* sequences.

In the case of multiband patterns on bread wheat, products were scarcely mapped at homeologous position. This is probably due to inter- and intrachromosomal duplications within the wheat genome. We have therefore concluded that all primer pairs isolated from *T.tauschii* amplified at least one product, which can be located to the D genome of bread wheat. This good transferability strongly indicates that the wheat and *T.tauschii* genomes are closely related. The apparent random distribution of the mapped loci indicates that microsatellite markers could provide excellent map coverage. On the other hand, the polymorphism detected by these markers varied between different regions of the genome as recently discussed by Temnykh (2000). It is probable that the process of domestication has had an influence in reducing the level of polymorphism. Our laboratory is now attempting to test this hypothesis, by comparing PIC values between wheat cultivars and synthetic wheats.

For polyploid species like wheat, the isolation of microsatellites from diploid ancestor species appears to be an efficient method to develop such molecular markers on a particular elementary genome. Further experiments to isolate microsatellites from the ancestor diploid donor of the A and B genomes (*Triticum urartu* and *Aegilops speltoides*) will perhaps confirm this fact. Primers identified from *T.tauschii* sequences produced at least one band, which mapped onto the D genome of bread wheat. In the future we will analyse the specificity and the transferability of these markers on a range of diploid species and hexaploid and synthetic wheats to investigate evolution of microsatellites after polyploidisation events.

## Materials and methods

### Plant material

For the evaluation of Nei's diversity index, also referred to as polymorphism information content (PIC), seven wheat lines were used: W-7984 (synthetic wheat [Van Deinze *et al*. 1995]), Opata, Courtot, Chinese Spring, Renan, Arche, Récital. *T.tauschii* number 15 was used as an amplification control.
A subset of 94 androgenetic derived doubled haploid lines issued from the cross between Courtot and Chinese Spring (for complete description, see Cadalen et al. 1997) was used for segregation analysis. DNA extraction was performed from fresh leaves by a modified CTAB protocol (Murigneux et al. 1993).

### Isolation of microsatellites markers

A microsatellite enriched library was constructed as described in Edwards et al. (1996) using total DNA from *T.tauschii* number 15. The enrichment procedure involved the following microsatellite motifs: $(GA)_{15}$, $(GT)_{15}$, $(AT)_{15}$, $(GC)_{15}$, $(CAA)_{10}$, $(ATT)_{10}$, $(GCC)_{10}$, $(CATA)_{10}$, $(GATA)_{10}$, $(ATAG)_{10}$.
Plasmids containing the enriched fragments were introduced into *E.coli* DH5α competent cells, which were then plated onto L-agar plates containing 100 μg/mL ampicillin, X-Gal and IPTG (800 ng and 750 ng per Petri dish). Colourless colonies were grown overnight in LB containing 100 μg/mL ampicillin and used to prepare glycerol stocks.

A total of 1,000 clones were sequenced on 377 ABI Prism sequencer (Perkin Elmer) at the Centre National de Sequençage (Evry, France). Primers flanking the microsatellites were designed with the Primer3 program release 0.6 with an optimal annealing temperature of 60°C and an optimal length of 20 bases for the amplification of products between 150 and 300 bp. Following amplification, the microsatellites were divided into useful and non-useful. A useful microsatellite was defined as a motif containing between one and six nucleotides length, repeated at least three times, forming a stretch of at least 12 nucleotides length and matching at a minimum level of 85 % with a perfect microsatellite.

### PCR amplification and product analysis

PCR reactions were performed as described by Tixier et al. (1998) with an annealing temperature of 60°C. Microsatellite polymorphisms were visualised by the silver staining method (Tixier et al. 1997). Enzymes and buffers were provided by Roche Dignostic. Primer pairs were synthesised by Genosys (Cambridge, United Kingdom).

**Estimation of polymorphism information content**

The polymorphism information content, described by Bolstein et al. (1980) and modified by Anderson et al. (1993), refers to the ability of a given marker to detect polymorphism within a population, depending on the number of detectable alleles and the evenness of their frequency distribution. Thus the occurrence of rare alleles has less impact on PIC than alleles occurring at high frequencies. In this study we used a simplified version according to Lagercrantz et al. (1993), which assumes that the inbred wheat lines are homozygous. The calculation of the PIC was carried out using the following formula:

$$PIC_i = 1 - \sum_j p_{ij}^2$$

where $p_{ij}$ is the frequency of the jth pattern for marker i and the summation extends over n patterns.

Mapping

The polymorphic microsatelites were genotyped with the 94 DH lines and the data integrated into an existing framework map (consisting of both RFLP and microsatellites; Cadalen et al. 1997). Map construction was carried out using Mapmaker 3.0 b (Lander et al. 1987) using a thresholds of LOD 3 and 50 cM for the ordering. Genetic distances were calculated using the Kosambi function (Kosambi 1944).

Chromosome arm assignments were done when there was a doubt about the mapping results from Mapmaker. In this case, PCR amplifications were performed on nullisomic-tetrasomic stock lines (Sears 1964). The microsatellite loci were assigned to the chromosomes corresponding to the nullisomic-tetrasomic lines for which no amplification product was obtained, following confirmation that all other nullisomic-tetrasomic lines amplification products were in order.

Statistics

Chi-squared tests for the detection of biased markers were performed with Excel 97. Deviance analyses of generalised linear model using Poisson family were performed to test the effect of both motif length and number of tandem repeats on polymorphism. This glm procedure assumed that the different alleles appeared randomly and independently, i.e. as a Poisson process and is therefore more appropriate than the classical linear model based on Gaussian law. Calculations were carried out using Splus 3.4 statistical package.

**Example 1: Characterisation**

Initially, colony hybridisation was used to identify clones containing microsatellite motifs (no method for colony hybridisation is contained within the material and methods). Following colony hybridisation, 321 (65 %) of the 490 clones tested contained at least one of the microsatellite motifs used in the enrichment procedure. Of these, 25 % contained $(GA)_n$ repeats, 25 % $(GT)_n$ repeats, and 24 % $(GCC)_n$ repeats (compound microsatellites could have been counted several times). A further 1000 randomly chosen clones (*i.e.* not subjected to screening), were subjected to sequence analysis. Of these 75% of clones were shown to contain at least one microsatellite motif. The extent of redundancy within these clones was tested, using a cut off level of 75% homology over 50 nucleotides. This produced a total of 566 different families, which resulted in 378 primers pairs. Following further characterisation 293 primer pairs were used for polymorphic tests. Among the 293 clones used, four types of microsatellites were detected according to Weber (1990): simple and perfect (48%) (type I), simple and imperfect (30%) (type II), compound and perfect (10%) (type III), compound and imperfect (12%) (type IV). Within type I+II sequences, the isolated microsatellites could be grouped in six categories: mono-, di-, tri-, tetra-, penta- and hexa-nucleotides. Di and tri- nucleotides together represented 70 % of the isolated microsatellites (table 1). Within the same type of sequences, $(GCC)_n$ was the most abundant (28%), followed by $(GT)_n$ (22 %) and $(GA)_n$ (21 %). Of the microsatellite motifs used in enrichment procedure, the enrichment was most efficient for the $(GA)_n$, $(GT)_n$ and $(GCC)_n$ motifs. However, surprisingly, other motifs than those used for in the enrichment, were also present in the library. Primers derived from these sequences were also used as molecular markers in this study.

**Example 2: Microsatellite transferability from *T.tauschii* to bread wheat**

Among the 293 primer pairs tested, only 17 failed to amplify all genotypes including the control (*T.tauschii* number 15). Six primer pairs amplified only the control and W-7984 samples. One hundred and thirty two (45 %) of the primer pairs did not detect any polymorphism in the genotypes used. Among the remaining 138 primer pairs, 84 were polymorphic between Courtot and Chinese Spring and 130 between W-7984 and Opata (parents of the ITMI population).

**Example 3: Mapping**

**3.1**

The 84 microsatellites (table 2), which were polymorphic between Courtot and Chinese Spring generated 100 loci. A distorted segregation ratio was observed (P<0.01) for 15 markers. Biased markers were assigned to regions already presenting distorted segregation, for instance chromosomes 4A and 6B (Cadalen et al. 1997). Among the remaining, 64 mapped to the D genome, 7 to the A genome and 11 to the B genome with 3 not assigned. Markers, which mapped to the D genome were evenly distributed all over the genome (figure 1) but coverage of 4D was very poor with only two markers assigned to this chromosome. For primer pairs, which produced only one band, polymorphic microsatellite loci mapped systematically to the D-genome of bread wheat. In the case of multiband patterns, polymorphic bands were not always located on the D genome of bread wheat. We verified that in the case of multiband patterns, at least one band was located on the D genome of bread wheat. When bands were monomorphic between Courtot and Chinese Spring, we assigned a map location using nullitetrasomic lines.

**3.2 Influence of the type of the motif on polymorphism detection of simple microsatellites (type I)**

The number of alleles per locus ranged between one and six in the seven wheat cultivars used. PIC values ranged between 0.245 and 0.816 for the polymorphic loci. As shown in table 1, dinucleotide motifs were more polymorphic (79 % of polymorphic microsatellites) than the other motif types. Deviance analysis in generalised linear model showed that the dinucleotides $(GA)_n$ and $(GT)_n$ had significantly higher number of alleles than trinucleotides. No significant difference was found between motifs of the same class (i.e di- versus tri-nucleotides). The distribution of allele number within each class of motif fitted well a Poisson law. Only 25% of trinucleotide motifs were polymorphic. $(GCC)_n$ microsatellites represented 74 % of trinucleotide motif microsatellite but showed only 23 % of polymorphism. Polymorphism of $(GA)_n$, $(GT)_n$ and $(GCC)_n$ microsatellite motifs is summarised in figure 2. The $(GA)_n$ and $(GT)_n$ average number of alleles (3.67 and 3.24 respectively) do not differ statistically (at $\alpha$=0.05). 29 % of tetra-nucleotide motifs were polymorphic. Penta and hexa-nucleotides were too scarcely represented to conclude concerning the level of polymorphism revealed. In Figure 2, O, P and A means respectively occurrence, proportion of polymorphic microsatellitesand average mean number for polymorphic microsatellites.

**3.3 Influence of stretch length on polymorphism**

The influence of the stretch length on polymorphism was tested in a generalised linear model. When stretch length is introduced solely as the unique term in the model, it is highly significant, however, when tested within each motif class, the effect of stretch length vanished.

**3.4 Influence of mutation inside the stretch (imperfections) on polymorphism**

Microsatellites could be divided into four classes. The average number of alleles was nearly the same for all types of polymorphic markers (table 3). However, the proportion of polymorphic markers was very different between classes. When we compared polymorphism revealed by compound microsatellites versus polymorphism revealed by simple microsatellites, no significant difference was detected neither on polymorphism ratio nor on PIC mean.

**References**

Anderson JA, Churchill GA, Sutrique JE, Tanksley SD, Sorrels ME (1993) Optimizing parental selection for genetic linkage maps. Genome 36: 181-186

Beckmann JS, Soller M (1990) Toward a unified approach to genetic mapping of eukaryotes base on sequence tagged microsatellite sites. Biotechnology 8: 930-932

Bell CJ and Ecker JR (1994) Assignment of 30 microsatellite loci to the linkage map of Arabidopsis. Genomics 19: 137-144

Bishop MD, Kappes SM, Kecle JN, Stone RT, Sanden SLF, Hawkins GA, Toldo SS, Fries K, Gusz MD, Yov J, and Beattie CW (1994) A genetic linkage map for cattle. Genetics 136: 619-639

Botstein D, White RL, Skolnick M, Davis RW (1980) Construction of a genetic linkage map in man using restriction fragment length polymorphisms. Am J Hum Genet 62: 1408-1415

Breiman A and Graur D (1995) Wheat evolution. Isr J Plant Sci 43: 85-98

Bryan GJ, Collins AJ, Stephenson P, Orry A, Smith JB, and Gale MD (1997) Isolation and characterisation of microsatellites from hexaploid bread wheat. Theor Appl Genet 94: 557-563

Cadalen T, Boeuf C, Bernard S, and Bernard M (1997) An intervarietal molecular marker map in Triticum aestivum L. Em. Thell. and comparison with a map from wide cross. Theor Appl Genet 94: 367-377

Chakraborty R, Kimmel M, Stivers DN, Davidson LJ, and Deka R (1997) Relative mutation rates at di-, tri-, and tetranucleotide microsatellite loci. Proceedings of the National Academy of Sciences of the United States of America 94: 1041-1046

Chao S, Sharp PJ, Worland AJ, Warham EJ, Koebner RMD, Gale MD (1989) RFLP-base genetics maps of wheat homeologous group 7 chromosomes. Theor Appl Genet 78: 495-504

Chin ECL, Senior ML, Shu H, Smith JSC (1996) Maize simple repetitive DNA sequences: abundance and allele variation. Genome 39:866-873

Dib C, Faure S, Fizames C, Samson D, Drouot N, Vignal A, Millasseau P, Marc S, Hazan J, Seboun E, Lathrop M, Gyapay G, Morisette J, and Weissenbach J (1996) A comprehensive genetic map of the human genome based on 5,264 microsatellites. Nature 380: 152-154

Edwards KJ, Barker JHA, Daly A, Jones C, and Karp A (1996) Microsatellite libraries enriched for several microsatellite sequences in plants. Biotechniques 20: 758-760

Feldman M, Liu B, Segal G, Abbo S, Levy AA, Vega JM (1997) Rapid elimination of low-copy DNA sequences in polyploid wheat: A possible mechanism for differentiation of homoelogous chromosomes. Genetics 147: 1381-1387

Gale MD, Atkinson MD, Chinoy CN, Harcourt RL, Jiu J, Li QY, Devos KM (1995) Genetic map of hexaploid wheat. In: Proceeding of 8$^{th}$ International Wheat Genetic Symposium, Liu ZS, Xin XY (eds), Beijing, China, pp 29-40

Gupta PK, Varshney RK, Sharma PC, and Ramesh B (1999) Molecular markers and their applications in wheat breeding. Plant Breed 118: 369-390

Karhu A, Dieterich JH, Savolainen, O (2000) Rapid expansion of microsatellite sequences in pines. Mol Biol Evol 17: 259-265

Kihara H (1950) Genome analysis in Triticum and Aegilops. Concl Rev Cyt 16: 101-123

Kosambi DD (1944) The estimation of map distances from recombination values. Ann Eugen 12: 172-175

Lagercrantz U, Ellegren H, and Anderson L (1993) The abundance of various polymorphic microsatellite motifs differs between plants and vertebrates. Nucleic Acids Res 21: 1111-1115

Lander ES, Green P, Abrahamson J, Barlow A, Daly MJ, Lincoln SE, and Newburg L (1987) Mapmaker: an interactive computer package for constructing primary genetic linkage maps of experimental and natural populations. Genomics 1: 174-181

Levinson G and Gutman GA (1987) Slipped-strand mispairing: a major mechanism for DNA sequence evolution. Mol Biol Evol 4: 203-221

Litt M and Lutty JA (1989) An hypervariable microsatellite revealed by in vitro amplification of a dinucleotide repeat within the cardiac muscle actin gene. Am J Hum Genet 44: 397-401

Liu Y, Tsunewaki K (1991) Restriction fragment length polymorphism (RFLP) analysis in wheat. II. Linkage maps of the RFLP sites in common wheat. Jpn J Genet 66:617-633

Morgante M and Olivieri AM (1993) PCR-amplified microsatellites as markers in plant genetics. Plant J 3: 175-182

Murigneux A, Barloy D, Leroy P, and Beckert M (1993) Molecular and morphological evaluation of doubled haploid lines in maize. 1. Homogeneity within DH lines. Theoretical and Applied Genetics 86: 837-842

Nelson JC, Sorrels ME, Van Deynze AE, LU YH, Atkinson M, Bernard M, Leroy p, Farris JD, Anderson JA (1995a) Molecular mapping of wheat: Major genes and rearrangements in homoelogous groups 4, 5, and 7. Genetics 141: 721-731

Nelson JC, Van Deynze AE, Autrique E, Sorrels ME, Lu YH, Merlino M, Atkinson MD, Leroy P (1995b) Molecular mapping of wheat. Homoelogous group 2. Genome 38: 516-524

Nelson JC, Van Deynze AE, Autrique E, Sorrels ME, Lu YH, Negre S, Bernard M, Leroy P (1995c) Molecular mapping of wheat. Homoelogous group 3. Genome 38: 525-533

Plaschke J, Ganal MW, Röder MS (1995) Detection of genetic diversity in closely related bread wheat using microsatellite markers. Theor Appl Genet 91: 1001-1007

Röder MS, Kurzun V, Wendehake K, Plaschke J, Tixier M-H, Leroy P, and Ganal MW (1998) A microsatellite map of wheat. Genetics 149: 2007-2023

Röder MS, Plaschke J, König SU, Börner A, Sorells ME, Tanksley SD, and Ganal MW (1995) Abundance, variability and chromosomal location of microsatelllites in wheat. Mol Gen Genet 246: 327-333

Sears RE (1964) Nullisomic-tetrasomic combinations in hexaploid wheat. In: Chromosome manipulations and plant genetics Riley R, Lewis KR (eds) Edinburgh Oliver and Boyd pp

Schug MD, Hutter CM, Wetterstrand KA, Gaudette MS, Mackay TFC, and Aquadro CF (1998) The mutation rates of di-, tri- and tetranucleotide repeats in Drosophila melanogaster. Mol Biol Evol 15: 1751-1760

Shanghai-Maroof MA, Biyashev RM, Yang GP, Zhang Q, Allard RW (1994) Extraordinarily polymorphic microsatellite DNA in barley: species diversity, chromosomal locations and population dynamics. Proc Natl Acad Sci USA 91: 5466-5470

Taramino G and Tingey S (1996) Simple sequence repeats for germplasm analysis and mapping in maize. Genome 39: 277-287

Taylor JS, Durkin JMH, and Breden F (1999) The death of a microsatellite: a phylogenetic perspective on microsatellite interruptions. Mol Biol Evol 16: 567-572

Temnykh S, Park WD, Ayres N, Cartinhour S, Hauck N, Lipovich L, Cho YG, Ishii T, and McCouch SR (2000) Mapping and genome organization of microsatellite sequences in rice (Oryza sativa L.). Theor Appl Genet 100: 697-712

Tixier MH, Sourdille P, Röder M, Leroy P, and Bernard M (1997) detection of wheat microsatellites using a non radioactive silver-nitrate staining method. J Genet Breed 51: 175-177

Tixier MH, Sourdille P, Charmet G, Gay G, Jaby C, Cadalen T, Bernard S, Nicolas P, Bernard M (1998) Detection of QTLs for crossability in wheat using a doubled-haploid population. Theor Appl Genet 97: 1076-1082

Van Deynze AE, Dubcovsky J, Gill KS, Nelson JC, Sorells ME, Dvorak J, Gill BS, Lagudah ES, McCouch SR, Appels R (1995) Molecular-genetic maps for group1 chromosomes of Triticeae species and their relation to chromosomes in rice and oats. Genome 38: 45-59

Wang Z, Weber JL, Zhong G, and Tanksley SD (1994) Survey of plant short tandem DNA repeats. Theor Appl Genet 88: 1-6

Weber JL and May PE (1989) Abundant class of human DNA polymorphism which can be typed using the polymerase chain reaction. Am J Hum Genet 44: 388-396

Weber JL (1990) Informativeness of human (dC-dA)n.(dG.dT)n polymorphisms. Genomics 7: 524-530

Wu KS and Tanksley SD (1993) Abundance, polymorphism and genetic mapping of microsatellites in rice. Mol Gen Genet 241: 225-235

## Claims

1. Map of the wheat D genome comprising the genome location of a microsatellite marker selected from the group consisting of sequences SEQ ID No 1 to 185.

2. Map according to claim 1, wherein it comprises the genome location of at least 25, 50, 100, 150 or preferably 185 microsatellite markers selected from the sequences SEQ ID No 1 to 185.

3. Map according to any one of claims 1 and 2, wherein it comprises the genome location of at least 10, 20, 30, 40 or preferably 45 microsatellite markers as depicted in figure 1 selected from the microsatellite markers of sequences SEQ ID No 3, 5, 7, 8, 9, 11, 12, 14, 17, 18, 19, 21, 23, 26, 27, 29, 31, 32, 34, 35, 36, 37, 38, 40, 41, 43, 44, 45, 46, 47, 53, 55, 56, 57, 58, 60, 62, 63, 64, 66, 68, 69, 70, 71, 79, 81.

4. Use of a map according to any one of claims 1 to 3, of microsatellite markers selected from sequences SEQ ID No 1 to 185 or primers selected from sequences SEQ ID No 186-555 or sequence complementary thereto for identifying genes responsible for a phenotypic trait of interest, notably QTLs in wheat.

5. Use according to claim 5 for analyzing diseases, for identifying the implicated genes of said diseases and their alleles, for identifying development factors, quality factors and factors conferring resistance to pathogens and xenobiotics.

6. Use of left primers selected from sequences SEQ ID No 186-370 and right primers selected from sequences SEQ ID No 371-555 or sequence complementary thereto to amplify and detect microsatellite markers selected from the sequences SEQ ID No 1 to 185 respectively.

7. Use of left primers selected from sequences SEQ ID No 186-370 and right primers selected from sequences SEQ ID No 371-555 or sequence complementary thereto for assessing the interspecific or intraspecific polymorphism of microsatellite markers selected from the sequences SEQ ID No 1 to 185 respectively.

8. Use of microsatellite markers selected from sequences SEQ ID No 1 to 185 or primers selected from sequences SEQ ID No 186-555 or sequence complementary thereto for mapping and genotyping diploid and polyploid species of Triticum, more particularly *Aegilops, Triticum monococcum*, *T durum, T aestivum*, related species and progenies thereof.

9. Use of microsatellite markers selected from sequences SEQ ID No 1 to 185 or primers selected from sequences SEQ ID No 186-555 or sequence complementary thereto for selecting species of Triticum, progenies thereof, and related species of interest.

10. Use of microsatellite markers selected from sequences SEQ ID No 1 to 185 or primers selected from sequences SEQ ID No 186-555 or sequence complementary thereto for studying diversity, phylogeny, and evolution of Triticum species and related species of commercial interest from wild species.

11. Use of microsatellite markers selected from sequences SEQ ID No 1 to 185 or primers selected from sequences SEQ ID No 186-555 or sequence complementary thereto for assessing the conformity, purity of a given Triticum line.

**12.** Use of microsatellite markers selected from sequences SEQ ID No 1 to 185 or primers selected from sequences SEQ ID No 186-555 or sequence complementary thereto for detecting mutations in Triticum and related species.

**13.** Use of microsatellite markers selected from sequences SEQ ID No 1 to 185 or primers selected from sequences SEQ ID No 186-555 or sequence complementary thereto for identifying cultivars and hybrids of Triticum and related species.

**14.** Use of a sequence selected from sequences SEQ ID No 1 to 555 to screen BAC, YAC, cosmid and phage librairies for identifying genes associated with QTLs.

**15.** Use of a sequence selected from sequences SEQ ID No 1 to 555 to assess whether or not a product comprises wheat or related species.

**16.** Use of a sequence selected from sequences SEQ ID No 1 to 555 to assess whether or not a product comprises genetically modified wheat or related species.

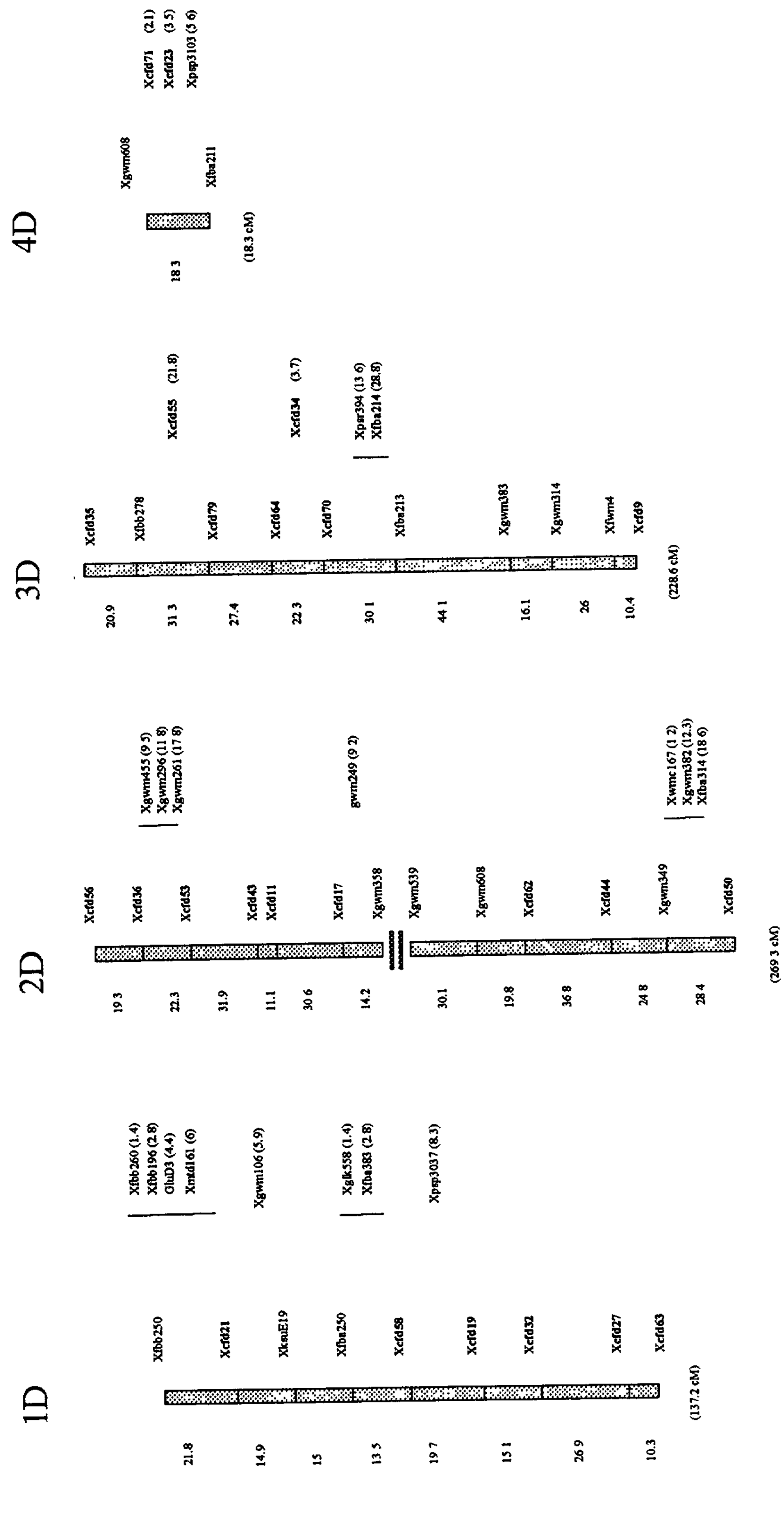
1D
Xfbb250
Xcfd21
XksuE19
Xfba250
Xcfd58
Xcfd19
Xcfd32
Xcfd27
Xcfd63
21.8
14.9
15
13.5
19.7
15.1
26.9
10.3
(137.2 cM)
Xfbb260 (1.4)
Xfbb196 (2.8)
GluD3 (4.4)
Xmtd161 (6)
Xgwm106 (5.9)
Xglk558 (1.4)
Xfba383 (2.8)
Xpsp3037 (8.3)
2D
Xcfd56
Xcfd36
Xcfd53
Xcfd43
Xcfd11
Xcfd17
Xgwm358
Xgwm539
Xgwm608
Xcfd62
Xcfd44
Xgwm349
Xcfd50
19.3
22.3
31.9
11.1
30.6
14.2
30.1
19.8
36.8
24.8
28.4
(269.3 cM)
Xgwm455 (9.5)
Xgwm296 (11.8)
Xgwm261 (17.8)
gwm249 (9.2)
Xwmc167 (1.2)
Xgwm382 (12.3)
Xfba314 (18.6)
3D
Xcfd35
Xfbb278
Xcfd79
Xcfd64
Xcfd70
Xfba213
Xgwm383
Xgwm314
Xfwm4
Xcfd9
20.9
31.3
27.4
22.3
30.1
44.1
16.1
26
10.4
(228.6 cM)
Xcfd55 (21.8)
Xcfd34 (3.7)
Xpsr394 (13.6)
Xfba214 (28.8)
4D
Xgwm608
Xfba211
18.3
(18.3 cM)
Xcfd71 (2.1)
Xcfd23 (3.5)
Xpsp3103 (5.6)

Figure 1/...

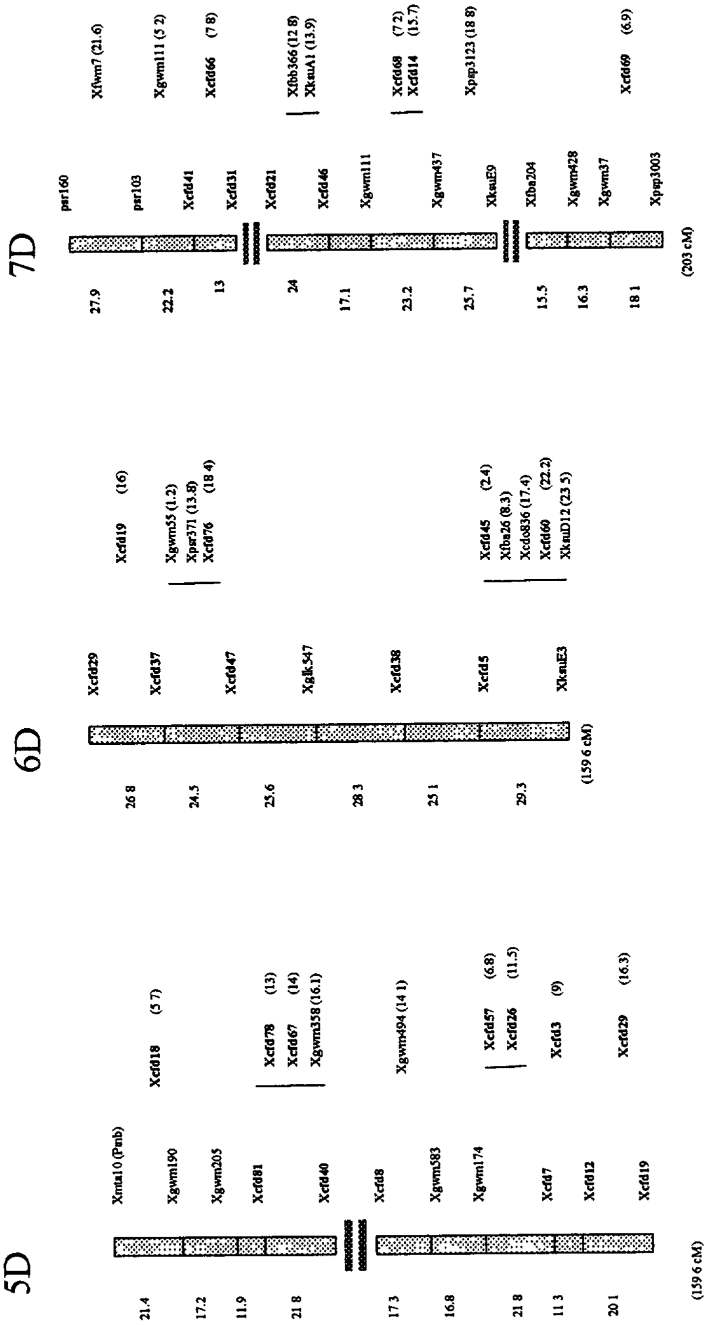
5D
Xnta10 (Pmb)
Xgwm190
Xgwm205
Xcfd81
Xcfd40
Xcfd8
Xgwm583
Xgwm174
Xcfd7
Xcfd12
Xcfd19
(159 6 cM)
Xcfd18 (5 7)
Xcfd78 (13)
Xcfd67 (14)
Xgwm358 (16.1)
Xgwm494 (14 1)
Xcfd57 (6.8)
Xcfd26 (11.5)
Xcfd3 (9)
Xcfd29 (16.3)
6D
Xcfd29
Xcfd37
Xcfd47
Xglk547
Xcfd38
Xcfd5
XksuE3
(159 6 cM)
Xcfd19 (16)
Xgwm55 (1.2)
Xpsr371 (13.8)
Xcfd76 (18 4)
Xcfd45 (2.4)
Xfba26 (8.3)
Xcdo836 (17.4)
Xcfd60 (22.2)
XksuD12 (23 5)
7D
psr160
psr103
Xcfd41
Xcfd31
Xcfd21
Xcfd46
Xgwm111
Xgwm437
XksuE9
Xfba204
Xgwm428
Xgwm37
Xpsp3003
(203 cM)
Xfwm7 (21.6)
Xgwm111 (5 2)
Xcfd66 (7 8)
Xfbb366 (12 8)
XksuA1 (13.9)
Xcfd68 (7 2)
Xcfd14 (15.7)
Xpsp3123 (18 8)
Xcfd69 (6.9)

**Figure 1**

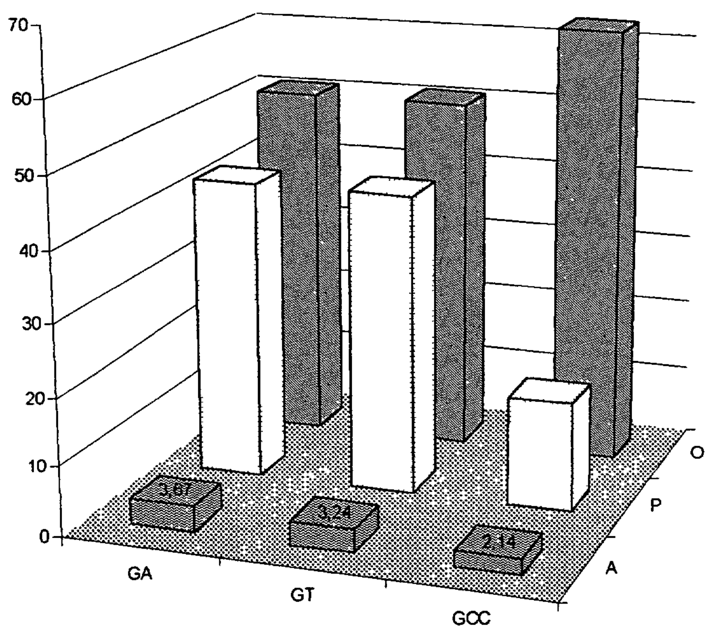


Figure 2

European Patent Office

# EUROPEAN SEARCH REPORT

Application Number
EP 00 40 3659

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| X | PESTSOVA E ET AL.: "Isolation and mapping of microsatellite markers specific for the D genome of bread wheat" GENOME, vol. 43, August 2000 (2000-08), pages 689-697, XP001014185 * see especially page 695, column 2, paragraph 3 * * the whole document * --- | 1-16 | C12Q1/68 |
| X | PESTSOVA E ET AL.: "Microsatellite analysis of Aegilops tauschii germplasm" THEORETICAL AND APPLIED GENETICS, vol. 101, July 2000 (2000-07), pages 100-106, XP001014237 * see especially table 1 * * the whole document * --- | 1-4, 6-13,15, 16 | |
| X | RÖDER M S ET AL.: "A microsatellite map of wheat" GENETICS, vol. 149, August 1998 (1998-08), pages 2007-2023, XP001014214 * the whole document * --- | 1-4,6-9, 12-16 | TECHNICAL FIELDS SEARCHED (Int.Cl.7) C12Q |
| X | LELLEY T ET AL.: "Analysis of relationships between Aegilops tauschii and the D genome of wheat utilizing microsatellites" GENOME, vol. 43, August 2000 (2000-08), pages 661-668, XP001014174 * the whole document * --- -/-- | 1,4, 6-13,15, 16 | |

~~The present search report has been drawn up for all claims~~

| Place of search | Date of completion of the search | Examiner |
|---|---|---|
| THE HAGUE | 20 July 2001 | Knehr, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number
EP 00 40 3659

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-16 (partial)

European Patent Office

# EUROPEAN SEARCH REPORT

Application Number
EP 00 40 3659

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PLASCHKE J ET AL: "THE USE OF WHEAT ANEUPLOIDS FOR THE CHROMOSOMAL ASSIGNMENT OF MICROSATELLITE LOCI" EUPHYTICA,NL,KLUWER ACADEMIC PRESS, AMSTERDAM, vol. 89, no. 1, 1996, pages 33-40, XP000604254 ISSN: 0014-2336 * the whole document * --- | 1-3 | |
| X | BRYAN G J ET AL.: "Isolation and characterization of microsatellites from hexaploid bread wheat" THEORETICAL AND APPLIED GENETICS, vol. 94, 1997, pages 557-563, XP001014216 * the whole document * --- | 1-3 | |
| Y | DE 195 25 284 A (INST PFLANZENGENETIK & KULTUR) 2 January 1997 (1997-01-02) * the whole document * --- | 1-16 | |
| Y | PRASAD M ET AL.: "The use of microsatellites for detecting DNA polymorphism, genotype identification and genetic diversity in wheat" THEORETICAL AND APPLIED GENETICS, vol. 100, February 2000 (2000-02), pages 584-592, XP001014236 * the whole document * --- | 1-16 | TECHNICAL FIELDS SEARCHED (Int.Cl.7) |
| D,Y | EDWARDS K J ET AL.: "Microsatellite libraries enriched for several microsatellite sequences in plants" BIOTECHNIQUES, vol. 20, no. 5, 1996, pages 758-760, XP002172534 * the whole document * --- | 1-16 | |
| | -/-- | | |

~~The present search report has been drawn up for all claims~~

| Place of search | Date of completion of the search | Examiner |
|---|---|---|
| THE HAGUE | 20 July 2001 | Knehr, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

**LACK OF UNITY OF INVENTION SHEET B**

Application Number
EP 00 40 3659

The Search Division considers that the present European patent application does not comply with the requirements of unity of invention and relates to several inventions or groups of inventions, namely:

1. Claims: 1-16 (partial)

INVENTION 1:
A map of the wheat D genome comprising a microsatellite marker consisting of SEQ ID NO:1, a primer pair referring to SEQ ID NOS: 186 and 371, suitable for its amplification, and the use of such a map, such a microsatellite marker, or such primers, for mapping and genotyping of wheat (Triticum) species.

2. Claims: 1-16 (partial)

INVENTION 2:
A map of the wheat D genome comprising a microsatellite marker consisting of SEQ ID NO:2, a primer pair referring to SEQ ID NOS: 187 and 372, suitable for its amplification, and the use of such a map, such a microsatellite marker, or such primers, for mapping and genotyping of wheat (Triticum) species.

3. Claims: 1-16 (partial)

INVENTIONS 3 TO 183:

INVENTION 3:
A map of the wheat D genome comprising a microsatellite marker consisting of SEQ ID NO:3, a primer pair referring to SEQ ID NOS: 188 and 373, suitable for its amplification, and the use of such a map, such a microsatellite marker, or such primers, for mapping and genotyping of wheat (Triticum) species.

...

ibidem

INVENTION 4 (microsatellite marker consisting of SEQ ID NO: 4, primers pair SEQ ID NOS:189 and 374),
INVENTION 5 (microsatellite marker consisting of SEQ ID NO: 5, primers pair SEQ ID NOS:190 and 375),
...
INVENTION 183 (microsatellite marker consisting of SEQ ID NO: 183, primers pair SEQ ID NOS:368 and 553),

4. Claims: 1-16 (partial)

INVENTION 184:

European Patent Office

**LACK OF UNITY OF INVENTION SHEET B**

Application Number
EP 00 40 3659

The Search Division considers that the present European patent application does not comply with the requirements of unity of invention and relates to several inventions or groups of inventions, namely:

A map of the wheat D genome comprising a microsatellite marker consisting of SEQ ID NO:184, a primer pair referring to SEQ ID NOS: 369 and 554, suitable for its amplification, and the use of such a map, such a microsatellite marker, or such primers, for mapping and genotyping of wheat (Triticum) species.

5. Claims: 1-16 (partial)

INVENTION 185:
A map of the wheat D genome comprising a microsatellite marker consisting of SEQ ID NO:185, a primer pair referring to SEQ ID NOS: 370 and 555, suitable for its amplification, and the use of such a map, such a microsatellite marker, or such primers, for mapping and genotyping of wheat (Triticum) species.

European Patent Office

# EUROPEAN SEARCH REPORT

Application Number
EP 00 40 3659

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| D,A | ROEDER M S ET AL: "ABUNDANCE, VARIABILITY AND CHROMOSOMAL LOCATION OF MICROSATELLITES IN WHEAT"<br>MOLECULAR AND GENERAL GENETICS,DE,SPRINGER VERLAG, BERLIN,<br>vol. 246, no. 3,<br>6 February 1995 (1995-02-06), pages 327-333, XP000605158<br>ISSN: 0026-8925<br>* the whole document *<br>--- | | |
| A | MA Z Q ET AL: "FREQUENCIES AND SEQUENCE CHARACTERISTICS OF DI-, TRI-, AND TETRA-NUCLEOTIDE MICROSATELLITES IN WHEAT"<br>GENOME,CA,OTTAWA,<br>vol. 39, no. 1,<br>1 February 1996 (1996-02-01), pages 123-130, XP000604119<br>ISSN: 0831-2796<br>* the whole document *<br>----- | | TECHNICAL FIELDS SEARCHED (Int.Cl.7) |

~~The present search report has been drawn up for all claims~~

| Place of search | Date of completion of the search | Examiner |
|---|---|---|
| THE HAGUE | 20 July 2001 | Knehr, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 40 3659

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2001

| Patent document cited in search report | Publication date | Patent family member(s) | Publication date |
|---|---|---|---|
| DE 19525284 A | 02-01-1997 | DE 19525284 A1 | 02-01-1997 |
| | | WO 9701567 A2 | 16-01-1997 |
| | | EP 0835324 A2 | 15-04-1998 |

EPO FORM P0459

For more details about this annex : see Official Journal of the European Patent Office, No. 12/82